(19) Europäisches Patentamt
European Patent Office
Office européen des brevets

(11) Veröffentlichungsnummer: **0 284 561 B1**

(12) # EUROPÄISCHE PATENTSCHRIFT

(45) Veröffentlichungstag der Patentschrift: **12.05.93**

(21) Anmeldenummer: **88810163.1**

(22) Anmeldetag: **16.03.88**

(51) Int. Cl.5: **C07C 225/02**, C07C 225/16, C07C 323/22, C07C 317/24, C07D 295/10, C07D 295/12, C08F 2/50, G03F 7/00

(54) **Neue alpha-Aminoacetophenone als Photoinitiatoren.**

(30) Priorität: **26.03.87 CH 1152/87**

(43) Veröffentlichungstag der Anmeldung:
**28.09.88 Patentblatt 88/39**

(45) Bekanntmachung des Hinweises auf die Patenterteilung:
**12.05.93 Patentblatt 93/19**

(84) Benannte Vertragsstaaten:
**AT BE CH DE ES FR GB IT LI NL SE**

(56) Entgegenhaltungen:
**EP-A- 0 003 002**
**EP-A- 0 088 050**
**EP-A- 0 117 233**
**EP-A- 0 138 754**
**GB-A- 943 266**
**GB-A- 1 386 029**
**GB-A- 1 574 894**

(73) Patentinhaber: **CIBA-GEIGY AG**
**Klybeckstrasse 141**
**CH-4002 Basel(CH)**

(72) Erfinder: **Desobry, Vincent, Dr.**
**Route du Confin 50**
**CH-1723 Marly(CH)**
Erfinder: **Dietliker, Kurt, Dr.**
**Av. Jean-Marie Musy 6**
**CH-1700 Fribourg(CH)**
Erfinder: **Hüsler, Rinaldo, Dr.**
**Route du Confin 52**
**CH-1723 Marly(CH)**
Erfinder: **Rutsch, Werner, Dr.**
**Av. Jean-Marie Musy 6**
**CH-1700 Fribourg(CH)**
Erfinder: **Rembold, Manfred, Dr.**
**Im Aeschfeld 21**
**CH-4147 Aesch(CH)**
Erfinder: **Sitek, Franciszek, Dr.**
**Grossmattweg 11**
**CH-4106 Therwil(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue Derivate des α-Aminoacetophenons, die durch eine Allyl- oder Aralkylgruppe in α-Stellung gekennzeichnet sind, sowie ihre Verwendung als Photoinitiatoren für die Photopolymerisation von ethylenisch ungesättigten Verbindungen, insbesondere für die Photohärtung von pigmentierten Systemen, wie Druckfarben oder Weisslack.

Derivate des α-Aminoacetophenons sind aus der EP-A-3002 als Photoinitiatoren für ethylenisch ungesättigte Verbindungen bekannt. Besitzen diese Verbindungen in 4-Stellung des Phenylrestes einen Schwefel oder Sauerstoff enthaltenden Substituenten, so sind die Verbindungen besonders geeignet als Photoinitiatoren für pigmentierte photohärtbare Systeme (EP-A-88.050 und 117.233), beispielsweise für UV-härtbare Druckfarben.

In der EP-A-138.754 sind Derivate des α-Aminoacetophenons beschrieben, die in 4-Stellung des Phenylrestes eine Aminogruppe besitzen. Diese Verbindungen werden in Kombination mit Photosensibilisatoren aus der Klasse der aromatischen Carbonylverbindungen verwendet.

Es wurde nunmehr gefunden, dass sich aus dieser allgemeinen Klasse von α-Aminoacetophenonen solche durch besonders hohe Wirksamkeit als Photoinitiatoren auszeichnen, die in α-Stellung mindestens eine Alkenyl- oder Aralkylgruppe enthalten. Diese Verbindungen eignen sich vor allem für die Verwendung in Druckfarben.

Im einzelnen handelt es sich um die Verbindungen der Formel I, II, III oder IIIa,

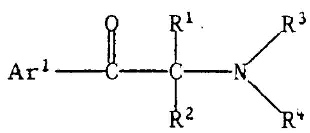
I

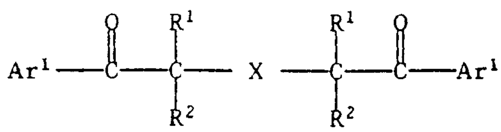
II

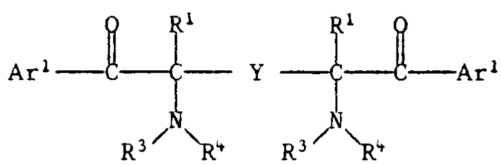
III

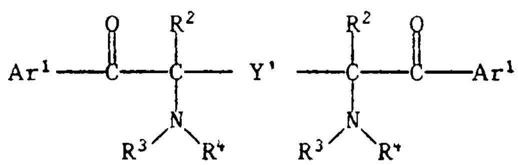
IIIa

worin $Ar^1$ einen aromatischen Rest der Formel IV, V, VI oder VII bedeutet,

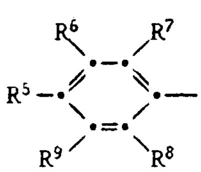
IV,

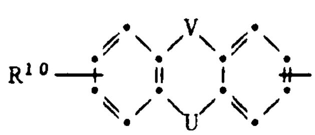
V

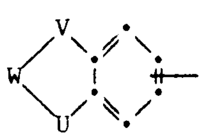
VI

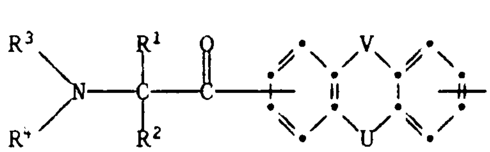
VII

worin

X einen zweiwertigen Rest der Formel

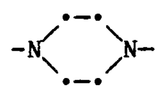 ,

$-N(R^{11})-$ oder $-N(R^{11})-R^{12}-N(R^{11})-$ bedeutet,

Y $C_1-C_6$-Alkylen, Cyclohexylen oder eine direkte Bindung bedeutet,

Y' Xylylen, $C_4-C_8$-Alkendiyl, $C_6-C_{10}$-Alkadiendiyl, Dipentendiyl oder Dihydroxylylen bedeutet,

U $-O-$, $-S-$ oder $-N(R^{17})-$ bedeutet,

V $-O-$, $-S-$, $-N(R^{17})-$, $-CO-$, $-CH_2-$, $-CH_2CH_2-$, $C_2-C_6$-Alkyliden oder eine direkte Bindung bedeutet,

W unverzweigtes oder verzweigtes $C_1-C_7$-Alkylen oder $C_2-C_6$-Alkyliden bedeutet,

$R^1$ entweder

(a) ein Rest der Formel

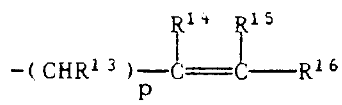

worin p null oder 1 ist,

oder

(b) ein Rest der Formel

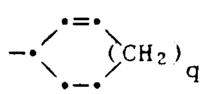

ist, wobei q 0, 1, 2 oder 3 bedeutet

oder

(c) ein Rest der Formel

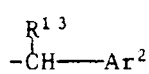

ist, worin $Ar^2$ einen unsubstituierten oder durch Halogen, OH, $C_1-C_{12}$-Alkyl oder durch OH, Halogen, $-N(R^{11})_2$, $-C_1-C_{12}$-Alkoxy, $-COO(C_1-C_{18}$-Alkyl), $-CO(OCH_2CH_2)_nOCH_3$ oder $-OCO(C_1-C_4)$-Alkyl substituiertes $C_1-C_4$-Alkyl, $C_1-C_{12}$-Alkoxy oder durch $-COO(C_1-C_{18}$-Alkyl) oder $-CO(OCH_2CH_2)_nOCH_3$ substituiertes $C_1-C_4$-Alkoxy, $-(OCH_2CH_2)_nOH$, $-(OCH_2CH_2)_nOCH_3$, $C_1-C_8$-Alkylthio, Phenoxy, $-COO(C_1-C_{18}$-Alkyl), $-CO(OCH_2CH_2)_nOCH_3$, Phenyl oder Benzoyl substituierten Phenyl-, Naphthyl-, Furyl-, Thienyl- oder Pyridylrest bedeutet, worin n 1-20 ist, oder

(d) zusammen mit $R^2$ einen Rest der Formel

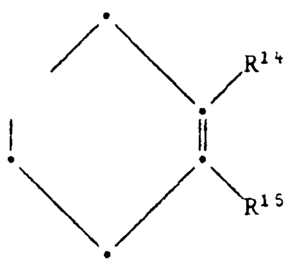

oder

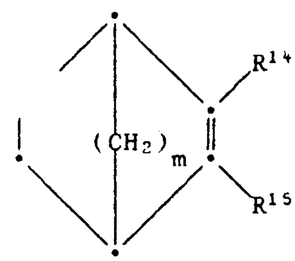

bildet, worin m 1 oder 2 ist,

$R^2$ eine der für $R^1$ gegebenen Bedeutungen hat oder $C_5-C_6$-Cycloalkyl, unsubstituiertes oder durch $C_1-C_4$-Alkoxy, Phenoxy, Halogen oder Phenyl substituiertes $C_1-C_{12}$-Alkyl oder unsubstituiertes oder durch Halogen, $C_1-C_{12}$-Alkyl oder $C_1-C_4$-Alkoxy substituiertes Phenyl bedeutet,

$R^3$ Wasserstoff, $C_1-C_{12}$-Alkyl, durch Hydroxy, $C_1-C_4$-Alkoxy, $-CN$ oder $-COO(C_1-C_4$-Alkyl) substituiertes $C_2-C_4$-Alkyl, $C_3-C_5$-Alkenyl, $C_5-C_{12}$-Cycloalkyl oder $C_7-C_9$-Phenylalkyl bedeutet,

$R^4$ $C_1-C_{12}$-Alkyl, durch Hydroxy, $C_1-C_4$-Alkoxy, $-CN$ oder $-COO(C_1-C_4$-Alkyl) substituiertes $C_2-C_4$-Alkyl, $C_3-C_5$-Alkenyl, $C_5-C_{12}$-Cycloalkyl, $C_7-C_9$-Phenylalkyl, Phenyl oder durch Halogen, $C_1-C_{12}$-Alkyl, $C_1-C_4$-Alkoxy oder $-COO(C_1-C_4$-Alkyl) substituiertes Phenyl bedeutet oder $R^4$ zusammen mit $R^2$ $C_1-C_7$-Alkylen, $C_7-C_{10}$-Phenylalkylen, o-Xylylen, 2-Butenylen oder $C_2-C_3$-Oxa- oder Azaalkylen bedeutet, oder

$R^3$ und $R^4$ zusammen $C_3-C_7$-Alkylen bedeuten, das durch $-O-$, $-S-$, $-CO-$ oder $-N(R^{17})-$ unterbrochen sein kann oder durch Hydroxy, $C_1-C_4$-Alkoxy oder $-COO(C_1-C_4$-Alkyl) substituiert sein kann,

$R^5$, $R^6$, $R^7$, $R^8$ und $R^9$ unabhängig voneinander Wasserstoff, Halogen, $C_1-C_{12}$-Alkyl, $C_5-C_6$-Cycloalkyl, Phenyl, Benzyl, Benzoyl oder eine Gruppe $-OR^{18}$, $-SR^{19}$, $-SO-R^{19}$, $-SO_2-R^{19}$, $-N(R^{20})(R^{21})$, $-NH-SO_2-R^{22}$ oder

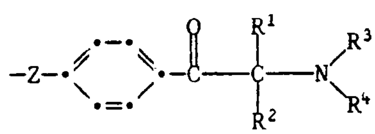

bedeuten, worin Z $-O-$, $-S-$, $-N(R^{11})-$, $-N(R^{11})-R^{12}-N(R^{11})-$ oder

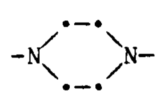

bedeutet, wobei im Falle, das $R^1$ Allyl und $R^2$ Methyl ist, $R^5$ nicht $-OCH_3$ ist, und im Falle, dass $R^1$ Benzyl ist und $R^2$ Methyl oder Benzyl ist, $R^5$ nicht $-OCH_3$, $-SCH_3$ oder $-SO-CH_3$ ist,

$R^{10}$ Wasserstoff, $C_1-C_{12}$-Alkyl, Halogen oder $C_2-C_8$-Alkanoyl bedeutet,

$R^{11}$ Wasserstoff, $C_1-C_8$-Alkyl, $C_3-C_5$-Alkenyl, $C_7-C_9$-Phenylalkyl, $C_1-C_4$-Hydroxyalkyl oder Phenyl bedeutet,

$R^{12}$ unverzweigtes oder verzweigtes $C_2-C_{16}$-Alkylen, das durch ein oder mehrere $-O-$, $-S-$ oder $-N(R^{11})-$ unterbrochen sein kann,

$R^{13}$ Wasserstoff, $C_1-C_8$-Alkyl oder Phenyl bedeutet,

$R^{14}$, $R^{15}$ und $R^{16}$ unabhängig voneinander Wasserstoff oder $C_1-C_4$-Alkyl bedeuten oder $R^{14}$ und $R^{15}$ zusammen $C_3-C_7$-Alkylen sind,

$R^{17}$ Wasserstoff, $C_1-C_{12}$-Alkyl, das durch ein oder mehrere -O- unterbrochen sein kann, $C_3-C_5$-Alkenyl, $C_7-C_9$-Phenylalkyl, $C_1-C_4$-Hydroxyalkyl, $-CH_2CH_2CN$, $-CH_2CH_2COO(C_1-C_4$-Alkyl), $C_2-C_8$-Alkanoyl oder Benzoyl bedeutet,

$R^{18}$ Wasserstoff, $C_1-C_{12}$-Alkyl, durch -CN, -OH, $C_1-C_4$-Alkoxy, $C_3-C_6$-Alkenoxy, $-OCH_2CH_2CN$, $-OCH_2CH_2COO(C_1-C_4$-Alkyl), -COOH oder $-COO(C_1-C_4$-Alkyl) substituiertes $C_1-C_6$-Alkyl, $-(CH_2CH_2O)_nH$ mit n = 2-20, $C_2-C_8$-Alkanoyl, $C_3-C_{12}$-Alkenyl, Cyclohexyl, Hydroxycyclohexyl, Phenyl, durch Halogen, $C_1-C_{12}$-Alkyl oder $C_1-C_4$-Alkoxy substituiertes Phenyl, $C_7-C_9$-Phenylalkyl oder $-Si(C_1-C_8 Alkyl)_r(Phenyl)_{3-r}$ mit r = 1, 2 oder 3 bedeutet,

$R^{19}$ Wasserstoff, $C_1-C_{12}$-Alkyl, $C_3-C_{12}$-Alkenyl, Cyclohexyl, durch -SH, -OH, -CN, $-COO(C_1-C_4$-Alkyl), $C_1-C_4$-Alkoxy, $-OCH_2CH_2CN$ oder $-OCH_2CH_2COO(C_1-C_4$-Alkyl) substituiertes $C_1-C_6$-Alkyl, Phenyl, durch Halogen, $C_1-C_{12}$-Alkyl oder $C_1-C_4$-Alkoxy substituiertes Phenyl oder $C_7-C_9$-Phenylalkyl bedeutet,

$R^{20}$ und $R^{21}$ unabhängig voneinander Wasserstoff, $C_1-C_{12}$-Alkyl, $C_2-C_4$-Hydroxyalkyl, $C_2-C_{10}$-Alkoxyalkyl, $C_3-C_5$-Alkenyl, $C_5-C_{12}$-Cycloalkyl, $C_7-C_9$-Phenylalkyl, Phenyl, durch Halogen, $C_1-C_{12}$-Alkyl oder $C_1-C_4$-Alkoxy substituiertes Phenyl, $C_2-C_3$-Alkanoyl oder Benzoyl bedeuten, oder $R^{20}$ und $R^{21}$ zusammen $C_2-C_8$-Alkylen bedeuten, das durch -O-, -S- oder $-N(R^{17})-$ unterbrochen sein kann, oder durch Hydroxy, $C_1-C_4$-Alkoxy oder $-COO(C_1-C_4$-Alkyl) substituiert sein kann,

$R^{22}$ $C_1-C_{18}$-Alkyl, unsubstituiertes oder durch Halogen, $C_1-C_{12}$-Alkyl oder $C_1-C_8$-Alkoxy substituiertes Phenyl oder Naphthyl bedeutet, oder um ein Säureadditionssalz einer solchen Verbindung,

insbesondere um Verbindungen der Formel I, worin $Ar^1$ eine Gruppe der Formel IV, V oder VII ist und $R^1$, $R^2$, $R^3$, $R^4$, $R^5$, $R^6$, $R^7$, $R^8$, $R^9$, $R^{10}$, V, U und W die oben gegebene Bedeutung haben.

$R^{14}$, $R^{15}$ und $R^{16}$ als $C_1-C_4$-Alkyl können z.B. Methyl, Ethyl, Propyl, Isopropyl, Butyl, Isobutyl,sec.Butyl oder tert.Butyl sein.

$R^2$, $R^{11}$ und $R^{13}$ als $C_1-C_8$-Alkyl können darüber hinaus auch z.B. Pentyl, Hexyl, Heptyl, Octyl, 2-Ethylhexyl oder 2,2,4,4-Tetramethylbutyl sein. $R^3$, $R^4$, $R^5$, $R^6$, $R^7$, $R^8$, $R^9$, $R^{10}$, $R^{17}$, $R^{18}$, $R^{19}$, $R^{20}$ und $R^{21}$ als $C_1-C_{12}$-Alkyl können darüber hinaus auch z.B. Nonyl, Decyl, Isodecyl, Undecyl oder Dodecyl sein.

$R^3$, $R^4$, $R^{11}$, $R^{17}$, $R^{20}$ und $R^{21}$ als $C_3-C_5$-Alkenyl können z.B. Allyl, Methallyl, Crotyl oder Dimethylallyl sein, wobei Allyl bevorzugt ist. $R^{18}$ und $R^{19}$ als $C_3-C_{12}$-Alkenyl können darüber hinaus auch z.B. Hexenyl, Octenyl oder Decenyl sein.

$R^2$, $R^5$, $R^6$, $R^7$, $R^8$ und $R^9$ als $C_5-C_6$-Cycloalkyl sind insbesondere Cyclohexyl. $R^3$, $R^4$, $R^{20}$ und $R^{21}$ als $C_5-C_{12}$-Cycloalkyl können darüber hinaus auch z.B. Cyclooctyl oder Cyclododecyl sein.

$R^3$, $R^4$, $R^{17}$, $R^{18}$, $R^{19}$, $R^{20}$ und $R^{21}$ als $C_7-C_9$-Phenylalkyl sind insbesondere Benzyl.

Y als $C_1-C_6$-Alkylen kann z.B. Methylen, Di-, Tri-, Tetra-, Penta- oder Hexamethylen sein. W als $C_1-C_7$-Alkylen kann z.B. Methylen, Ethylen, Propylen-1,2 oder Hexylen-1,2 sein.

V und W als $C_2-C_6$-Alkyliden können z.B. Ethyliden, Propyliden, Butyliden, Isobutyliden oder Hexyliden sein.

Beispiele für $Ar^2$ sind die Gruppen Phenyl, 1-Naphthyl, 2-Naphthyl, 2-Furyl, 2-Thienyl, 3-Pyridyl, 4-Chlorphenyl, Tolyl, 4-Isopropylphenyl, 4-Octylphenyl, 3-Methoxyphenyl, 4-Phenoxyphenyl, 4-Phenylphenyl, 4-Benzoylphenyl, 4-Chlor-1-naphthyl oder 4-Methyl-2-pyridyl.

Beispiele für $R^2$ als substituiertes Alkyl sind die Gruppen 2-Methoxyethyl, 3-Butoxypropyl, 2-Isopropoxyethyl, 4-Phenoxybutyl, 2-Chlorethyl, 3-Chlorpropyl, 2-Phenylethyl oder 3-Phenylpropyl. Beispiele für $R^2$ als substituiertes Phenyl sind die Gruppen 4-Chlorphenyl, 3-Methoxyphenyl, 4-Tolyl oder 4-Butylphenyl.

$R^3$ und $R^4$ als substituiertes Alkyl können z.B. 2-Hydroxyethyl, 2-Hydroxypropyl, 2-Hydroxyisobutyl, 2-Ethoxyethyl, 2-Methoxypropyl, 2-Butoxyethyl, 2-Cyanethyl, 2-Ethoxycarbonylethyl oder 2-Methoxycarbonylethyl sein.

$R^4$ als substituiertes Phenyl kann z.B. 3-Chlorphenyl, 4-Chlorphenyl, 4-Tolyl, 4-tert.Butylphenyl, 4-Dodecylphenyl, 3-Methoxyphenyl oder 3-Methoxycarbonylphenyl sein.

Wenn $R^4$ zusammen mit $R^2$ Alkylen oder Phenylalkylen bedeuten, so ergeben diese zusammen mit dem C-Atom und dem N-Atom, an das sie gebunden sind vorzugsweise einen 5- oder 6-gliedrigen heterocyclischen Ring.

Wenn $R^3$ und $R^4$ zusammen Alkylen oder unterbrochenes Alkylen bedeuten, so ergeben diese zusammen mit dem N‑Atom, an das sie gebunden sind, vorzugsweise einen 5‑ oder 6‑gliedrigen heterocyclischen Ring, z.B. einen Pyrrolidin‑, Piperidin‑, Morpholin‑, Thiomorpholin‑, Piperidon‑ oder Piperazinring, der durch eine oder mehrere Alkyl‑, Hydroxy, Alkoxy‑ oder Estergruppen substituiert sein kann,

$R^{10}$, $R^{17}$ und $R^{18}$ als $C_2-C_8$‑Alkanoyl können z.B. Propionyl, Butyryl, Isobutyryl, Hexanoyl oder Octanoyl, insbesondere aber Acetyl sein.

$R^{11}$, $R^{17}$, $R^{20}$ und $R^{21}$ als $C_1-C_4$‑Hydroxyalkyl bzw. $C_2-C_4$‑Hydroxyalkyl können z.B. Hydroxymethyl, 2‑Hydroxyethyl, 2‑Hydroxypropyl oder 4‑Hydroxybutyl sein.

$R^{12}$ als Alkylen oder unterbrochenes Alkylen kann z.B. Ethylen, Tri‑, Tetra‑, Penta‑, Hexa‑, Octa‑ oder Dodecamethylen, 2,2‑Dimethyl‑trimethylen,1,3,3‑Trimethyltetramethylen, 3‑Oxa‑pentamethylen, 3‑Oxaheptamethylen, 4,7‑Dioxa‑decamethylen, 4,9‑Dioxadodecamethylen, 3,6,9,12‑Tetraoxa‑tetradecamethylen, 4‑Aza‑heptamethylen, 4,7‑Di(methylaza)‑decamethylen oder 4‑Thia‑heptamethylen sein.

Wenn $R^{14}$ und $R^{15}$ zusammen $C_3-C_7$‑Alkylen bedeuten, so bedeuten sie insbesondere 1,3‑ oder 1,4‑Alkylen wie z.B. 1,3‑Propylen, 1,3‑Butylen, 2,4‑Pentylen, 1,3‑Hexylen, 1,4‑Butylen, 1,4‑Pentylen oder 2,4‑Hexylen.

$R^{18}$, $R^{19}$, $R^{20}$ und $R^{21}$ als substituiertes Phenyl kann z.B. 4‑Chlorphenyl, 3‑Chlorphenyl, 4‑Tolyl, 4‑tert.Butylphenyl, 4‑Nonylphenyl, 4‑Dodecylphenyl, 3‑Methoxyphenyl oder 4‑Ethoxyphenyl sein.

$R^{18}$ als Gruppe $-Si(C_1-C_8\text{-Alkyl})_r\,(\text{Phenyl})_{3-r}$ kann insbesondere $-Si(CH_3)_3$, $-Si(\text{Phenyl})_2CH_3$, $-Si(CH_3)_2$ Phenyl, $-Si(CH_3)_2[C(CH_3)_2CH(CH_3)_2]$ und $-Si(\text{Phenyl})_3$ sein.

$R^{18}$ als substituiertes $C_1-C_6$‑Alkyl kann z.B. 2‑Hydroxyethyl, 2‑Methoxyethyl oder 2‑Allyloxyethyl sein.

$R^{19}$ als substituiertes $C_1-C_6$‑Alkyl kann z.B. 2‑Mercaptoethyl, 2‑Hydroxyethyl, 2‑Hydroxypropyl, 2‑Methoxyethyl, $-CH_2CH_2OCH_2CH_2CN$ oder $-CH_2CH_2OCH_2CH_2COOCH_3$ sein.

$R^{20}$ und $R^{21}$ als Alkoxyalkyl können z.B. Methoxyethyl, Ethoxyethyl, 2‑Ethoxypropyl, 2‑Butoxyethyl, 3‑Methoxypropyl oder 2‑Hexyloxyethyl sein.

$R^{20}$ und $R^{21}$ als $C_2-C_3$‑Alkanoyl sind insbesondere Acetyl.

$R^{22}$ als substituiertes Phenyl oder Naphthyl kann z.B. 4‑Tolyl, 4‑Bromphenyl, 3‑Chlorphenyl, 4‑Butylphenyl, 4‑Octylphenyl, 4‑Decylphenyl, 4‑Dodecylphenyl, 3‑Methoxyphenyl, 4‑Isopropoxyphenyl, 4‑Butoxyphenyl, 4‑Octyloxyphenyl, Chlornaphthyl, Nonylnaphthyl oder Dedecylnaphthyl sein.

Wenn $R^{20}$ und $R^{21}$ zusammen Alkylen oder unterbrochenes Alkylen bedeuten, so bildet dieses zusammen mit dem N‑Atom, an das es gebunden ist, einen heterocyclischen Ring, vorzugsweise einen 5‑oder 6‑gliedrigen Ring, der durch Alkyl‑, Hydroxy‑, Alkoxy‑ oder Estergruppen substituiert sein kann. Beispiele für solche Ringe sind ein Pyrrolidin‑, Piperidin‑, 4‑Hydroxypiperidin‑, 3‑Ethoxycarbonyl‑piperidin‑, Morpholin‑, 2,6‑Dimethylmorpholin‑, Piperazin‑oder 4‑Methylpiperazinring.

Alle diese Verbindungen besitzen mindestens eine basische Aminogruppe und lassen sich daher durch Addition von Säuren in die entsprechenden Salze überführen. Die Säuren können anorganische oder organische Säuren sein. Beispiele für solche Säuren sind HCl, HBr, $H_2SO_4$, $H_3PO_4$, Mono‑ oder Polycarbonsäuren wie z.B. Essigsäure, Oelsäure, Bernsteinsäure, Sebacinsäure, Weinsäure oder $CF_3COOH$, Sulfonsäuren wie z.B. $CH_3SO_3H$, $C_{12}H_{25}SO_3H$, $p-C_{12}H_{25}-C_6H_4-SO_3H$, $p-CH_3-C_6H_4-SO_3H$ oder $CF_3SO_3H$.

Bevorzugt sind Verbindungen der Formel I, worin $Ar^1$ eine Gruppe der Formel IV ist, $R^5$ und $R^6$ Wasserstoff, Halogen, $C_1-C_{12}$‑Alkyl oder eine Gruppe $-OR^{18}$, $-SR^{19}$, $-SOR^{19}$, $-SO_2-R^{19}$, $-N(R^{20})-(R^{21})$, $-NHSO_2R^{22}$ oder

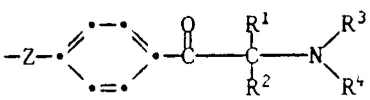

bedeuten, worin Z $-O-$, $-S-$, $-N(R^{11})-$ oder $-N(R^{11})-R^{12}-N(R^{11})-$bedeutet, $R^7$ und $R^8$ Wasserstoff oder Halogen und $R^9$ Wasserstoff, Halogen oder $C_1-C_{12}$‑Alkyl sind und $R^1$, $R^2$, $R^3$, $R^4$, $R^{11}$, $R^{12}$, $R^{18}$, $R^{19}$, $R^{20}$, $R^{21}$ und $R^{22}$ die oben gegebenen Bedeutungen haben, wobei im Falle, dass $R^1$ Allyl ist und $R^2$ Methyl ist, $R^5$ nicht $-OCH_3$ ist, und im Falle, dass $R^1$ Benzyl ist und $R^2$ Methyl oder Benzyl ist, $R^5$ nicht $-OCH_3$, $-SCH_3$ oder $-SOCH_3$ ist.

Unter den Verbindungen der Formel I, worin $Ar^1$ eine Gruppe der Formel IV ist, in der $R^5$ eine Gruppe $-OR^{18}$, $-SR^{19}$, $-N(R^{20})(R^{21})$ oder

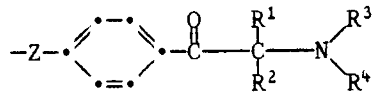

bedeutet, sind diejenigen bevorzugt, bei denen $R^6$ Wasserstoff, Halogen oder $C_1-C_4$-Alkyl bedeutet oder eine der für $R^5$ gegebenen Bedeutungen hat, $R^7$ und $R^8$ Wasserstoff oder Halogen und $R^9$ Wasserstoff oder $C_1-C_4$-Alkyl bedeuten, Z $-O-$, $-S-$ oder $-N(R^{11})-$ bedeutet,
$R^1$ entweder
(a) ein Rest der Formel

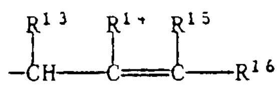

ist oder
(b) ein Rest der Formel $-CH(R^{13})-Ar^2$ ist, worin $Ar^2$ ein unsubstituierter oder durch Halogen, $C_1-C_4$-Alkyl Methylthio, Methoxy oder Benzoyl substituierter Phenylrest ist,
$R^2$ eine der für $R^1$ gegebenen Bedeutungen hat oder $C_1-C_6$-Alkyl ist,
$R^3$ und $R^4$ unabhängig voneinander $C_1-C_{12}$-Alkyl, durch $C_1-C_4$-Alkoxy, $-CN$ oder $-COO(C_1-C_4$-Alkyl) substituiertes $C_2-C_4$-Alkyl, Allyl, Cyclohexyl oder Benzyl bedeuten oder $R^3$ und $R^4$ zusammen $C_4-C_6$-Alkylen bedeuten, welches durch $-O-$ oder $-N(R^{17})-$ unterbrochen sein kann,
$R^{11}$ Wasserstoff, $C_1-C_4$-Alkyl, Allyl, Benzyl oder $C_2-C_4$-Alkanoyl bedeutet,
$R^{12}$ $C_2-C_6$-Alkylen bedeutet,
$R^{13}$, $R^{14}$, $R^{15}$ und $R^{16}$ unabhängig voneinander Wasserstoff oder Methyl bedeuten,
$R^{17}$ Wasserstoff, $C_1-C_4$-Alkyl, Benzyl, 2-Hydroxyethyl oder Acetyl bedeutet,
$R^{18}$ Wasserstoff, $C_1-C_4$-Alkyl, 2-Hydroxyethyl, 2-Methoxyethyl, 2-Allyloxyethyl, Allyl, Cyclohexyl, Phenyl, Benzyl oder $-Si(CH_3)_3$ bedeutet,
$R^{19}$ Wasserstoff, $C_1-C_{12}$-Alkyl, 2-Hydroxyethyl, 2-Methoxyethyl, Phenyl, p-Tolyl oder Benzyl bedeutet,
$R^{20}$ und $R^{21}$ unabhängig voneinander Wasserstoff, $C_1-C_{12}$-Alkyl, $C_2-C_6$-Alkoxy-alkyl, Acetyl, Allyl oder Benzyl bedeuten oder $R^{20}$ und $R^{21}$ zusammen $C_4-C_6$-Alkylen bedeuten, das durch $-O-$ oder $-N(R^{17})-$ unterbrochen sein kann,
wobei im Falle, dass $R^1$ Allyl ist, $R^5$ nicht $-OCH_3$ ist, und im Falle, dass $R^1$ Benzyl ist und $R^2$ Methyl oder Benzyl ist, $R^5$ nicht $-OCH_3$ oder $-SCH_3$ ist.

Besonders bevorzugt sind Verbindungen der Formel I, worin $Ar^1$ eine Gruppe der Formel IV ist, in der $R^5$ eine Gruppe $-OR^{18}$, $-SR^{19}$ oder $-N(R^{20})(R^{21})$ bedeutet, $R^6$ Wasserstoff, Chlor oder $C_1-C_4$-Alkyl bedeutet oder eine der für $R^5$ gegebenen Bedeutungen hat, $R^7$ und $R^8$ Wasserstoff oder Chlor und $R^9$ Wasserstoff oder $C_1-C_4$-Alkyl bedeuten, $R^1$ entweder (a) ein Rest der Formel $-CH_2-C(R^{14})=CH(R^{15})$ ist oder (b) ein Rest der Formel $-CH_2-Ar^2$ ist,
worin $Ar^2$ ein unsubstituierter oder durch Halogen, $C_1-C_4$-Alkyl, $CH_3S-$, $CH_3O-$ oder Benzyl substituierter Phenylrest ist,
$R^2$ eine der für $R^1$ gegebenen Bedeutungen hat oder $C_1-C_4$-Alkyl ist,
$R^3$ und $R^4$ unabhängig voneinander $C_1-C_6$-Alkyl, 2-Methoxyethyl, Allyl oder Benzyl sind oder $R^3$ und $R^4$ zusammen Tetramethylen, Pentamethylen oder 3-Oxapentamethylen bedeuten,
$R^{14}$ und $R^{15}$ Wasserstoff oder Methyl bedeuten,
$R^{18}$ $C_1-C_4$-Alkyl, 2-Hydroxyethyl, 2-Methoxyethyl oder Phenyl bedeutet,
$R^{19}$ $C_1-C_{12}$-Alkyl, 2-Hydroxyethyl, 2-Methoxyethyl, Phenyl oder p-Tolyl bedeutet,
$R^{20}$ und $R^{21}$ Wasserstoff, $C_1-C_4$-Alkyl, 2-Methoxyethyl, Acetyl oder Allyl bedeuten oder
$R^{20}$ und $R^{21}$ zusammen $C_4-C_5$-Alkylen bedeuten, das durch $-O-$ oder $-N(CH_3)-$ unterbrochen sein kann,
wobei im Falle, dass $R^1$ Allyl ist, $R^5$ nicht $-OCH_3$ ist, und im Falle, dass $R^1$ Benzyl ist und $R^2$ Methyl oder Benzyl ist, $R^5$ nicht $-OCH_3$ oder $-SCH_3$ ist.

Unter diesen Verbindungen sind solche bevorzugt, worin $R^5$ eine Gruppe $-SR^{19}$ ist, $R^1$ ein Rest der Formel

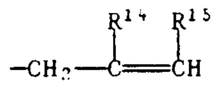

ist, und entweder $R^7$ und $R^8$ Wasserstoff sind oder $R^6$, $R^7$, $R^8$ und $R^9$ Wasserstoff sind, sowie solche, worin $R^1$ Allyl ist.

Unter den Verbindungen der Formel I, worin $Ar^1$ eine Gruppe der Formel IV ist, in der $R^5$ eine Gruppe $-N(R^{20})(R^{21})$ ist, sind diejenigen bevorzugt, bei denen $R^7$ und $R^8$ Wasserstoff sind, sowie diejenigen, bei denen $R^6$, $R^7$, $R^8$ und $R^9$ Wasserstoff sind, sowie diejenigen, bei denen $R^1$ Allyl oder Benzyl ist.

Bevorzugt sind weiterhin Verbindungen der Formel I, worin $Ar^1$ eine Gruppe der Formel IV ist, in der $R^5$ Wasserstoff, Halogen oder $C_1-C_{12}$-Alkyl ist und $R^6$, $R^7$, $R^8$ und $R^9$ Wasserstoff sind, $R^1$ Allyl oder Benzyl bedeutet, $R^2$ $C_1-C_6$-Alkyl, Allyl oder Benzyl bedeutet, $R^3$ und $R^4$ unabhängig voneinander $C_1-C_{12}$-Alkyl, durch $C_1-C_4$-Alkoxy, -CN oder $-COO(C_1-C_4$-Alkyl) substituiertes $C_2-C_4$-Alkyl, Allyl, Cyclohexyl oder Benzyl bedeuten oder $R^3$ und $R^4$ zusammen $C_4-C_6$-Alkylen bedeuten, welches durch -O- oder $-N(R^{17})$- unterbrochen sein kann, und $R^{17}$ Wasserstoff, $C_1-C_4$-Alkyl oder 2-Hydroxyethyl bedeutet.

Beispiele für einzelne Verbindungen der Formel I sind:

1. 2-(Dimethylamino)-2-ethyl-1-(4-morpholinophenyl)-4-penten-1-on
2. 2-(Dimethylamino)-2-methyl-1-(4-morpholinophenyl)-4-penten-1-on
3. 2-Benzyl-2-(dimethylamino)-1-(4-morpholinophenyl)-propan-1-on
4. 4-Morpholino-4-(4-morpholinobenzyl)-hepta-1,6-dien
5. 2-Ethyl-2-morpholino-1-(4-morpholinophenyl)-4-penten-1-on
6. 2-Benzyl-2-(dimethylamino)-1-[4-(dimethylamino)-phenyl]-butan-1-on
7. 4-Dimethylamino-4-(4-dimethylaminobenzoyl]-hepta-1,6-dien
8. 4-(Dimethylamino)-4-(4-morpholinobenzoyl)-hepta-1,6-dien
9. 2-(Dimethylamino)-2-(4-dimethylaminophenyl)-2-ethyl-4-penten-1-on
10. 2-Benzyl-2-(dimethylamino)-1-(4-morpholinophenyl)-butan-1-on
11. 2-Benzyl-2-(dimethylamino)-1-[4-(dimethylamino)-phenyl]-4-penten-1-on
12. 2-Benzyl-1-[4-(dimethylamino)-phenyl]-2-(dimethylamino)-3-phenyl-propan-1-on
13. 2-Ethyl-1-[4-(methylthio)-phenyl]-2-morpholino-4-penten-1-on
14. 4-[4-(Methylthio)-benzoyl]-4-morpholino-hepta-1,6-dien
15. 4-(Dimethylamino)-4-(4-methoxybenzoyl)-hepta-1,6-dien
16. 4-(4-Methoxybenzoyl)-4-morpholino-hepta-1,6-dien
17. 1-(4-Methoxyphenyl)-2-morpholino-2-phenyl-4-penten-1-on
18. 2-Ethyl-1-(4-methoxyphenyl)-2-morpholino-4-penten-1-on
19. 2-Benzyl-2-(dimethylamino)-1-[4-(methylthio)-phenyl]-butan-1-on
20. 2-(Dimethylamino)-2-ethyl-1-[4-(methylthio)-phenyl]-4-penten-1-on
21. 2-Benzyl-2-(dimethylamino)-1-[4-(methylthio)-phenyl]-4-penten-1-on
22. 4-(Dimethylamino)-4-[4-(methylthio)-benzoyl]-1,6-heptadien
23. 2-(Dimethylamino)-3-(4-fluorphenyl)-2-methyl-1-[4-(methylthio)-phenyl]-propan-1-on
24. 3-(4-Chlorphenyl)-2-(dimethylamino)-2-methyl-1-[4-(methylthio)-phenyl]-propan-1-on
25. 3-(2-Chlorphenyl)-2-(dimethylamino)-2-methyl-1-[4-(methylthio)-phenyl]-propan-1-on
26. 3-(4-Bromphenyl)-2-(dimethylamino)-2-methyl-1-[4-(methylthio)-phenyl]-propan-1-on
27. 2-Ethyl-4-methyl-1-[4-(methylthio)-phenyl]-2-morpholino-4-penten-1-on
28. 2-Ethyl-1-[4-(methylthio)-phenyl]-2-morpholino-4-hexen-1-on
29. 2-Benzyl-1-[4-(methylthio)-phenyl]-2-morpholino-4-penten-1-on
30. 2-Allyl-1-[4-(methylthio)-phenyl]-2-morpholino-hexan-1-on
31. 2-(Dimethylamino)-1-[4-(methylthio)-phenyl]-2-methyl-3-(4-methylphenyl)-propan-1-on
32. 2-(Dimethylamino)-1,3-bis-[4-(methylthio)-phenyl]-2-methyl-propan-1-on
33. 2-(Dimethylamino)-1-[4-(methylthio)-phenyl]-2-methyl-3-(4-methoxyphenyl)-propan-1-on
34. 1-[4-(Methylthio)-phenyl]-2-methyl-2-morpholino-4-penten-1-on

35. 2 – (Dimethylamino) – 1 – [4 – (methylthio) – phenyl] – 2 – methyl – 4 – penten – 1 – on
36. 1 – [4 – (Methylthio) – phenyl] – 2 – morpholino – 2 – phenyl – 4 – penten – 1 – on
37. 2 – (Dimethylamino) – 1 – [4 – (methylthio) – phenyl] – 2 – phenyl – 4 – penten – 1 – on
38. 2 – (Dimethylamino) – 1 – [4 – (methylthio) – phenyl] – 2,3 – diphenyl – propan – 1 – on
39. 2 – Methyl – 2 – morpholino – 1 – (4 – morpholinophenyl) – 4 – penten – 1 – on
40. 2 – Benzyl – 2 – morpholino – 1 – (4 – morpholinophenyl) – 4 – penten – 1 – on
41. 2 – Ethyl – 2 – morpholino – 1 – (4 – morpholinophenyl) – 4 – penten – 1 – on
42. 1 – [4 – (Dimethylamino) – phenyl] – 2 – methyl – 2 – morpholino – 4 – penten – 1 – on
43. 2 – Benzyl – 2 – (dimethylamino) – 1 – (4 – morpholinophenyl) – 3 – phenyl – propan – 1 – on
44. 4 – [4 – (Dimethylamino) – benzoyl] – 4 – morpholino – hepta – 1,6 – dien
45. 2 – (Dimethylamino) – 1 – [4 – (dimethylamino) – phenyl] – 2 – methyl – 4 – penten – 1 – on
46. 2 – Benzyl – 2 – (dimethylamino) – 1 – (4 – morpholinophenyl) – 4 – penten – 1 – on
47. 1 – [4 – (Dimethylamino) – phenyl] – 2 – ethyl – 4 – methyl – 2 – morpholino – 4 – penten – 1 – on
48. 1 – [4 – (Dimethylamino) – phenyl] – 2 – ethyl – 2 – morpholino – 4 – hexen – 1 – on
49. 2 – Ethyl – 2 – morpholino – 1 – (4 – morpholinophenyl) – 4 – hexen – 1 – on
50. 2 – Ethyl – 4 – methyl – 2 – morpholino – 1 – (4 – morpholinophenyl) – 4 – penten – 1 – on
51. 1 – [4 – (Bis – (2 – methoxyethyl)amino) – phenyl] – 2 – methyl – 2 – morpholino – 4 – penten – 1 – on
52. 1 – [4 – (Dibutylamino)phenyl] – 2 – methyl – 2 – morpholino – 4 – penten – 1 – on
53. 2 – Methyl – 1 – [4 – (4 – methyl – piperazin – 1 – yl) – phenyl] – 2 – morpholino – 4 – penten – 1 – on
54. 2 – Benzyl – 2 – (dimethylamino) – 1 – (4 – methoxyphenyl) – butan – 1 – on
55. 1 – [4 – (Diethylamino) – phenyl] – 2 – methyl – 2 – morpholino – 4 – penten – 1 – on
56. 2 – Methyl – 2 – morpholino – 1 – [4 – (pyrrolidin – 1 – yl) – phenyl] – 4 – penten – 1 – on
57. 2 – Benzyl – 2 – (dimethylamino) – 1 – (4 – piperidinophenyl) – butan – 1 – on
58. 2 – Ethyl – 2 – (dimethylamino) – 1 – (4 – piperidinophenyl) – 4 – penten – 1 – on
59. 2 – Benzyl – 1 – [4 – (diethylamino)phenyl] – 2 – ethyl – butan – 1 – on
60. 1 – [4 – (Diethylamino)phenyl] – 2 – ethyl – 4 – penten – 1 – on
61. 2 – Benzyl – 2 – (dimethylamino) – 1 – [4 – (2 – hydroxyethylthio) – phenyl] – butan – 1 – on
62. 2 – Ethyl – 1 – [4 – (2 – hydroxyethylthio) – phenyl] – 2 – morpholino – 4 – penten – 1 – on
63. 1 – [4 – (Diallylamino) – phenyl] – 2 – methyl – 2 – morpholino – 4 – penten – 1 – on
64. 3 – (4 – Benzoylphenyl) – 2 – (dimethylamino) – 2 – methyl – 1 – [4 – (methylthio) – phenyl] – propan – 1 – on
65. 2 – (Dimethylamino) – 3 – (3,4 – dimethoxyphenyl) – 2 – methyl – 1 – phenyl – propan – 1 – on
66. 2 – (Dimethylamino) – 3 – (3,4 – dimethoxyphenyl) – 2 – methyl – 1 – [4 – (methylthio) – phenyl] – propan – 1 – on
67. 3 – (4 – Benzoylphenyl) – 2 – (dimethylamino) – 2 – methyl – 1 – phenyl – propan – 1 – on
68. 2 – Benzyl – 2 – (dimethylamino) – 1 – (4 – fluorphenyl) – butan – 1 – on
69. 4 – (Dimethylamino) – 4 – (4 – fluorbenzoyl) – hepta – 1,6 – dien
70. 2 – Ethyl – 2 – morpholino – 1 – [4 – (4 – methylphenylsulfonyl) – phenyl] – 4 – penten – 1 – on
71. 2 – (Dimethylamino) – 2 – ethyl – 1 – [4 – (methylsulfonyl)phenyl] – 4 – penten – 1 – on
72. 2 – Benzyl – 2 – (dimethylamino) – 1 – [4 – (methylphenylsulfonyl)phenyl] – butan – 1 – on
73. 2 – Benzyl – 2 – (dimethylamino) – 1 – [4 – (methylsulfonyl)phenyl] – butan – 1 – on
74. 1 – (4 – Fluorphenyl) – 2 – methyl – 2 – morpholino – 4 – penten – 1 – on
75. 4 – (4 – Fluorbenzoyl) – 4 – morpholino – hepta – 1,6 – dien
76. 2 – Benzyl – 2 – (dimethylamino) – 1 – (4 – fluorphenyl) – 4 – penten – 1 – on
77. 2 – Benzyl – 2 – (dimethylamino) – 1 – (4 – fluorphenyl) – 3 – phenyl – propan – 1 – on
78. 2 – (Dimethylamino) – 2 – ethyl – 1 – (4 – fluorphenyl) – 4 – penten – 1 – on
79. 2 – Benzyl – 1 – (4 – fluorphenyl) – 2 – morpholino – 4 – penten – 1 – on
80. 2 – Ethyl – 1 – (4 – fluorphenyl) – 2 – morpholino – 4 – penten – 1 – on
81. 2 – Benzyl – 2 – (dimethylamino) – 1 – (4 – fluorphenyl) – propan – 1 – on
82. 2 – (Dimethylamino) – 1 – (4 – fluorphenyl) – 2 – methyl – 4 – penten – 1 – on
83. 2 – Benzyl – 2 – (dimethylamino) – 1 – (4 – hydroxyphenyl) – butan – 1 – on
84. 2 – Benzyl – 1 – [4 – (ethoxycarbonylmethyloxy)phenyl] – 2 – (dimethylamino) – butan – 1 – on
85. 2 – Benzyl – 2 – (dimethylamino) – 1 – [4 – (2 – hydroxyethyloxy)phenyl] – butan – 1 – on
86. 2 – Benzyl – 1 – (4 – chlorphenyl) – 2 – (dimethylamino) – butan – 1 – on
87. 2 – Benzyl – 1 – (4 – bromphenyl) – 2 – (dimethylamino) – butan – 1 – on
88. 1 – (4 – Bromphenyl) – 2 – ethyl – 2 – morpholino – 4 – penten – 1 – on
89. 2 – Ethyl – 1 – (4 – methoxyphenyl) – 2 – morpholino – 3 – penten – 1 – on
90. 2 – (Dimethylamino) – 2 – ethyl – 1 – (4 – methoxyphenyl) – 4 – penten – 1 – on

91. 2 – Benzyl – 1 – [4 – (dimethylamino)phenyl] – 2 – morpholino – 4 – penten – 1 – on
92. 2 – Benzyl – 2 – (dimethylamino) – 1 – [4 – (dimethylamino)phenyl] – propan – 1 – on
93. 2 – Methyl – 2 – morpholino – 1 – phenyl – 4 – penten – 1 – on
94. 2 – Benzyl – 2 – morpholino – 1 – phenyl – 4 – penten – 1 – on
95. 2 – (Dimethylamino) – 2 – methyl – 1 – phenyl – 4 – penten – 1 – on
96. 2 – Benzyl – 2 – (dimethylamino) – 1 – phenyl – propan – 1 – on
97. 4 – Benzoyl – 4 – (dimethylamino) – hepta – 1,6 – dien
98. 2 – Benzyl – 2 – (dimethylamino) – 1,3 – diphenyl – propan – 1 – on
99. 2 – Benzyl – 2 – (dimethylamino) – 1 – phenyl – 4 – penten – 1 – on
100. 2 – (Dimethylamino) – 2 – ethyl – 1 – phenyl – 4 – penten – 1 – on
101. 2 – Benzyl – 2 – (dimethylamino) – 1 – phenyl – butan – 1 – on
102. 1,2 – Diphenyl – 2 – morpholino – 4 – penten – 1 – on
103. 3 – (4 – Chlorphenyl) – 2 – (dimethylamino) – 2 – methyl – 1 – phenyl – propan – 1 – on
104. 3 – (4 – Bromphenyl) – 2 – (dimethylamino) – 2 – methyl – 1 – phenyl – propan – 1 – on
105. 3 – (2 – Chlorphenyl) – 2 – (dimethylamino) – 2 – methyl – 1 – phenyl – propan – 1 – on
106. 3 – (3,4 – Dimethoxyphenyl) – 2 – (dimethylamino) – 2 – methyl – 1 – phenyl – propan – 1 – on
107. 2 – (Dimethylamino) – 2 – methyl – 3 – (4 – methylphenyl) – 1 – phenyl – propan – 1 – on
108. 2 – (Dimethylamino) – 2 – methyl – 3 – [4 – (methylthio) – henyl] – 1 – phenyl – propan – 1 – on
109. 2 – (Dimethylamino) – 3 – (4 – fluorphenyl) – 2 – methyl – 1 – phenyl – propan – 1 – on
110. 2 – (Dimethylamino) – 3 – (4 – methoxy – phenyl) – 2 – methyl – 1 – phenyl – propan – 1 – on
111. 2 – Ethyl – 1 – (4 – fluorphenyl) – 4 – methyl – 2 – morpholino – 4 – penten – 1 – on
112. 2 – Ethyl – 1 – (4 – fluorphenyl) – 5 – methyl – 2 – morpholino – 4 – penten – 1 – on
113. 2 – (Benzylmethylamino) – 2 – ethyl – 1 – (4 – morpholinophenyl) – 4 – penten – 1 – on
114. 2 – (Allylmethylamino) – 2 – ethyl – 1 – (4 – morpholinophenyl) – 4 – penten – 1 – on
115. 2 – Benzyl – 2 – (benzylmethylamino) – 1 – (4 – morpholinophenyl) – 4 – butan – 1 – on
116. 2 – Benzyl – 2 – (butylmethylamino) – 1 – (4 – morpholinophenyl) – 4 – butan – 1 – on
117. 2 – (Butylmethylamino) – 1 – (4 – morpholinophenyl) – 4 – penten – 1 – on
118. 1 – (4 – Acetylaminophenyl) – 2 – benzyl – 2 – dimethylamino – butan – 1 – on
119. 2 – Benzyl – 2 – dimethylamino – 1 – (4 – morpholinophenyl) – pentan – 1 – on
120. 2 – Allyl – 2 – dimethylamino – 1 – (4 – morpholinophenyl) – pentan – 1 – on
121. 2 – Morpholino – 1 – [4 – (2 – methoxyethyloxy)phenyl] – 2 – methyl – 4 – penten – on
122. 4 – Morpholino – 4 – [4 – (2 – hydroxyethylthio)benzoyl] – 5 – methyl – 1 – hexen
123. 1 – (4 – Bromphenyl) – 2 – morpholino – 2 – methyl – 4 – penten – 1 – on
124. 4 – (4 – Brombenzoyl) – 4 – morpholino – 5 – methyl – 1 – hexen
125. 1 – (4 – [2 – Hydroxyethylthio]phenyl) – 2 – methyl – 2 – morpholino – 4 – penten – 1 – on
126. 1 – (4 – [2 – (Allyloxy) – ethoxy]phenyl) – 2 – ethyl – 2 – morpholino – 4 – penten – 1 – on
127. 1 – (4 – [2 – (Allyloxy) – ethoxy]phenyl) – 2 – methyl – 2 – morpholino – 4 – penten – 1 – on
128. 1 – (4 – [2 – (Methoxy) – ethoxy]phenyl) – 2 – ethyl – 2 – morpholino – 4 – penten – 1 – on
129. 2 – Benzyl – 2 – dimethylamino – 1 – [4 – (2 – methoxyethylamino)phenyl] – butan – 1 – on
130. 2 – Benzyl – 2 – dimethylamino – 1 – (4 – methylaminophenyl) – butan – 1 – on
131. 2 – Benzyl – 2 – dimethylamino – 1 – [4 – (N – acetylmethylamino)phenyl] – butan – 1 – on
132. 2 – Benzyl – 2 – diethylamino – 1 – (4 – morpholino – phenyl) – butan – 1 – on
133. 2 – Diethylamino – 2 – ethyl – 1 – (4 – morpholino – phenyl) – 4 – penten – 1 – on
140. 2 – Benzyl – 2 – (dimethylamino) – 1 – (3,5 – dimethyl – 4 – methoxy – phenyl) – butan – 1 – on
141. 2 – Benzyl – 1 – (2,4 – dichlorphenyl) – 2 – (dimethylamino) – butan – 1 – on
142. 2 – (Dimethylamino) – 2 – ethyl – 1 – (3,4 – dichlorphenyl) – 4 – penten – 1 – on
143. 2 – Benzyl – 1 – (3,4 – dichlorphenyl) – 2 – (dimethylamino) – butan – 1 – on
144. 1 – (3 – Chlor – 4 – morpholino – phenyl) – 2 – (dimethylamino) – butan – 1 – on
145. 2 – Benzyl – 1 – (3 – Chlor – 4 – morpholino – phenyl) – 2 – (dimethylamino) – butan – 1 – on
146. 2 – Benzyl – 2 – dimethylamino – 1 – (4 – dimethylamino – 3 – ethyl) – butan – 1 – on
147. 2 – Benzyl – 2 – dimethylamino – 1 – (4 – dimethylamino – 2 – methyl – phenyl) – butan – 1 – on
148. 9 – Butyl – 3,6 – di(2 – benzyl – 2 – dimethylamino – butyryl)carbazol
149. 9 – Butyl – 3,6 – di(2 – methyl – 2 – morpholino – 4 – penten – 1 – on – 1 – yl) – carbazol
150. 2 – Benzyl – 2 – dimethylamino – 1 – (4 – morpholinophenyl) – butan – 1 – on – Trifluoracetat
151. 2 – Benzyl – 2 – dimethylamino – 1 – (4 – morpholinophenyl) – butan – 1 – on – p – Toluolsulfonat
152. 2 – Benzyl – 2 – dimethylamino – 1 – (4 – morpholinophenyl) – butan – 1 – on – Camphersulfonat
153. 4 – (Dimethylamino) – 4 – [4 – (phenyloxy) – benzoyl] – hepta – 1,6 – dien
154. 2 – Benzyl – 2 – (dimethylamino) – 1 – (4 – isopropyloxyphenyl) – 4 – penten – 1 – on

155. 1 – (4 – Butyloxyphenyl) – 2 – ethyl – 2 – morpholino – 4 – penten – 1 – on
156. 1 – (4 – Allyloxyphenyl) – 2 – benzyl – 2 – (dimethylamino) – butan – 1 – on
157. 2 – Methyl – 2 – morpholino – 1 – [4 – (trimethylsilyloxy) – phenyl] – 4 – penten – 1 – on
158. 2 – Benzyl – 2 – (dimethylamino) – 1 – [4 – ((1,1,2 – trimethylpropyl – dimethyl) – silyloxy) – phenyl] – butan – 2 – on
159. 2 – Ethyl – 1 – [4 – (ethylthio) – phenyl] – 2 – morpholino – 4 – penten – 1 – on
160. 2 – Benzyl – 1 – [4 – (butylthio) – phenyl] – 2 – (dimethylamino) – 3 – phenyl – propan – 1 – on
161. 1 – [4 – (Isopropylthio) – phenyl] – 2 – methyl – 2 – morpholino – 4 – penten – 1 – on
162. 1 – [4 – (Allylthio) – phenyl] – 2 – ethyl – 2 – morpholino – 4 – penten – 1 – on
163. 1 – [4 – (Benzylthio) – phenyl] – 2 – benzyl – 2 – (dimethylamino) – propan – 1 – on
164. 2 – Benzyl – 2 – (dimethylamino) – 1 – (4 – mercaptophenyl) – butan – 1 – on
165. 2 – Benzyl – 1 – [4 – (cyclohexylthio) – phenyl] – 2 – (dimethylamino) – 3 – phenyl – propan – 1 – on
166. 2 – Ethyl – 1 – [4 – (4 – methylphenylthio) – phenyl] – 2 – morpholino – 4 – penten – 1 – on
167. 2 – Benzyl – 2 – (dimethylamino) – 1 – [4 – (octylthio) – phenyl] – butan – 1 – on
168. 2 – Benzyl – 1 – [4 – (chlorphenylthio) – phenyl] – 2 – (dimethylamino) – 4 – penten – 1 – on
169. 2 – Methyl – 1 – [4 – (2 – methoxycarbonylethylthio) – phenyl] – 2 – morpholino – 4 – penten – 1 – on
170. 1 – [4 – (Butylsulfinyl) – phenyl] – 2 – (dimethylamino) – 2 – ethyl – 4 – penten – 1 – on
171. 1 – [4 – (Benzolsulfonyl) – phenyl] – 2 – benzyl – 2 – (dimethylamino) – butan – 1 – on
172. 2 – Benzyl – 2 – (dimethylamino) – 1 – [4 – (methylsulfinyl) – phenyl] – butan – 1 – on
173. 2 – Ethyl – 1 – [4 – (4 – methylphenylsulfonyl) – phenyl] – 2 – morpholino – 4 – penten – 1 – on
174. 1 – (3,4 – Dimethoxyphenyl) – 2 – ethyl – 2 – morpholino – 4 – penten – 1 – on
175. 2 – Benzyl – 1 – (3,4 – dimethoxyphenyl) – 2 – (dimethylamino) – butan – 1 – on
176. 4 – (3,4 – Dimethoxybenzoyl) – 4 – (dimethylamino) – hepta – 1,6 – dien
177. 1 – (1,3 – Benzodioxol – 5 – yl) – 2 – benzyl – 2 – (dimethylamino) – butan – 1 – on
178. 1 – (1,3 – Benzodioxol – 5 – yl) – 2 – methyl – 2 – morpholino – 4 – penten – 1 – on
179. 2 – Benzyl – 2 – (dimethylamino) – 1 – (3,4,5 – trimethoxyphenyl) – butan – 1 – on
180. 1 – (Dibenzofuran – 3 – yl) – 2 – ethyl – 2 – morpholino – 4 – penten – 1 – on
181. 1 – (4 – Benzoylphenyl) – 2 – benzyl – 2 – (dimethylamino) – propan – 1 – on
182. 2 – [2 – Benzyl – 2 – (dimethylamino) – butanoyl] – fluorenon
183. 2 – (2 – Methyl – 2 – morpholino – 4 – pentenoyl) – xanthon
184. 2 – [2 – Allyl – 2 – (dimethylamino) – 4 – pentenoyl] – acridanon
185. 2 – [2 – Benzyl – 2 – (dimethylamino) – butanoyl] – dibenzosuberon
186. 1 – (N – Butylcarbazol – 3 – yl) – 2 – ethyl – 2 – morpholino – 4 – penten – 1 – on
187. 2 – Benzyl – 1 – (N – butylcarbazol – 3 – yl) – 2 – (dimethylamino) – butan – 1 – on
188. 2 – Allyl – 2 – (dimethylamino) – 1 – (N – methylphenothiazin – 2 – yl) – 4 – penten – 1 – on
189. 2 – Benzyl – 1 – (N – butyl – phenoxazin – 2 – yl) – 2 – morpholino – propan – 1 – on
190. 2 – Benzyl – 2 – (dimethylamino) – 1 – (xanthen – 2 – yl) – butan – 1 – on
191. 1 – (Chroman – 6 – yl) – 2 – ethyl – 2 – morpholino – 4 – penten – 1 – on
192. 2 – Benzyl – 2 – (dimethylamino) – 1 – (N – methylindolin – 5 – yl) – propan – 1 – on
193. 1 – (N – Butylindolin – 5 – yl) – 2 – ethyl – 2 – morpholino – 4 – penten – 1 – on
194. 1 – (5,10 – Dibutyl – 5,10 – dihydrophenazin – 6 – yl) – 2 – (dimethylamino) – 4 – penten – 1 – on
195. 2 – Benzyl – 1 – (1,4 – dimethyl – 1,2,3,4 – tetrahydrochinoxalin – 6 – yl) – 2 – (dimethylamino) – butan – 1 – on
196. 1 – (1,4 – Dibutyl – 1,2,3,4 – tetrahydrochinoxalin – 6 – yl) – 2 – ethyl – 2 – morpholino – 4 – penten – 1 – on
197. 2 – Benzyl – 1 – (2,3 – dihydro – 2,3 – dimethyl – benzothiazol – 5 – yl) – 2 – (dimethylamino)butan – 1 – on
198. 1 – (2,3 – Dihydrobenzofuran – 5 – yl) – 2 – methyl – 2 – morpholino – 4 – penten – 1 – on
199. 2 – Benzyl – 1 – (2,3 – dihydrobenzofuran – 5 – yl) – 2 – (dimethylamino) – butan – 2 – on
200. 1 – (4 – Aminophenyl) – 2 – benzyl – 2 – (dimethylamino) – butan – 2 – on
201. 1 – [4 – (Butylamino)phenyl] – 2 – methyl – 2 – morpholino – 4 – penten – 1 – on
202. 2 – Benzyl – 2 – (dimethylamino) – 1 – [4 – (isopropylamino)phenyl] – butan – 1 – on
203. 2 – Ethyl – 1 – (4 – methoxyphenyl) – 2 – piperidino – 4 – penten – 1 – on
204. 2 – Methyl – 2 – (N – methylpiperazino) – 1 – [4 – (N – methylpiperazino)phenyl] – 4 – penten – 1 – on
205. 2 – Benzyl – 2 – [di(2 – methoxyethyl) – amino] – 1 – [4 – (thiomethyl)phenyl] – butan – 1 – on
206. 2 – (Dibutylamino) – 1 – (4 – methoxyphenyl) – 2 – methyl – 4 – penten – 1 – on
207. 1 – [4 – (Dimethylamino) – phenyl] – 2 – ethyl – 2 – (methylphenylamino) – 4 – penten – 1 – on
208. 2 – Methyl – 1 – (methoxyphenyl) – 2 – oxazolidino – 4 – penten – 1 – on

209. 2 – Ethyl – 1 – (4 – morpholinophenyl) – 2 – piperidino – 4 – penten – 1 – on
210. 2 – Methyl – 2 – piperidino – 1 – (4 – piperidinophenyl) – 4 – penten – 1 – on
211. 2 – Ethyl – 1 – [4 – (methylthio)phenyl] – 2 – piperidino – 4 – penten – 1 – on
212. 2 – Benzyl – 2 – (dibutylamino) – 1 – [4 – (methylthio)phenyl] – butan – 1 – on
213. 2 – (Dibutylamino) – 2 – methyl – 1 – [4 – (dimethylamino)phenyl] – 4 – penten – 1 – on
214. 2 – Benzyl – 2 – (dibutylamino) – 1 – (4 – morpholinophenyl) – butan – 1 – on
215. 2 – (Dimethylamino) – 1 – [4 – (dimethylamino) – phenyl] – 2 – [(1 – cyclohexenyl) – methyl] – butan – 1 – on
216. 2 – (Dimethylamino) – 2 – (2 – cyclopentenyl) – 1 – (4 – morpholinophenyl) – propan – 1 – on
217. 2 – Ethyl – 2 – (4 – morpholinobenzoyl) – N – methyl – 1,2,3,6 – tetrahydropyridin
218. 2 – (Dimethylamino) – 1 – [4 – (dimethylamino) – phenyl] – 2,4,5 – trimethyl – 4 – hexen – 1 – on
219. 2 – (Dimethylamino) – 1 – [4 – (dimethylamino) – phenyl] – 2 – (2 – pinen – 10 – yl) – butan – 1 – on
220. 2 – Benzyl – 2 – (dimethylamino) – 1 – [4 – (2,6 – dimethylmorpholin – 4 – yl)phenyl] – butan – 1 – on
221. 2 – Ethyl – 2 – (2,6 – dimethylmorpholin – 4 – yl) – 1 – [4 – (2,6 – dimethylmorpholin – 4 – yl)phenyl] – 4 – penten – 1 – on
222. 1 – [4 – (Dimethylamino)phenyl] – 2 – ethyl – 2 – (2,6 – dimethylmorpholin – 4 – yl) – 4 – penten – 1 – on
223. 1 – [4 – (2,6 – Dimethylmorpholin – 4 – yl)phenyl] – 2 – methyl – 2 – morpholino – 4 – penten – 1 – on
224. 2 – Ethyl – 1 – [4 – (2 – hydroxyethyloxy)phenyl] – 2 – morpholino – 4 – penten – 1 – on
225. 1 – [4 – (2 – Methoxyethyloxy)phenyl] – 2 – methyl – 2 – morpholino – 4 – penten – 1 – on
226. 1 – [4 – (2 – Hydroxyethylthio)phenyl] – 2 – morpholino – 2 – propyl – 4 – penten – 1 – on
227. 2 – Benzyl – 2 – (dimethylamino) – 1 – [4 – (2 – methoxyethylthio)phenyl] – butan – 1 – on
228. 2 – (Dimethylamino) – 2 – isopropyl – 1 – (4 – morpholino – phenyl) – 4 – penten – 1 – on
229. 2 – Benzyl – 1 – (3,5 – dichlorphenyl) – 2 – (dimethylamino) – butan – 1 – on
230. 1 – (3,5 – Dichlor – 4 – methoxyphenyl) – 2 – methyl – 2 – morpholino – 4 – penten – 1 – on
231. 2 – (Diallylamino) – 2 – ethyl – 1 – (4 – morpholinophenyl) – 4 – penten – 1 – on
232. 1 – [4 – (Dimethylamino)phenyl] – 2 – methyl – 2 – (pyrrolidin – 1 – yl) – 4 – penten – 1 – on
233. 2 – Benzyl – 2 – (dimethylamino) – 1 – (4 – methylphenyl)butan – 1 – on
234. 1 – (4 – Dodecylphenyl) – 2 – ethyl – 2 – morpholino – 4 – penten – 1 – on
235. 2 – Methyl – 1 – (4 – methylphenyl) – 2 – morpholino – 4 – penten – 1 – on
236. Dodecylbenzolsulfonat von 2 – Ethyl – 2 – morpholino – 1 – (4 – morpholinophenyl) – 4 – penten – 1 – on
237. Dodecylbenzolsulfonat von 2 – Benzyl – 2 – (dimethylamino) – 1 – (4 – morpholinophenyl) – butan – 1 – on.
238. 2 – Dimethylamino – 2 – (4 – dodecylbenzyl) – 1 – (4 – morpholinophenyl) – butan – 1 – on
239. 2 – (4 – Ethylbenzyl) – 2 – dimethylamino – 1 – (4 – morpholinophenyl) – butan – 1 – on
240. 2 – Dimethylamino – 2 – (4 – isopropylbenzyl) – 1 – (4 – morpholinophenyl) – butan – 1 – on
241. 2 – Dimethylamino – 1 – (4 – dimethylaminophenyl) – 1 – (4 – methylbenzyl) – butan – 1 – on
242. 2 – Dimethylamino – 2 – (4 – hydroxymethylbenzyl) – 1 – (4 – morpholinphenyl) – butan – 1 – on
243. 2 – (4 – [Acetyloxyethyl]benzyl) – 2 – dimethylamino – 1 – (4 – morpholinophenyl) – butan – 1 – on
244. 2 – Dimethylamino – 2 – (4 – [2 – (2 – methoxyethyloxy) – ethyloxy]benzyl) – 1 – (4 – morpholinophe – nyl) – butan – 1 – on
245. 2 – Dimethylamino – 2 – (4 – [2 – (2 – [2 – methoxyethoxy] – ethoxycarbonyl) – ethyl]benzyl) – 1 – (4 – morpholinophenyl) – butan – 1 – on
246. 2 – (4 – [2 – Bromethyl]benzyl) – 2 – dimethylamino – 1 – (4 – morpholinophenyl) – butan – 1 – on
247. 2 – (4 – [2 – Diethylaminoethyl]benzyl) – 2 – dimethylamino – 1 – (4 – morpholinophenyl) – butan – 1 – on
248. 2 – Dimethylamino – 1 – (4 – dimethylaminophenyl) – 2 – (4 – dodecylbenzyl) – butan – 1 – on
249. 2 – Dimethylamino – 1 – (4 – dimethylaminophenyl) – 2 – (4 – isopropylbenzyl) – butan – 1 – on
250. 2 – Dimethylamino – 2 – (3,4 – dimethylbenzyl) – 1 – (4 – morpholinophenyl) – butan – 1 – on
251. 2 – Dimethylamino – 2 – [4 – (2 – (2 – methoxyethoxy) – ethoxycarbonyl)benzyl] – 1 – (4 – morpholino – phenyl) – butan – 1 – on
264. 2 – (Dimethylamino) – 2 – (4 – methylbenzyl) – 1 – (4 – morpholinophenyl) – butan – 1 – on
265. 2 – (4 – Butylbenzyl) – 2 – (dimethylamino) – 1 – (4 – morpholinophenyl) – butan – 1 – on
266. 2 – (Dimethylamino) – 2 – (4 – isobutylbenzyl) – 1 – (4 – morpholinophenyl) – butan – 1 – on
267. 2 – Benzyl – 2 – (dimethylamino) – 1 – (4 – [3 – methoxypropylamino]phenyl) – butan – 1 – on
268. 1 – (4 – [N – Acetyl – 3 – methoxypropylamino]phenyl) – 2 – benzyl – 2 – (dimethylamino) – butan – 1 – on
269. 2 – Benzyl – 2 – (di[2 – methoxyethyl]amino) – 1 – (4 – morpholinophenyl) – butan – 1 – on

270. 2 – Ethyl – 2 – (di[2 – methoxyethyl]amino) – 1 – (4 – morpholinophenyl) – 4 – penten – 1 – on
271. 2 – Benzyl – 2 – (dimethylamino) – 1 – (4 – morpholinophenyl) – hexan – 1 – on
272. 2 – Benzyl – 2 – (dimethylamino) – 1 – (4 – morpholinophenyl) – heptan – 1 – on
273. 2 – Benzyl – 2 – (dimethylamino) – 1 – (4 – morpholinophenyl) – octan – 1 – on
274. 2 – Benzyl – 2 – (dimethylamino) – 4,5,5 – trimethyl – 1 – (4 – morpholinophenyl) – hexan – 1 – on
275. 2 – (Dimethylamino) – 2 – (4 – methoxybenzyl) – 1 – (4 – morpholinophenyl) – butan – 1 – on
276. 2 – (4 – Butyloxybenzyl) – 2 – (dimethylamino) – 1 – (4 – morpholinophenyl) – butan – 1 – on
277. 2 – (Dimethylamino) – 2 – (4 – [2 – hydroxyethoxy] – benzyl) – 1 – (4 – morpholinophenyl) – butan – 1 – on
278. 2 – (Dimethylamino) – 2 – (4 – [2 – methoxyethoxy] – benzyl) – 1 – (4 – morpholinophenyl) – butan – 1 – on
279. 2 – (Dimethylamino) – 2 – (4 – isopropylbenzyl) – 1 – (4 – morpholinophenyl) – butan – 1 – on
280. 2 – (Dimethylamino) – 2 – (4 – dodecylbenzyl) – 1 – (4 – morpholinophenyl) – octan – 1 – on
281. 2 – (Dimethylamino) – 2 – (2 – isopropylbenzyl) – 1 – (4 – morpholinophenyl) – pentan – 1 – on
282. 2 – (Dimethylamino) – 1 – (4 – (dimethylamino)phenyl – 2 – (4 – [2 – methoxy] – benzyl) – heptan – 1 – on
283. 2 – (Butylmethylamino) – 2 – (4 – isopropylbenzyl) – 1 – (4 – morpholinophenyl) – butan – 1 – on
284. 2 – (4 – Isobutylbenzyl) – 2 – (butylmethylamino) – 1 – (4 – morpholinophenyl) – pentan – 1 – on
285. 2 – (4 – Methylbenzyl) – 2 – (dioctylamino) – 1 – (4 – morpholinophenyl) – hexan – 1 – on
286. 2 – (4 – Butoxybenzyl) – 2 – (butylmethylamino) – 1 – (4 – morpholinophenyl) – pentan – 1 – on
287. 2 – (4 – Butylbenzyl) – 2 – (butylmethylamino) – 1 – (4 – morpholinophenyl) – hexan – 1 – on

Beispiele für einzelne Verbindungen der Formel II sind:

252. N,N' – Bis[1 – ethyl – 1 – (4 – morpholinobenzoyl) – 3 – butenyl] – piperazin
253. N,N' – Bis[1 – allyl – 1 – (4 – (dimethylamino) – benzoyl) – 3 – butenyl] – piperazin
254. N,N – Bis[1 – benzyl – 1 – (4 – methoxybenzoyl) – propanyl] – methylamin
255. N,N' – Bis[1 – ethyl – 1 – (4 – (methylthio) – benzoyl) – butenyl] – hexamethylendiamin
256. N,N' – Bis[1 – benzyl – 1 – (4 – morpholinobenzoyl) – propan – 1 – yl] – N,N' – dimethyl – 3,6,9,12 – tetraoxa – tetradecamethylendiamin

Beispiele für einzelne Verbindungen der Formel III sind:

257. 1,10 – Bis[4 – dimethylamino) – phenyl] – 2,9 – diallyl – 2,9 – dimorpholinodecan – 1,10 – dion
258. 1,6 – Bis[4 – (methylthio) – phenyl] – 2,4 – bis(dimethylamino) – 2,4 – dibenzylhexan – 1,6 – dion
259. 1,4 – Bis[2 – (dimethylamino) – 2 – (4 – morpholinobenzoyl) – 4 – pentenyl] – benzol
260. 1,4 – Bis[2 – (dimethylamino) – 2 – (4 – methoxybenzoyl) – 3 – phenyl – propanyl] – benzol
261. 1,4 – Bis[2 – (dimethylamino) – 2 – (4 – morpholinobenzoyl) – butyl)benzol
262. 3,8 – Bis(dimethylamino) – 3,8 – bis(4 – morpholinobenzoyl) – dec – 5 – en
263. 3,8 – Bis(dimethylamino) – 5,6 – dimethyliden – 3,8 – bis(4 – morpholinobenzoyl) – decan

Bei den meisten dieser Verbindungen ist $R^1$ – oder $R^1$ und $R^2$ – ein Substituent vom Allyl – oder Benzyltyp. Die Synthese solcher Verbindungen schliesst meist eine C – Allylierung oder C – Benzylierung ein. Die Einführung der Aminogruppe – $NR^3R^4$ erfolgt vorzugsweise vor der Allylierung/Benzylierung. Die Synthese erfolgt dann in der folgenden Reihenfolge von Reaktionsschritten:

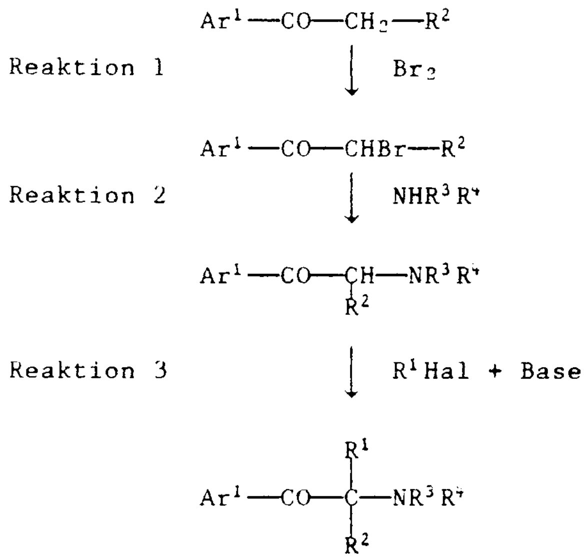


Die Ausgangsketone sind bekannte Verbindungen, die z.B. durch eine Friedel-Crafts-Reaktion hergestellt werden können. Die Reaktionsschritte 1 und 2 sind bekannte Reaktionen, die z.B. in der EP-A-3002 näher beschrieben sind. Man kann beide Reaktionen hintereinander ausführen ohne das Bromketon zu isolieren.

Die Reaktion 3 wird im folgenden ausführlich beschrieben. Im Falle einer C-Allylierung kann sie über den Enol-allylether als Zwischenprodukt verlaufen im Sinne einer Claisen-Umlagerung. Im Falle einer C-Benzylierung oder C-Allylierung kann diese über ein quartäres Benzylammoniumsalz bzw. Allylammoniumsalz als Zwischenprodukt verlaufen im Sinne einer Stevens-Umlagerung. In beiden Fällen wird jedoch das Zwischenprodukt nicht isoliert. Wenn sowohl $R^1$ wie $R^2$ Allyl- bzw. Benzylgruppen sind, so startet man die oben aufgeführte Reaktionsfolge mit einem Aryl-methyl-keton $Ar^1-CO-CH_3$ und führt die Reaktion 3 zweimal durch, wobei man einmal $R^1Hal$ und einmal $R^2Hal$ verwendet.

Befinden sich am aromatischen Rest $Ar^1$ Substituenten, die gegenüber den Reaktionen 1, 2 oder 3 nicht inert sind, so führt man die Synthese mit einem Hilfssubstituenten durch, der in einem anschliessenden Schritt 4 in den gewünschten Substituenten verwandelt wird. Beispielsweise kann man die Synthesen mit einer Nitroarylverbindung durchführen und diese anschliessend zur entsprechenden Aminoverbindung reduzieren. Oder man startet mit einer Halogenarylverbindung und ersetzt anschliessed das Halogen durch $-OR^{18}$, $-SR^{19}$ oder $-NR^{20}R^{21}$ in einer nukleophilen Austauschreaktion. Eine Gruppe $-SR^{19}$ kann anschliessend oxidiert werden zu $-SO-R^{19}$ oder $-SO_2R^{19}$.

Zur Synthese von Verbindungen der Formel II verwendet man in der Reaktionsstufe 2 ein primäres Amin $R^{11}NH_2$ oder ein sekundäres Diamin $R^{11}-NH-R^{12}-NH-R^{11}$ oder Piperazin und schliesst die Reaktion 3) und gegebenenfalls 4) an.

Zur Synthese von Verbindungen der Formel III startet man mit einem Bis-arylketon der Formel

$$Ar^1—CO—CH_2—Y—CH_2—CO—Ar^1$$

und unterwirft es den Reaktionen 1, 2, 3.

Ist $R^1$ ein Substituent vom Vinyl-Typ, so lassen sich solche Verbindungen aus den entsprechenden Allylverbindungen durch eine katalysierte Doppelbindungsisomerisierung herstellen. Ist $R^1$ ein Substituent der Formel

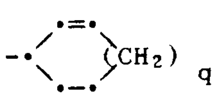 ,

so wird dieser wie ein Allylrest eingeführt unter Verwendung einer Halogenverbindung der Formel

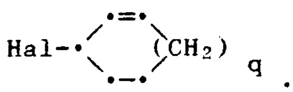

Eine weitere Möglichkeit zur Synthese von Verbindungen der Formel I ist die Umsetzung eines $\alpha$-Aminonitrils mit einer Aryl-lithium-Verbindung und anschliessender Hydrolyse:

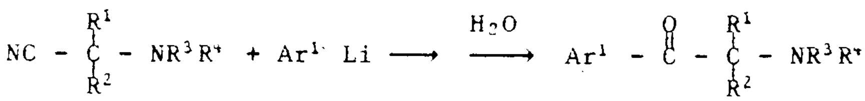


Solche Reaktionen sind z.B. von Cromwell und Hess im J. Am. Chem. Soc. 83, 1237 (1961) beschrieben worden. Die $\alpha$-Aminonitrile sind durch eine Strecker-Synthese aus $R^1-CO-R^2$ direkt zugänglich oder können durch Allylierung oder Benzylierung eines $\alpha$-Aminonitrils $NC-CHR^2-NR^3R^4$ hergestellt werden. Die Allylierung von $\alpha$-Aminonitrilen ist z.B. von T.S. Stevens im J. Chem. Soc. 1930, 2119 beschrieben.

Verbindungen der Formel I, worin $R^1$ und $R^2$ zusammen mit dem C-Atom, an das sie gebunden sind, einen Cycloalkenring bilden, können analog durch Umsetzung von Aryl-lithium-Verbindungen mit den entsprechenden cyclischen Nitrilen hergestellt werden. Die cyclischen Nitrile lassen sich durch Cycloaddition von $\alpha$-Aminocrylonitrilen an 1,3-Diene gewinnen, wie dies z.B. von Brucher und Stella in Tetrahedron 41, 875 (1985) beschrieben wurde:

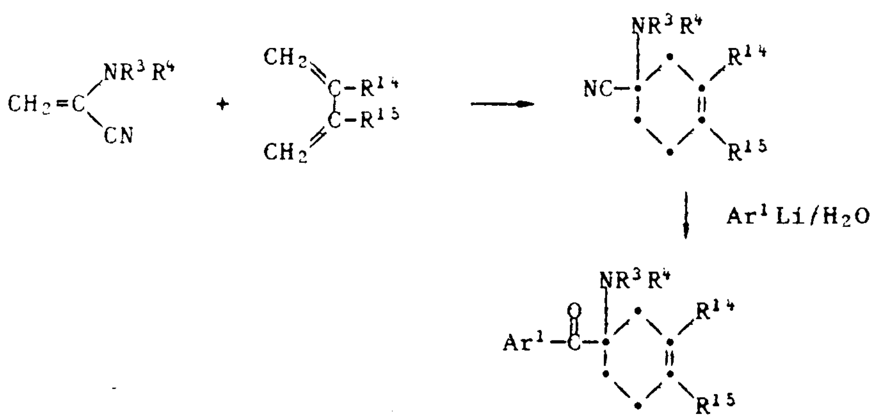


Schliesslich besteht auch die Möglichkeit, die $\alpha$-Bromketone mit einem tertiären Allyl- oder Benzylamin umzusetzen und das entstandene Quartärsalz einer Stevens-Umlagerung zu unterwerfen:

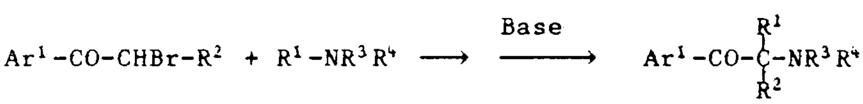


Erfindungsgemäss können die Verbindungen der Formel I, II und III als Photoinitiatoren für die Photopolymerisation von ethylenisch ungesättigten Verbindungen bzw. Gemischen, die solche Verbindungen enthalten, verwendet werden. Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkyl-acrylate oder -methacrylate, wie z.B. Methyl-, Ethyl-, Butyl-, 2-Ethylhexyl- oder 2-Hydroxyethylacrylat, Isobornylacrylat, Methyl- oder Ethylmethacrylat. Weitere Beispiele hierfür sind Acrylnitril, Acrylamid, Methacrylamid, N-substituierte (Meth)acrylamide, Vinylester wie Vinylacetate, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol-, oder Bisphenol-A-diacrylat, 4,4'-Bis(2-acryloyloxyethoxy)-diphenylpropan, Trimethylolpropan-triacrylat, Pentaerythrit-triacrylat oder -tetraacrylat, Vinylacrylat, Divinylbenzol, Divinylsuccinat, Diallylphthalat, Triallylphosphat, Triallylisocyanurat oder Tris-(2-acryloyloxy-ethyl)isocyanurat.

Beispiele für höhermolekulare (oligomere) mehrfach ungesättigte Verbindungen sind acrylierte Epoxidharze, acrylierte Polyether, acrylierte Polyurethane oder acrylierte Polyester. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Solche ungesättigte Oligomere kann man auch als Prepolymere bezeichnen.

Häufig verwendet man Zweikomponenten-Gemische eines Prepolymeren mit einem mehrfach ungesättigten Monomeren oder Dreikomponentengemische, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass man ein Lösungsmittel verwenden muss.

Solche Zwei- und Dreikomponentensysteme auf der Basis eines Prepolymeren werden sowohl für Druckfarben als auch für Lacke, Photoresists oder andere photohärtbare Massen verwendet. Als Bindemittel für Druckfarben werden vielfach auch Einkomponenten-Systeme auf der Basis photohärtbarer Prepolymerer verwendet.

Ungesättigte Polyesterharze werden meist in Zweikomponentensystemen zusammen mit einem einfach ungesättigten Monomer, vorzugsweise mit Styrol, verwendet. Für Photoresists werden oft spezifische Einkomponentensysteme verwendet, wie z.B. Polymaleinimide, Polychalkone oder Polyimide, wie sie in der DE-OS 2 308 830 beschrieben sind.

Die ungesättigten Verbindungen können auch im Gemisch mit nicht-photopolymerisierbaren filmbildenden Komponenten verwendet werden. Diese können z.B. physikalisch trocknende Polymere bzw. deren Lösungen in organischen Lösungsmitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Die photopolymerisierbaren Gemische können ausser dem Photoinitiator verschiedene Additive enthalten. Beispiele hierfür sind thermische Inhibitoren, die eine vorzeitige Polymerisation verhindern sollen, wie z.B. Hydrochinon oder sterisch gehinderte Phenole. Zur Erhöhung der Dunkellagerstabilität können z.B. Kupferverbindungen, Phosphorverbindungen, quartäre Ammoniumverbindungen oder Hydroxylaminderivate verwendet werden. Zwecks Ausschluss des Luftsauerstoffes während der Polymerisation kann man Paraffin oder ähnliche wachsartige Stoffe zusetzen, die bei Beginn der Polymerisation an die Oberfläche wandern. Als Lichtschutzmittel können in geringer Menge UV-Absorber, wie z.B. solche vom Benztriazol-, Benzophenon- oder Oxalanilid-Typ, zugesetzt werden. Noch besser ist der Zusatz von Lichtschutzmitteln, die UV-Licht nicht absorbieren, wie z.B. von sterisch gehinderten Aminen (HALS).

In bestimmten Fällen kann es von Vorteil sein, Gemische von zwei oder mehr der erfindungsgemässen Photoinitiatoren zu verwenden. Selbstverständlich können auch Gemische mit bekannten Photoinitiatoren verwendet werden, z.B. Gemische mit Benzophenon, Acetophenonderivaten, Benzoinethern oder Benzilketalen.

Zur Beschleunigung der Photopolymerisation können Amine zugesetzt werden, wie z.B. Triethanolamin, N-Methyl-diethanolamin, p-Dimethylaminobenzoesäure-ethylester oder Michlers Keton. Die Wirkung der Amine kann verstärkt werden durch den Zusatz von aromatischen Ketonen vom Typ des Benzophenons.

Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Benzophenon-, Thioxanthon-, Anthrachinon- und 3-Acylcumarinderivate sowie 3-(Aroylmethylen)-thiazoline.

Die Wirksamkeit der erfindungsgemässen Photoinitiatoren lässt sich steigern durch Zusatz von Titanocenderivaten mit fluororganischen Resten, wie sie in den EP-A-122.223 und 186.626 beschrieben sind, z.B. in einer Menge von 1-20 %. Beispiele für solche Titanocene sind Bis(methylcyclopentadienyl)-bis-(2,3,6-trifluorphenyl)-titan, Bis(cyclopentadienyl)-bis(4-dibutylamino-2,3,5,6-tetrafluorphenyl)-titan, Bis(methylcyclopentadienyl)-2-(trifluormethyl)phenyl-titan-isocyanat, Bis(cyclopentadienyl)-2-

(trifluormethyl)phenyl-titan-trifluoracetat oder Bis(methylcyclopentadienyl)-bis(4-decyloxy-2,3,5,6-tetrafluorphenyl)-titan. Für diese Gemische eignen sich vor allem flüssige α-Aminoketone.

Die erfindungsgemässen photohärtbaren Zusammensetzungen können für verschiedene Zwecke verwendet werden. In erster Linie ist ihre Verwendung in pigmentierten oder eingefärbten Systemen von Bedeutung, wie z.B. für Druckfarben, für photographische Reproduktionsverfahren, Bildaufzeichnungsverfahren und zur Herstellung von Reliefformen.

Ein weiteres wichtiges Einsatzgebiet sind Anstrichstoffe, die pigmentiert oder unpigmentiert sein können. Besonders wertvoll sind die Gemische in Weisslacken, darunter versteht man durch $TiO_2$ pigmentierte Anstrichstoffe. Weitere Einsatzgebiete sind die Strahlenhärtung von Photoresists, die Photovernetzung silberfreier Filme sowie die Herstellung von Druckplatten. Eine weitere Verwendung ist die für Aussenanstriche, die im Tageslicht oberflächlich nachhärten.

Die Photoinitiatoren werden für die angeführten Anwendungsgebiete zweckmässig in Mengen von 0,1 bis 20 Gew.%, vorzugsweise etwa 0,5 bis 5 Gew.%, bezogen auf die photopolymerisierbare Zusammensetzung, angewendet.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, superaktinische Leuchtstoffröhren, Metallhalogenid-Lampen oder Laser geeignet, deren Emissionsmaxima im Bereich zwischen 250 und 450 nm liegen. Im Falle einer Kombination mit Photosensibilisatoren oder Ferrocenderivaten kann auch längerwelliges Licht oder Laserstrahlen bis 600 nm verwendet werden.

Die Herstellung und Verwendung der erfindungsgemässen Photoinitiatoren ist in den folgenden Beispielen näher beschrieben. Hierin bedeuten Teile Gewichtsteile, Prozente Gewichtsprozente und die Temperatur ist in Celsius-Graden angegeben.

Beispiel 1: Herstellung eines α-Benzylketons

A) 1-(4-Fluorphenyl)-2-dimethylamino-butanon-1

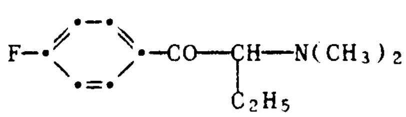

240 g (0,98 mol) 1-(4-Fluorphenyl)-2-brombutanon-1 (hergestellt durch Bromierung von 4-Fluorbutyrophenon nach der in der EP-A-3002 beschriebenen Methode) werden in 250 ml Diethylether gelöst. Diese Lösung wird zu einer Mischung von 265 g (5,87 mol) Dimethylamin in 1250 ml Diethylether bei 0° langsam zugetropft. Nach 12-stündigem Rühren bei 0° wird das überschüssige Dimethylamin bei Raumtemperatur durch Durchblasen von $N_2$ entfernt und die Suspension auf Wasser gegossen. Die Etherphase wird mit Wasser gewaschen und über $MgSO_4$ getrocknet. Nach Filtration und Eindampfen der Lösung verbleiben 202,8 g Rohprodukt als farbloses Oel, das ohne weitere Reinigung für die weitere Umsetzung verwendet wird.

Das NMR-Spektrum ($CDCl_3$) des Rohproduktes stimmt mit der angegebenen Struktur überein: 7,8-8,23 (m, 2H); 6,8-7,3 (m, 2H); 3,75 (d x d, 1H); 2,3 (s, 6H); 1,46-2,3 (m, 2H); 0,83 (t, 3H).

B) 1 – (4 – Fluorphenyl) – 2 – dimethylamino – 2 – benzyl – butanon – 1

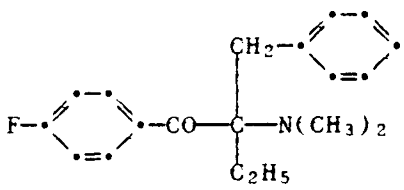

100 g (0,48 mol) 1 – (4 – Fluorphenyl) – 2 – dimethylamino – butanon – 1 (Rohprodukt aus A) werden in 330 ml Acetonitril gelöst. Dazu werden unter Rühren 98,1 g (0,57 mol) Benzylbromid langsam zugetropft. Nach 12 – stündigem Rühren bei Raumtemperatur wird das Lösungsmittel im Vakuum abdestilliert. Der Rückstand wird in 500 ml Wasser gelöst und die Lösung auf 55 – 60° erwärmt. Bei dieser Temperatur tropft man 113 g einer 34%igen NaOH – Lösung (0,96 mol) zu und rührt 30 Minuten nach.

Nach Abkühlung wird das Reaktionsgemisch mit Diethylether extrahiert, die Etherphase über $MgSO_4$ getrocknet und eingedampft. Es hinterbleiben 117,1 g Rohprodukt, das ohne weitere Reinigung für die weitere Umsetzung verwendet wird. Das NMR – Spektrum des Rohproduktes stimmt mit der angegebenen Struktur überein.

NMR ($CDCl_3$) – $\delta$ (ppm): 8,1 – 8,53 (m, 2H); 6,76 – 7,5 (m, 7,H); 3,16 (s, 2H); 2,33 (s, 6H); 1,53 – 2,2 (m, 2H); 0,65 (t, 3H).

C) 1 – (4 – Dimethylaminophenyl) – 2 – dimethylamino – 2 – benzyl – butanon – 1

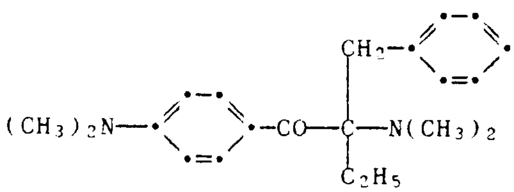

Ein Rührautoklav wird gefüllt mit 50 g (0,167 mol) 1 – (4 – Fluorphenyl) – 2 – dimethylamino – 2 – benzylbutanon – 1 (Rohprodukt aus B), 300 ml Dimethylformamid und 23,1 g (0,167 mol) Kaliumcarbonat. Dann werden 22,6 g (0,5 mol) Dimethylamin under Druck (3 – 4 bar) zugegeben. Die Mischung wird auf 100° erwärmt und 24 h bei dieser Temperatur gerührt.

Nach dem Abkühlen wird das überschüssige Dimethylamin verdampft, die Reaktionsmischung auf Eis/Wasser gegossen und mit Diethylether extrahiert. Die Etherphase wird mit Wasser gewaschen, über $MgSO_4$ getrocknet und im Vakuum eingedampft. Der flüssige Rückstand wird mittels Mitteldruck – Chromatographie gereinigt, als Elutionsmittel wird Ethylacetat/Hexan 15:85 verwendet.

Man erhält 44 g Reinprodukt, das aus Ethanol kristallisiert.

Smp. 77° – 80°.

| Analyse | | | |
|---|---|---|---|
| Ber.<br>Gef. | C 77,74 %<br>C 77,59 % | H 8,70 %<br>H 8,71 % | N 8,63 %<br>N 8,62 %. |

Beispiel 2: Herstellung eines $\alpha$ – Allylketons

D) 1 – (4 – Fluorphenyl) – 2 – morpholino – 2 – ethylpent – 4 – en – 1 – on

Eine Dispersion von 12,4 g (0,51 mol) Natriumhydrid in 50 ml Hexan wird mit 300 ml Dimethylformamid (DMF) verdünnt. In diese Suspension tropft man unter Rühren innerhalb von 2 Stunden eine Lösung von 117,7 g (0,47 mol) 1 – (4 – Fluorphenyl) – 2 – morpholinobutanon – 1 in 250 ml DMF.

Dazu gibt man bei Raumtemperatur innerhalb einer Stunde 70,8 g (0,58 mol) Allylbromid und erwärmt das Reaktionsgemisch auf 110° bis in einer Probe dünnschicht-chromatographisch kein Ausgangsmaterial mehr festgestellt werden kann. Nach dem Abkühlen wird das Reaktionsgemisch auf Eis/Wasser gegossen und mit Diethylether extrahiert. Die Etherphase wird über $MgSO_4$ getrocknet und im Vakuum eingedampft.

Man erhält als Rückstand 127 g eines öligen Rohproduktes, das ohne weitere Reinigung für die anschliessende Reaktion verwendet werden kann. Das NMR-Spektrum des Rohproduktes entspricht der angegebenen Struktur.

NMR ($CDCl_3$), δ (ppm): 8,46 (d x d, 2H); 7 (t, 2H); 4,8-6,5 (m, 3H); 3,5-3,9 (m, 4H); 2,4-3,2 (m, 6H); 1,7-2,3 (m, 2H); 0,75 (t, 3H).

Beispiel 3: Drucklose Kernaminierung

E) 2-Benzyl-2-dimethylamino-1-(4-morpholinophenyl)-butanon-1

608,7 g (2,03 mol) 1-(4-Fluorphenyl)-2-dimethylamino-2-benzyl-butanon-1(Beispiel 1, B), 354,2 g (4,06 mol) Morpholin, 562 g (4,06 mol) $K_2CO_3$ und 2000 ml Dimethylsulfoxid werden unter Rühren 12 h auf 160° erwärmt. Nach dieser Zeit zeigt eine Probe im Dünnschichtchromatogramm kein Ausgangsketon mehr. Die auf Raumtemperatur abgekühlte Reaktionslösung wird auf Eis gegossen und mit Dichlormethan extrahiert. Die organische Phase wird über $MgSO_4$ getrocknet, filtriert und eingedampft. Der ölige Rückstand kristallisiert aus Ethanol. Das Produkt schmilzt bei 111-119°.

| Analyse | | | |
|---|---|---|---|
| Ber. | C 75,37 % | H 8,25 % | N 7,64 % |
| Gef. | C 75,40 % | H 8,27 % | N 7,63 %. |

Beispiel 4: Herstellung eines Diallylketons

F) 2-Dimethylamino-1-(4-fluorphenyl)-4-pentenon-1

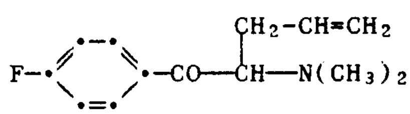

118 g (0,65 mol) α-Dimethylamino-4-fluoracetophenon werden in 350 ml Acetonitril gelöst. In dieser Lösung werden bei ca. 25° 94,5 g (0,78 mol) Allylbromid langsam zugetropft und das Reaktionsgemisch 12 h bei Raumtemperatur gerührt. Das Lösungsmittel wird im Vakuum verdampft und der feste Rückstand in warmem Wasser (ca. 60°) gelöst. Nach langsamer Zugabe von 169 ml 30%iger wässriger NaOH (1,27 mol) und Kühlung auf Raumtemperatur wird mit Diethylether extrahiert. Die Etherphase wird getrocknet und eingedampft. Man erhält als Rückstand das rohe 2-Dimethylamino-1-(4-fluorphenyl)-4-pentenon-1 als Oel, das ohne weitere Reinigung für die folgende Umsetzung verwendet wird. Das NMR-Spektrum des Produktes stimmt mit der angegebenen Struktur gut überein: 7,83-8,23 m (2H, $H_B$), 6,9-7,26 m (2H, $H_A$), 4,76-6,0 m (3H, $H_{olefin}$), 3,96 d x d (1H, J = 6 Hz, J = 8,5 Hz, Hc), 2,2-2,7 m (2H, $H_D$), 2,3s (6H, $H_E$).

G) 4-Dimethylamino-4-(4-fluorbenzoyl)-heptadien-1,6

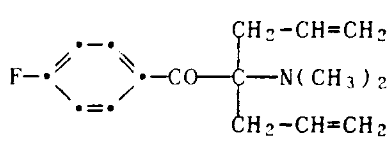

131 g (0,59 mol) des Rohproduktes aus F) in 350 ml Acetonitril und 85,9 g (0,71 mol) Allylbromid werden wie bei F) umgesetzt. Die Neutralisation des Reaktionsgemisches erfolgt mit 160 ml 30%iger NaOH

(1,2 mol). Die Isolierung des Rohproduktes geschieht ebenfalls wie bei F). Das Reaktionsprodukt ist ein gelbliches Oel, das ohne weitere Reinigung zur nächsten Stufe (Kernaminierung) verwendet werden kann.
NMR ($CDCl_3$): 8,53 – 8,1 m (2H, $H_B$), 7,2 – 6,7 m (2H, $H_A$), 6,1 – 4,7 m (6H, $H_{olefin}$), 2,8 – 2,5 m (4H, $H_{allyl}$), 2,4s (6H, N – $CH_3$).

Beispiel 5: Synthese von 3,6 – Dibutyryl – 9 – butyl – 9H – carbazol

H) Synthese von N – Butylcarbazol

Eine Suspension von 100 g (0,6 mol) Carbazol in 200 ml Toluol wird unter Rühren zum Rückfluss erwärmt. Nach Abkühlen auf 95° gibt man zuerst 26,8 g (0,12 mol) Triethylbenzylammoniumchlorid und dann eine Lösung von 169,8 g KOH in 180 ml Wasser zu. Die Temperatur sinkt dadurch auf 65°. Nun gibt man innerhalb von 5 Minuten 205,6 g (1,5 mol) Butylbromid unter kräftigem Rühren zu, wobei die Temperatur auf 92° steigt. Man hält noch 10 Minuten am Rückfluss, wobei sich alles Carbazol auflöst. Dann trennt man die wässrige Phase ab. Die Toluolphase wird mit wenig Wasser gewaschen, über $Na_2CO_3$ getrocknet und im Vakuum eingedampft. Der ölige Rückstand wird in 300 ml Hexan warm gelöst und geklärt. Beim Abkühlen kristallisiert das Produkt in beigefarbenen Kristallen, die bei 50 – 52° schmelzen.

I) Friedel – Crafts – Reaktion

In eine Suspension von 93,3 g (0,7 mol) $AlCl_3$ in 100 ml Methylenchlorid werden unter Rühren und Kühlung auf – 10° bis – 5° in 30 Minuten 46,9 g (0,44 mol) Buttersäurechlorid zugetropft. Anschliessend wird bei – 10° bis – 5° innerhalb von 2 Stunden eine Lösung von 22,3 g (0,1 mol) N – Butylcarbazol in 50 ml $CH_2Cl_2$ zugetropft. Die Suspension wird 16 h bei 0° bis 20° gerührt und dann auf Eis gegossen. Die entstandene Emulsion wird zweimal mit $CHCl_3$ extrahiert, das Extrakt wird mit Wasser gewaschen, über $MgSO_4$ getrocknet und eingedampft. Das rohe 3,6 – Dibutyryl – 9 – butyl – 9H – carbazol wird aus 80 ml Ethanol umkristallisiert. Die erhaltenen Kristalle schmelzen bei 107 – 109°.

In analoger Weise erhält man aus 81,4 g Propionylchlorid und 44,7 g N – Butylcarbazol das 3,6 – Dipropionyl – 9 – butyl – 9H – carbazol, das bei 142 – 144° schmilzt.

In Analogie zu den allgemeinen Herstellungsbeispielen A – I werden die in den folgenden Tabellen aufgeführten Produkte hergestellt.

Tabelle 1

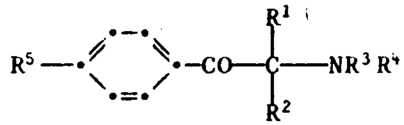

| Verbindung Nr. | $R^5$ | $R^1$ | $R^2$ | $-NR^3R^4$ | Physikalische Eigenschaften | | Analyse C | H | N |
|---|---|---|---|---|---|---|---|---|---|
| 1 | 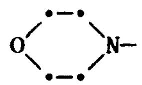 | Allyl | Ethyl | $-N(CH_3)_2$ | Smp. 58- 61° | ber. | 72,11 | 8,92 | 8,85 % |
| | | | | | | gef. | 72,08 | 8,97 | 8,73 % |
| 2 | 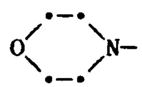 | Allyl | Methyl | $-N(CH_3)_2$ | Oel | ber. | 71,49 | 8,67 | 9,25 % |
| | | | | | | gef. | 71,56 | 8,64 | 9,04 % |
| 3 | 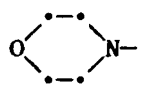 | Benzyl | Methyl | $-N(CH_3)_2$ | Smp. 90-92° | ber. | 74,97 | 8,01 | 7,95 % |
| | | | | | | gef. | 75,09 | 8,09 | 7,71 % |
| 4 | 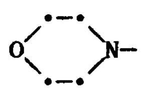 | Allyl | Allyl | 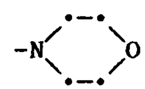 | Oel | ber. | 71,32 | 8,16 | 7,56 % |
| | | | | | | gef. | 71,24 | 8,12 | 7,57 % |
| 5 | 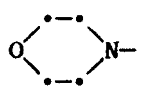 | Allyl | Ethyl | 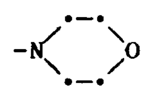 | Oel | ber. | 70,36 | 8,43 | 7,81 % |
| | | | | | | gef. | 69,87 | 8,41 | 7,64 % |
| 6 | $(CH_3)_2N-$ | Benzyl | Ethyl | $-N(CH_3)_2$ | Smp. 77-80° | ber. | 77,74 | 8,70 | 8,63 % |
| | | | | | | gef. | 77,59 | 8,71 | 8,62 % |
| 7 | $(CH_3)_2N-$ | Allyl | Allyl | $-N(CH_3)_2$ | Oel | ber. | 75,48 | 9,15 | 9,78 % |
| | | | | | | gef. | 75,48 | 9,25 | 9,48 % |

| Verbindung Nr. | $R^5$ | $R^1$ | $R^2$ | $-NR^3R^4$ | Physikalische Eigenschaften | | Analyse C | H | N |
|---|---|---|---|---|---|---|---|---|---|
| 8 | 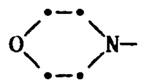 | Allyl | Allyl | $-N(CH_3)_2$ | Smp. 65-67° | ber.<br>gef. | 73,14<br>73,05 | 8,59<br>8,51 | 8,59 %<br>8,51 % |
| 9 | $(CH_3)_2N-$ | Allyl | Ethyl | $-N(CH_3)_2$ | Smp. 34-37° | ber.<br>gef. | 74,41<br>74,29 | 9,55<br>9,55 | 10,21 %<br>10,23 % |
| 10 | 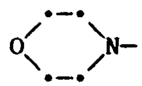 | Benzyl | Ethyl | $-N(CH_3)_2$ | Smp. 110-119° | ber.<br>gef. | 75,37<br>75,38 | 8,25<br>8,32 | 7,64 %<br>7,62 % |
| 11 | $(CH_3)_2N-$ | Benzyl | Allyl | $-N(CH_3)_2$ | Smp. 68-70° | ber.<br>gef. | 78,53<br>78,54 | 8,39<br>8,38 | 8,33 %<br>8,10 % |
| 12 | $(CH_3)_2N-$ | Benzyl | Benzyl | $-N(CH_3)_2$ | Smp. 132-133° | ber.<br>gef. | 80,79<br>80,71 | 7,82<br>7,91 | 7,25 %<br>7,21 % |
| 13 | $CH_3S-$ | Allyl | Ethyl | 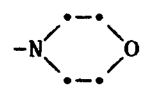 | Oel | ber.<br>gef. | 67,68<br>67,43 | 7,89<br>7,81 | 4,38 %<br>4,22 % |
| 14 | $CH_3S-$ | Allyl | Allyl | 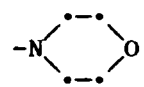 | Oel | ber.<br>gef. | 68,85<br>68,83 | 7,61<br>7,56 | 4,23 %<br>4,17 % |
| 15 | $CH_3O-$ | Allyl | Allyl | $-N(CH_3)_2$ | Oel | ber.<br>gef. | 74,69<br>74,54 | 8,48<br>8,48 | 5,12 %<br>5,04 % |
| 16 | $CH_3O-$ | Allyl | Allyl | 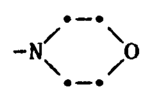 | Oel | ber.<br>gef. | 72,35<br>72,51 | 7,99<br>8,09 | 4,44 %<br>4,14 % |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 17 | $CH_3O-$ | Allyl | Phenyl | 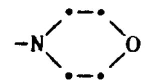 | Smp. 84-86° | ber.<br>gef. | 75,19<br>74,97 | 7,17<br>7,14 | 3,99 %<br>3,91 % | |
| 18 | $CH_3O-$ | Allyl | Ethyl | 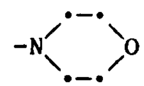 | Oel | ber.<br>gef. | 71,26<br>71,19 | 8,30<br>8,44 | 4,62 %<br>4,57 % | |
| 19 | $CH_3S-$ | Benzyl | Ethyl | $-N(CH_3)_2$ | Oel | ber.<br>gef. | 73,35<br>73,47 | 7,69<br>7,69 | 4,28<br>4,12 | 9,79 %<br>9,61 % |
| 20 | $CH_3S-$ | Allyl | Ethyl | $-N(CH_3)_2$ | Oel | ber.<br>gef. | 69,27<br>69,24 | 8,35<br>8,41 | 5,05<br>5,00 | 11,55 %<br>11,41 % |
| 21 | $CH_3S-$ | Allyl | Benzyl | $-N(CH_3)_2$ | Oel | ber.<br>gef. | 74,29<br>74,22 | 7,42<br>7,44 | 4,12<br>3,92 | 9,44 %<br>9,26 % |
| 22 | $CH_3S-$ | Allyl | Allyl | $-N(CH_3)_2$ | Oel | ber.<br>gef. | 70,55<br>70,58 | 8,01<br>8,00 | 4,82<br>4,70 | 11,08 %<br>10,93 % |
| 23 | $CH_3S-$ | 4-Fluor-benzyl | Methyl | $-N(CH_3)_2$ | Oel | ber.<br>gef. | 68,85<br>68,89 | 6,69<br>6,84 | 4,22<br>4,06 | 9,67 %<br>9,57 % |
| 24 | $CH_3S-$ | 4-Chlor-benzyl | Methyl | $-N(CH_3)_2$ | Smp. 69-70° | ber.<br>gef. | 65,60<br>65,30 | 6,37<br>6,34 | 4,03<br>3,92 | 9,22 %<br>9,25 % |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 25 | $CH_3S-$ | 2-Chlor-benzyl | Methyl | $-N(CH_3)_2$ | Oel | ber. | 65,59 | 6,37 | 4,03 | 9,22 % |
| | | | | | | gef. | 65,98 | 6,51 | 3,97 | 9,09 % |
| 26 | $CH_3S-$ | 4-Brom-benzyl | Methyl | $-N(CH_3)_2$ | Oel | ber. | 58,16 | 5,65 | 3,57 | 8,17 % |
| | | | | | | gef. | 58,35 | 5,71 | 3,44 | 7,99 % |
| 27 | $CH_3S-$ | Methallyl | Ethyl | 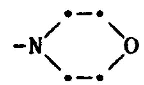 | Oel | ber. | 68,43 | 8,16 | 4,20 | 9,61 % |
| | | | | | | gef. | 68,41 | 8,28 | 3,93 | 9,53 % |
| 28 | $CH_3S-$ | 2-Butenyl | Ethyl | 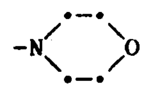 | Oel | ber. | 68,43 | 8,16 | 4,20 | 9,61 % |
| | | | | | | gef. | 68,46 | 8,20 | 4,08 | 9,63 % |
| 29 | $CH_3S-$ | Allyl | Benzyl | 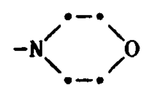 | Oel | ber. | 72,40 | 7,13 | 3,67 | 8,40 % |
| | | | | | | gef. | 72,51 | 7,31 | 3,52 | 8,25 % |
| 30 | $CH_3S-$ | Allyl | Butyl | 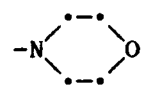 | Oel | ber. | 69,12 | 8,41 | 4,03 | 9,23 % |
| | | | | | | gef. | 68,98 | 8,29 | 3,80 | 9,26 % |
| 31 | $CH_3S-$ | 4-Methyl-benzyl | Methyl | $-N(CH_3)_2$ | Smp. 74-75° | ber. | 73,35 | 7,69 | 4,28 | 9,79 % |
| | | | | | | gef. | 72,84 | 7,72 | 4,29 | 9,66 % |
| 32 | $CH_3S-$ | 4-Methyl-thio-benzyl | Methyl | $-N(CH_3)_2$ | Oel | ber. | 66,81 | 7,01 | 3,89 | 17,83 % |
| | | | | | | gef. | 66,70 | 7,17 | 3,69 | 17,41 % |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 33 | $CH_3S-$ | 4-Methoxy-benzyl | Methyl | $-N(CH_3)_2$ | Oel | ber.<br>gef. | 69,93<br>69,50 | 7,34<br>7,38 | 4,08<br>3,84 | 9,33 %<br>9,49 % |
| 34 | $CH_3S-$ | Allyl | Methyl | 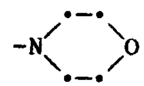 | Oel | ber.<br>gef. | 66,85<br>66,92 | 7,59<br>7,60 | 4,59<br>4,53 | 10,50 %<br>10,50 % |
| 35 | $CH_3S-$ | Allyl | Methyl | $-N(CH_3)_2$ | Oel | ber.<br>gef. | 68,40<br>68,09 | 8,04<br>8,07 | 5,32<br>5,32 | 12,17 %<br>12,16 % |
| 36 | $CH_3S-$ | Allyl | Phenyl | 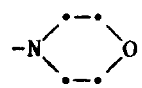 | Smp.97-99° | ber.<br>gef. | 71,90<br>71,76 | 6,86<br>7,00 | 3,81<br>3,70 | 8,72 %<br>8,74 % |
| 37 | $CH_3S-$ | Allyl | Phenyl | $-N(CH_3)_2$ | Oel | ber.<br>gef. | 73,81<br>73,36 | 7,12<br>7,10 | 4,30<br>4,05 | 9,85 %<br>9,78 % |
| 38 | $CH_3S-$ | Benzyl | Phenyl | $-N(CH_3)_2$ | Smp.142-143° | ber.<br>gef. | 76,76<br>76,95 | 6,71<br>6,75 | 3,73<br>3,77 | 8,54 %<br>8,50 % |
| 39 | 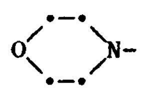 | Allyl | Methyl | 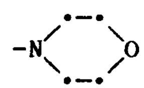 | Oel | ber.<br>gef. | 69,74<br>69,43 | 8,19<br>8,33 | 8,13 %<br>7,87 % | |
| 40 | 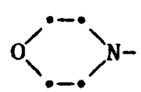 | Allyl | Benzyl | 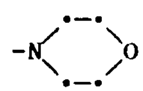 | Smp.150° | ber.<br>gef. | 74,26<br>74,22 | 7,67<br>7,66 | 6,66 %<br>6,68 % | |
| 41 | $(CH_3)_2N-$ | Allyl | Ethyl | 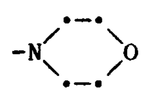 | Oel | ber.<br>gef. | 72,12<br>72,14 | 8,92<br>8,95 | 8,85 %<br>8,62 % | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 42 | $(CH_3)_2N-$ | Allyl | Methyl | 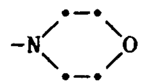 | wachs-artig | ber.<br>gef. | 71,49<br>71,51 | 8,67<br>8,72 | 9,26 %<br>9,12 % | |
| 43 | 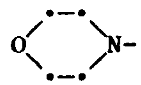 | Benzyl | Benzyl | $-N(CH_3)_2$ | Smp.177-178° | ber.<br>gef. | 78,47<br>78,57 | 7,53<br>7,59 | 6,54 %<br>6,47 % | |
| 44 | $(CH_3)_2N-$ | Allyl | Allyl | 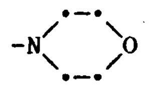 | Oel | ber.<br>gef. | 73,14<br>73,42 | 8,59<br>8,84 | 8,53 %<br>7,83 % | |
| 45 | $(CH_3)_2N-$ | Allyl | Methyl | $-N(CH_3)_2$ | Oel | ber.<br>gef. | 73,81<br>73,41 | 9,29<br>9,06 | 10,76 %<br>9.65 % | |
| 46 | 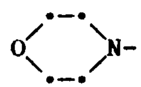 | Allyl | Benzyl | $-N(CH_3)_2$ | Smp. 91-93° | ber.<br>gef. | 76,16<br>76,27 | 7,99<br>8,04 | 7,40 %<br>7,12 % | |
| 47 | $(CH_3)_2N-$ | Methallyl | Ethyl | 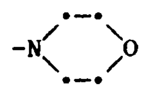 | Smp.79-84° | ber.<br>gef. | 72,69<br>72,57 | 9,15<br>9,24 | 8,48 %<br>8,49 % | |
| 48 | $(CH_3)_2N-$ | 2-Butenyl | Ethyl | 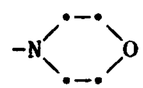 | Oel | ber.<br>gef. | 72,69<br>72,12 | 9,15<br>9,17 | 8,48 %<br>7,99 % | |
| 49 | 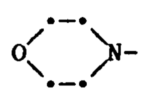 | 2-Butenyl | Ethyl | 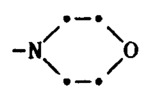 | Oel | ber.<br>gef. | 70,93<br>70,61 | 8,66<br>8,75 | 7,52 %<br>6,93 % | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 50 | 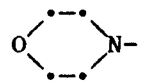 | Methallyl | Ethyl | 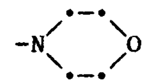 | Oel | ber. | 70,93 | 8,66 | 7,52 % | |
| | | | | | | gef. | 70,42 | 8,71 | 7,11 % | |
| 51 | $(CH_3OCH_2CH_2)_2N-$ | Allyl | Methyl | 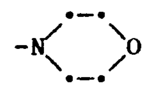 | Oel | ber. | 67,66 | 8,78 | 7,17 % | |
| | | | | | | gef. | 67,81 | 8,76 | 7,25 % | |
| 52 | $(C_4H_9)_2N-$ | Allyl | Methyl | 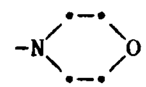 | Oel | ber. | 74,57 | 9,91 | 7,25 % | |
| | | | | | | gef. | 74,62 | 9,87 | 7,30 % | |
| 53 | 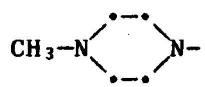 | Allyl | Methyl | 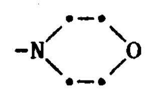 | Oel | ber. | 70,55 | 8,74 | 11,75 % | |
| | | | | | | gef. | 70,27 | 8,81 | 11,64 % | |
| 54 | $CH_3O-$ | Benzyl | Ethyl | $-N(CH_3)_2$ | Oel | ber. | 77,13 | 8,09 | 4,50 % | |
| | | | | | | gef. | 77,09 | 8,15 | 4,47 % | |
| 55 | $(C_2H_5)_2N-$ | Allyl | Methyl | 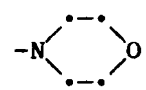 | Oel | ber. | 72,69 | 9,15 | 8,48 % | |
| | | | | | | gef. | 72,66 | 9,15 | 8,24 % | |
| 56 | 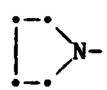 | Allyl | Methyl | 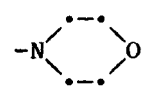 | Smp.98-99° | ber. | 73,14 | 8,59 | 8,53 % | |
| | | | | | | gef. | 73,10 | 8,59 | 8,48 % | |
| 57 | 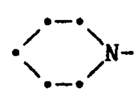 | Benzyl | Ethyl | $-N(CH_3)_2$ | Smp.76-81° | ber. | 79,08 | 8,85 | 7,68 % | |
| | | | | | | gef. | 78,96 | 8,79 | 7,60 % | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 58 | 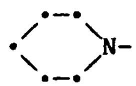 | Allyl | Ethyl | $-N(CH_3)_2$ | Oel | ber.<br>gef. | 76,39<br>76,40 | 9,62<br>9,50 | 8,90 %<br>8,74 % | |
| 59 | $(C_2H_5)_2N-$ | Benzyl | Ethyl | $-N(CH_3)_2$ | Oel | ber.<br>gef. | 78,36<br>77,79 | 9,15<br>9,08 | 7,95 %<br>7,14 % | |
| 60 | $(C_2H_5)_2N-$ | Allyl | Ethyl | $-N(CH_3)_2$ | Oel | ber.<br>gef. | 75,45<br>75,35 | 9,99<br>9,80 | 9,26 %<br>8,70 % | |
| 61 | $HOCH_2CH_2S-$ | Benzyl | Ethyl | $-N(CH_3)_2$ | Oel | ber.<br>gef. | 70,55<br>70,18 | 7,61<br>7,51 | 3.92<br>3,71 | 8,97 %<br>8,95 % |
| 62 | $HOCH_2CH_2S-$ | Allyl | Ethyl | 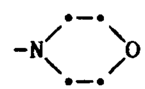 | Oel | ber.<br>gef. | 65,30<br>64,54 | 7,79<br>7,76 | 4.01<br>3,79 | 9,17 %<br>9,35 % |
| 63 | $(CH_2=CH-CH_2)_2N-$ | Allyl | Methyl | 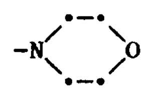 | Oel | | | | | |
| 64 | $CH_3S-$ | 4-Benzoyl-benzyl | Methyl | $-N(CH_3)_2$ | Smp.135-136° | ber.<br>gef. | 74,79<br>74,53 | 6,52<br>6,56 | 3.35<br>3,24 | 7,68 %<br>7,55 % |
| 65 | H | 3,4-Dimeth-oxybenzyl | Methyl | $-N(CH_3)_2$ | Smp.116-117° | ber.<br>gef. | 73,36<br>73,03 | 7,69<br>7,73 | 4.27 %<br>4,11 % | |
| 66 | $CH_3S-$ | 3,4-Dimeth-oxybenzyl | Methyl | $-N(CH_3)_2$ | Oel | ber.<br>gef. | 67,53<br>67,41 | 7,29<br>7,32 | 3.75<br>4,03 | 8,58 %<br>8,99 % |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 67 | H | 4-Benzoyl-benzyl | Methyl | $-N(CH_3)_2$ | Smp.106-108° | ber.<br>gef. | 80,83<br>80,22 | 6,78<br>6,75 | 3.77 %<br>3,79 % | |
| 68 | F | Benzyl | Ethyl | $-N(CH_3)_2$ | Smp.62-65° | ber.<br>gef. | 76,22<br>76,07 | 7,41<br>7,40 | 4.68 %<br>4,60 % | |
| 69 | F | Allyl | Allyl | $-N(CH_3)_2$ | Oel | | | | | |
| 70 | $CH_3-$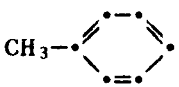$-SO_2-$ | Allyl | Ethyl | 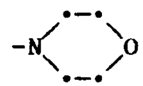 | glasig | ber.<br>gef. | 67,42<br>66,98 | 6,48<br>7,10 | 3,28<br>3,12 | 7,50 %<br>7,51 % |
| 71 | $CH_3SO_2-$ | Allyl | Ethyl | $-N(CH_3)_2$ | Smp.74-75° | ber.<br>gef. | 62,11<br>62,03 | 7,49<br>7,48 | 4,53<br>4,50 | 10,36 %<br>10,27 % |
| 72 | $CH_3-$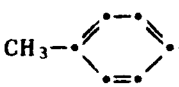$-SO_2-$ | Benzyl | Ethyl | $-N(CH_3)_2$ | Smp.107-108° | ber.<br>gef. | 71,69<br>71,46 | 6,71<br>6,68 | 3,22<br>3,16 | 7,36 %<br>7,39 % |
| 73 | $CH_3SO_2-$ | Benzyl | Ethyl | $-N(CH_3)_2$ | glasig | ber.<br>gef. | 66,82<br>66,74 | 7,01<br>7,01 | 3,90<br>3,81 | 8,92 %<br>8,94 % |
| 74 | F | Allyl | Methyl | 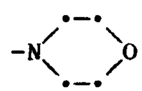 | Oel | ber.<br>gef. | 69,29<br>69,02 | 7,27<br>7,47 | 5,05 %<br>4,86 % | |
| 75 | F | Allyl | Allyl | 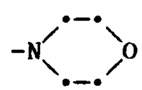 | Oel | ber.<br>gef. | 71,26<br>71,19 | 7,31<br>7,25 | 4,62 %<br>4,50 % | |
| 76 | F | Allyl | Benzyl | $-N(CH_3)_2$ | Oel | | | | | |

| Verbindung Nr. | $R^5$ | $R^1$ | $R^2$ | $-NR^3R^4$ | Physikalische Eigenschaften | Analyse | C | H | N | S |
|---|---|---|---|---|---|---|---|---|---|---|
| 77 | F | Benzyl | Benzyl | $-N(CH_3)_2$ | Oel | | | | | |
| 78 | F | Allyl | Ethyl | $-N(CH_3)_2$ | Oel | | | | | |
| 79 | F | Allyl | Benzyl | 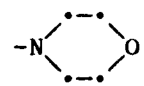 | Oel | | | | | |
| 80 | F | Allyl | Ethyl | 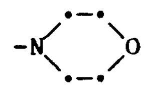 | Oel | | | | | |
| 81 | F | Benzyl | Methyl | $-N(CH_3)_2$ | Oel | | | | | |
| 82 | F | Allyl | Methyl | $-N(CH_3)_2$ | Oel | | | | | |
| 83 | HO- | Benzyl | Ethyl | $-N(CH_3)_2$ | Smp.110-119° | | | | | |
| 84 | $C_2H_5OOCCH_2O-$ | Benzyl | Ethyl | $-N(CH_3)_2$ | Oel | ber.<br>gef. | 72,04<br>72,00 | 7,62<br>7,62 | 3,65 %<br>3,57 % | |
| 85 | $HOCH_2CH_2O-$ | Benzyl | Ethyl | $-N(CH_3)_2$ | Oel | ber.<br>gef. | 73,87<br>73,83 | 7,97<br>8,09 | 4,10 %<br>3,82 % | |
| 86 | Cl | Benzyl | Ethyl | $-N(CH_3)_2$ | Smp.67-69° | ber.<br>gef. | 72,25<br>72,32 | 7,02<br>7,03 | 4,43 %<br>4,34 % | |

| Verbindung Nr. | $R^5$ | $R^1$ | $R^2$ | $-NR^3R^4$ | Physikalische Eigenschaften | Analyse | C | H | N | S |
|---|---|---|---|---|---|---|---|---|---|---|
| 87 | Br | Benzyl | Ethyl | $-N(CH_3)_2$ | Smp. 53-55° | ber.<br>gef. | 63,34<br>63,22 | 6,15<br>6,19 | 3,89 %<br>3,87 % | |
| 88 | Br | Allyl | Ethyl | 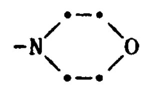 | Oel | ber.<br>gef. | 57,96<br>58,42 | 6,30<br>6,42 | 3,98 %<br>3,94 % | |
| 89 | $CH_3O-$ | 1-Propenyl | Ethyl | 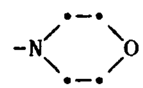 | Oel | ber.<br>gef. | 71,26<br>71,31 | 8,31<br>8,31 | 4,62 %<br>4,42 % | |
| 90 | $CH_3O-$ | Allyl | Ethyl | $-N(CH_3)_2$ | Oel | | | | | |
| 91 | $(CH_3)_2N-$ | Allyl | Benzyl | 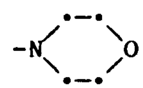 | Oel | ber.<br>gef. | 76,16<br>76,17 | 7,99<br>7,98 | 7,40 %<br>7,13 % | |
| 92 | $(CH_3)_2N-$ | Benzyl | Methyl | $-N(CH_3)_2$ | Smp. 107-108° | | | | | |
| 93 | H | Allyl | Methyl | 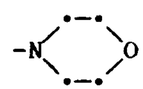 | flüssig | ber.<br>gef. | 74,10<br>74,28 | 8,16<br>8,37 | 5,40 %<br>5,31 % | |
| 94 | H | Allyl | Benzyl | 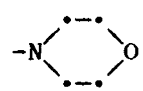 | Oel | ber.<br>gef. | 78,77<br>78,82 | 7,51<br>7,76 | 4,11 %<br>3,73 % | |
| 95 | H | Allyl | Methyl | $-N(CH_3)_2$ | flüssig | ber.<br>gef. | 77,40<br>77,34 | 8,80<br>8,79 | 6,45 %<br>6,41 % | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 96 | H | Benzyl | Methyl | $-N(CH_3)_2$ | flüssig | ber.<br>gef. | 80,86<br>80,80 | 7,92<br>8,06 | 5,24 %<br>5,17 % | |
| 97 | H | Allyl | Allyl | $-N(CH_3)_2$ | Oel | ber.<br>gef. | 79,97<br>78,77 | 8,70<br>8,75 | 5,76 %<br>5,64 % | |
| 98 | H | Benzyl | Benzyl | $-N(CH_3)_2$ | Oel | | | | | |
| 99 | H | Allyl | Benzyl | $-N(CH_3)_2$ | Oel | ber.<br>gef. | 81,87<br>81,70 | 7,90<br>7,84 | 4,77 %<br>4,65 % | |
| 100 | H | Allyl | Ethyl | $-N(CH_3)_2$ | flüssig | ber.<br>gef. | 77,88<br>77,85 | 9,15<br>9,17 | 6,05 %<br>6,01 % | |
| 101 | H | Benzyl | Ethyl | $-N(CH_3)_2$ | Oel | ber.<br>gef. | 81,10<br>81,14 | 8,24<br>8,45 | 4,98 %<br>4,93 % | |
| 102 | H | Allyl | Phenyl | 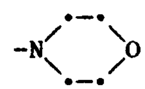 | Smp.87-88° | ber.<br>gef. | 78,47<br>78,17 | 7,21<br>7,10 | 4,36 %<br>4,32 % | |
| 103 | H | 4-Chlor-benzyl | Methyl | $-N(CH_3)_2$ | Smp.86-87° | ber.<br>gef. | 71,63<br>71,58 | 6,68<br>6,67 | 4,64 %<br>4,62 % | |
| 104 | H | 4-Brom-benzyl | Methyl | $-N(CH_3)_2$ | Smp.101-102° | ber.<br>gef. | 62,43<br>62,35 | 5,82<br>5,86 | 4,04 %<br>3,96 % | |
| 105 | H | 2-Chlor-benzyl | Methyl | $-N(CH_3)_2$ | Oel | ber.<br>gef. | 71,63<br>71,12 | 6,68<br>6,66 | 4,64 %<br>4,35 % | |

| Verbindung Nr. | $R^5$ | $R^1$ | $R^2$ | $-NR^3R^4$ | Physikalische Eigenschaften | Analyse | C | H | N | S |
|---|---|---|---|---|---|---|---|---|---|---|
| 106 | H | 3,4-Dimethoxybenzyl | Methyl | $-N(CH_3)_2$ | Smp.116-117° | ber.<br>gef. | 73,36<br>73,03 | 7,69<br>7,69 | 4,27 %<br>4,11 % | |
| 107 | H | 4-Methylbenzyl | Methyl | $-N(CH_3)_2$ | Oel | ber.<br>gef. | 81,10<br>80,57 | 8,24<br>8,23 | 4,98 %<br>4,94 % | |
| 108 | H | 4-Methylthiobenzyl | Methyl | $-N(CH_3)_2$ | Oel | ber.<br>gef. | 72,80<br>72,70 | 7,40<br>7,39 | 4,47 %<br>4,34 % | |
| 109 | H | 4-Fluorbenzyl | Methyl | $-N(CH_3)_2$ | Oel | ber.<br>gef. | 75,76<br>75,82 | 7,06<br>7,09 | 4,91 %<br>4,74 % | |
| 110 | H | 4-Methoxybenzyl | Methyl | $-N(CH_3)_2$ | Oel | ber.<br>gef. | 76,74<br>76,39 | 7,80<br>7,78 | 4,70 %<br>4,51 % | |
| 111 | F | Methallyl | Ethyl | 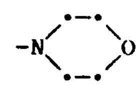 | Oel | | | | | |
| 112 | F | 2-Butenyl | Ethyl | 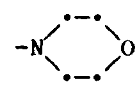 | Oel | | | | | |
| 113 | 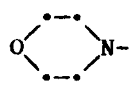 | Allyl | Ethyl | $-N(CH_3)$(Benzyl) | Oel | ber.<br>gef. | 76,49<br>76,58 | 8,22<br>8,30 | 7,14 %<br>6,81 % | |
| 114 | 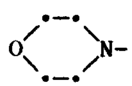 | Allyl | Ethyl | $-N(CH_3)$(Allyl) | Oel | ber.<br>gef. | 73,65<br>73,68 | 8,83<br>8,82 | 8,18 %<br>7,90 % | |

| Verbindung Nr. | $R^5$ | $R^1$ | $R^2$ | $-NR^3R^4$ | Physikalische Eigenschaften | Analyse | C | H | N | S |
|---|---|---|---|---|---|---|---|---|---|---|
| 115 | 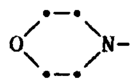 | Benzyl | Ethyl | $-N(CH_3)$(Benzyl) | Smp. 60-65° | ber.<br>gef. | 78,70<br>78,59 | 7,74<br>7,87 | 6,33 %<br>6,66 % | |
| 116 | 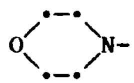 | Benzyl | Ethyl | $-N(CH_3)(C_4H_9)$ | Oel | ber.<br>gef. | 76,43<br>76,31 | 8,88<br>8,83 | 6,86 %<br>6,73 % | |
| 117 | 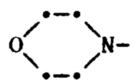 | Allyl | Ethyl | $-N(CH_3)(C_4H_9)$ | Oel | ber.<br>gef. | 73,70<br>73,60 | 9,56<br>9,56 | 7,81 %<br>7,66 % | |
| 118 | $CH_3CONH-$ | Benzyl | Ethyl | $-N(CH_3)_2$ | Glas | | | | | |
| 119 | 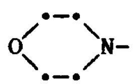 | Benzyl | Propyl | $-N(CH_3)_2$ | Glas | ber.<br>gef. | 75,75<br>76,08 | 8,48<br>8,54 | 7,36 %<br>6,96 % | |
| 120 | 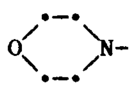 | Allyl | Propyl | $-N(CH_3)_2$ | Smp. 62-64° | ber.<br>gef. | 72,69<br>72,51 | 9,15<br>8,97 | 8,48 %<br>8,53 % | |
| 121 | $CH_3OCH_2CH_2O-$ | Allyl | Methyl | 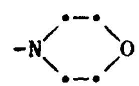 | Oel | ber.<br>gef. | 68,44<br>68,26 | 8,16<br>8,24 | 4,20 %<br>3,97 % | |
| 122 | $HOCH_2CH_2S-$ | Allyl | Isopropyl | 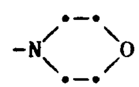 | Oel | | | | | |

| Verbindung Nr. | $R^5$ | $R^1$ | $R^2$ | $-NR^3R^4$ | Physikalische Eigenschaften | Analyse | C | H | N | S |
|---|---|---|---|---|---|---|---|---|---|---|
| 123 | Br | Allyl | Methyl | 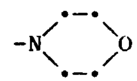 | Oel | | | | | |
| 124 | Br | Allyl | Isopropyl | 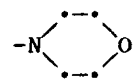 | Oel | | | | | |
| 125 | $HOCH_2CH_2S-$ | Allyl | Methyl | 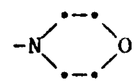 | Oel | ber.<br>gef. | 64,45<br>64,78 | 7,51<br>7,48 | 4,18<br>3,92 | 9,56 %<br>9,63 % |
| 126 | $CH_2=CHCH_2OCH_2CH_2O-$ | Allyl | Ethyl | 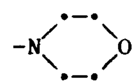 | Oel | ber.<br>gef. | 70,75<br>70,75 | 8,37<br>8,25 | 3,75 %<br>3,76 % | |
| 127 | $CH_2=CHCH_2OCH_2CH_2O-$ | Allyl | Methyl | 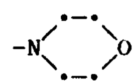 | Oel | | | | | |
| 128 | $CH_3OCH_2CH_2O-$ | Allyl | Ethyl | 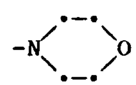 | Oel | ber.<br>ber. | 69,14<br>69,23 | 8,41<br>8,34 | 4,03 %<br>3,93 % | |
| 129 | $CH_3OCH_2CH_2NH-$ | Benzyl | Ethyl | $-N(CH_3)_2$ | Oel | | | | | |
| 130 | $CH_3NH-$ | Benzyl | Ethyl | $-N(CH_3)_2$ | Oel | | | | | |
| 131 | 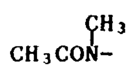 | Benzyl | Ethyl | $-N(CH_3)_2$ | Oel | | | | | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 132 | 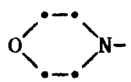 | Benzyl | Ethyl | $-N(C_2H_5)_2$ | Smp. 76-79° | ber.<br>gef. | 76,10<br>75,83 | 8,69<br>8,64 | 7,10 %<br>7,09 % | |
| 133 | 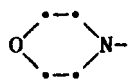 | Allyl | Ethyl | $-N(C_2H_5)_2$ | Oel | ber.<br>gef. | 73,22<br>73,03 | 9,36<br>9,40 | 8,13 %<br>7,64 % | |
| 186 | 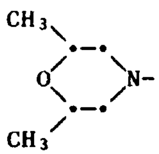 | Benzyl | Ethyl | $-N(CH_3)_2$ | Smp. 103-105°C | ber.<br>gef. | 76,10<br>75,80 | 8,68<br>8,75 | 7,10 %<br>7,07 % | |
| 240 | 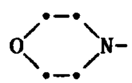 | 4-Isobutyl-benzyl | Ethyl | $-N(CH_3)_2$ | Smp. 136-137°C | ber.<br>gef. | 76,43<br>76,26 | 8,88<br>8,83 | 6,86 %<br>6,71 % | |
| 246 | 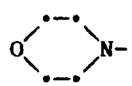 | 4-(2-Brom-ethyl)-benzyl | Ethyl | $-N(CH_3)_2$ | Harz | ber.<br>gef. | 63,42<br>63,13 | 7,03<br>7,28 | 5,92 %<br>5,54 % | |
| 264 | 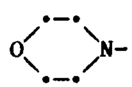 | 4-Metyhl-benzyl | Ethyl | $-N(CH_3)_2$ | Smp. 75-76°C | ber.<br>gef. | 75,75<br>75,61 | 8,48<br>8,53 | 7,36 %<br>7,26 % | |
| 265 | 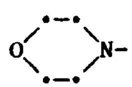 | 4-Butyl-benzyl | Ethyl | $-N(CH_3)_2$ | Harz | ber.<br>gef. | 76,74<br>76,58 | 9,06<br>9,12 | 6,63 %<br>6,45 % | |

| Verbindung Nr. | $R^5$ | $R^1$ | $R^2$ | $-NR^3R^4$ | Physikalische Eigenschaften | | Analyse C | H | N | S |
|---|---|---|---|---|---|---|---|---|---|---|
| 266 | 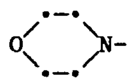 N- | 4-Isobutyl-benzyl | Ethyl | $-N(CH_3)_2$ | Smp. 77-80°C | ber.<br>gef. | 76,74<br>76,60 | 9,06<br>9,10 | 6,63 %<br>6,62 % | |
| 267 | $CH_3O(CH_2)_3NH-$ | Benzyl | Ethyl | $-N(CH_3)_2$ | Oel | ber.<br>gef. | 74,96<br>75,39 | 8,57<br>8,52 | 7,60 %<br>7,40 % | |
| 268 | $CH_3CO$<br>$CH_3O(CH_2)_3N-$ | Benzyl | Ethyl | $-N(CH_3)_2$ | Oel | ber.<br>gef. | 73,14<br>73,07 | 8,35<br>8,07 | 6,82 %<br>6,77 % | |
| 269 | 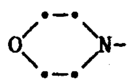 N- | Benzyl | Ethyl | $-N(CH_2CH_2OCH_3)_2$ | Oel | ber.<br>gef. | 71,34<br>71,11 | 8,43<br>8,64 | 6,16 %<br>5,96 % | |
| 270 | 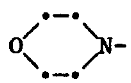 N- | Allyl | Ethyl | $-N(CH_2CH_2OCH_3)_2$ | Oel | ber.<br>gef. | 68,29<br>67,91 | 8,97<br>9,21 | 6,92 %<br>6,75 % | |

Tabelle 2

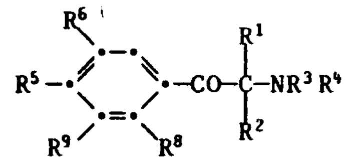

| Verbindung Nr. | $R^5$ | $R^6$ | $R^8$ | $R^9$ | $R^1$ | $R^2$ | $-NR^3R^4$ | Physikalische Eigenschaften | | Analyse C | H | N |
|---|---|---|---|---|---|---|---|---|---|---|---|---|
| 140 | $CH_3O-$ | $CH_3$ | H | $CH_3$ | Benzyl | Ethyl | $-N(CH_3)_2$ | Smp.68-70° | ber.<br>gef. | 77,84<br>77,71 | 8,61<br>8,63 | 4,13 %<br>3,93 % |
| 141 | Cl | H | Cl | H | Benzyl | Ethyl | $-N(CH_3)_2$ | Oel | ber.<br>gef. | 65,15<br>65,07 | 6,04<br>5,93 | 4,00 %<br>3,95 % |
| 142 | Cl | Cl | H | H | Allyl | Ethyl | $-N(CH_3)_2$ | Oel | ber.<br>gef. | 60,01<br>59,95 | 6,38<br>6,39 | 4,67 %<br>4,80 % |
| 143 | Cl | Cl | H | H | Benzyl | Ethyl | $-N(CH_3)_2$ | Oel | ber.<br>gef. | 65,15<br>65,19 | 6,04<br>6,23 | 4,00 %<br>3,86 % |
| 144 | 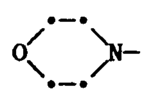 | Cl | H | H | Allyl | Ethyl | $-N(CH_3)_2$ | Oel | ber.<br>gef. | 65,04<br>64,40 | 7,76<br>7,82 | 7,98 %<br>7,72 % |
| 145 | 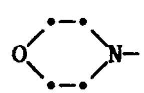 | Cl | H | H | Benzyl | Ethyl | $-N(CH_3)_2$ | Smp.110-111° | | | | |
| 146 | $(CH_3)_2N-$ | H | H | $C_2H_5$ | Benzyl | Ethyl | $-N(CH_3)_2$ | Oel | | | | |
| 147 | $(CH_3)_2N-$ | H | $CH_3$ | H | Benzyl | Ethyl | $-N(CH_3)_2$ | Smp. 101-103° | ber.<br>gef. | 78,06<br>77,97 | 8,93<br>8,92 | 8,28 %<br>8,22 % |

Tabelle 3

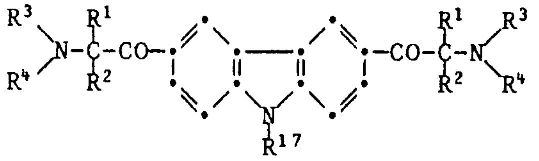

| Verb. Nr. | $R^1$ | $R_2$ | $-NR^3R^4$ | $R^{17}$ |
|---|---|---|---|---|
| 148 | Benzyl | Ethyl | $-N(CH_3)_2$ | $C_4H_9$ |
| 149 | Allyl | Methyl | 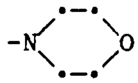 | $C_4H_9$ |

| Verb. Nr. | Physik. Eigenschaft | | C | H | N |
|---|---|---|---|---|---|
| 148 | Glas | ber. | 80,08 | 8,16 | 6,67 % |
| | | gef. | 80,24 | 7,40 | 6,41 % |
| 149 | Glas | ber. | 73,81 | 8,09 | 7,17 % |
| | | gef. | 73,66 | 8,05 | 6,87 % |

Tabelle 4 - Salze

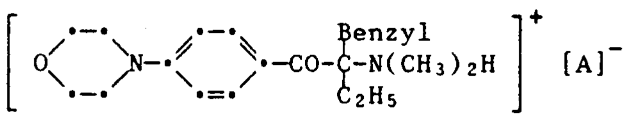

| Verb. Nr. | A | Smp. | Analyse | C | H | N |
|---|---|---|---|---|---|---|
| 150 | $CF_3COO-$ | 55-56° | ber. | 62,49 | 6,50 | 5,83 % |
| | | | gef. | 62,18 | 6,64 | 5,54 % |
| 151 | 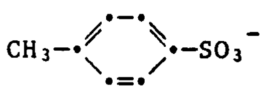 | 90-95° | ber. | 66,89 | 7,11 | 5,20 % |
| | | | gef. | 66,76 | 7,38 | 4,78 % |
| 152 | 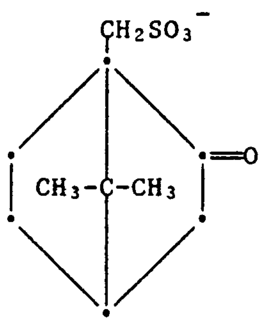 | 95-105° | ber. | 66,19 | 7,74 | 4,68 % |
| | | | gef. | 65,57 | 8,04 | 4,38 % |

Beispiel 5: Photohärtung einer blauen Druckfarbe

Eine blaue Druckfarbe wird nach folgender Rezeptur hergestellt:
62 Teile Setalin® AP 565 (Urethanacrylatharz der Fa. Synthese, Holland),
15 Teile 4,4′-Di-($\beta$-acryloyloxyethoxy)-diphenylpropan-2,2 (Ebecryl® 150, UCB, Belgien)
23 Teile Irgalithblau® GLSM (Ciba-Geigy AG, Basel).

Das Gemisch wird auf einem 3-Walzenstuhl homogenisiert und bis auf eine Korngrösse von < 5 $\mu$m gemahlen.

Von dieser Druckfarbe werden jeweils 5 g mit der gewünschten Menge an Photoinitiator auf einer Tellerreibmaschine unter einem Druck von 180 $kg/m^2$ unter Wasserkühlung homogen vermischt. Es werden Proben mit 3 % und 6 % Photoinitiator – bezogen auf die Druckfarbe – hergestellt.

Von diesen Druckfarben werden mit einem Probedruckgerät (Fa. Prüfbau, BR Deutschland) Offset–drucke auf 4 x 20 cm Streifen aus Kunstdruckpapier gemacht. Die Druckbedingungen sind

| | |
|---|---|
| Auflage Druckfarbe | 1,5 $g/m^2$ |
| Anpressdruck (Liniendruck) | 25 kp/cm |
| Druckgeschwindigkeit | 1 m/sec |

Hierbei wird eine Druckwalze mit Metalloberfläche (Aluminium) verwendet.

Die bedruckten Proben werden in einem UV–Bestrahlungsgerät der Fa. PPG mit zwei Lampen von je 80 W/cm gehärtet. Die Bestrahlungszeit wird durch Variation der Transportgeschwindigkeit der Probe variiert.

Die Oberflächentrocknung der Druckfarbe wird unmittelbar nach der Bestrahlung durch den sogenann–ten Transfer–Test geprüft. Dabei wird ein weisses Papier unter einem Liniendruck von 25 kp/cm an die bedruckte Probe angepresst. Wenn das Papier farblos bleibt, ist der Test bestanden. Wenn sichtbare Mengen Farbe auf den Teststreifen übertragen werden, so ist dies ein Zeichen, dass die Oberfläche der Probe noch nicht genügend gehärtet ist.

In der Tabelle 5 ist die maximale Transportgeschwindigkeit angegeben, bei der der Transfer–Test noch bestanden wurde.

Zur Prüfung der Durchhärtung der Druckfarbe werden ebenfalls Offset–Drucke hergestellt wie vorhin beschrieben, jedoch werden Druckwalzen mit Gummi–Oberfläche verwendet und es wird die Metallseite von Aluminiumbeschichteten Papierstreifen bedruckt.

Die Bestrahlung geschieht wie oben beschrieben. Unmittelbar nach der Bestrahlung wird die Durch–härtung in einem REL–Durchhärtungsprüfgerät getestet. Dabei wird auf die bedruckte Probe ein mit Stoff überspannter Aluminium–Zylinder aufgesetzt und unter einem Druck von 220 $g/cm^2$ innerhalb 10 sec einmal um die eigene Achse gedreht. Wenn dabei auf der Probe sichtbare Beschädigungen entstehen, so ist die Druckfarbe ungenügend durchgehärtet. In den Tabellen wird die maximale Transportgeschwindigkeit angegeben, bei der der REL–Test noch bestanden wurde.

Tabelle 5

| Photoinitiator | Konz. | Transfer-Test (m/min) | REL-Test (m/min) |
|---|---|---|---|
| Verbindung Nr. 1 | 3 % | > 170 | 150 |
| | 6 % | > 170 | > 170 |
| Verbindung Nr. 2 | 3 % | > 170 | 130 |
| | 6 % | > 170 | > 170 |
| Verbindung Nr. 3 | 3 % | > 170 | 140 |
| | 6 % | > 170 | > 170 |
| Verbindung Nr. 4 | 3 % | > 170 | 130 |
| | 6 % | > 170 | > 170 |
| Verbindung Nr. 5 | 3 % | > 170 | 120 |
| | 6 % | > 170 | > 170 |
| Verbindung Nr. 6 | 3 % | > 170 | 160 |
| | 6 % | > 170 | > 170 |
| Verbindung Nr. 7 | 3 % | > 170 | 130 |
| | 6 % | > 170 | > 170 |
| Verbindung Nr. 8 | 3 % | > 170 | 150 |
| | 6 % | > 170 | > 170 |

| | | | |
|---|---|---|---|
| Verbindung Nr. 9 | 3 % | > 170 | 130 |
| | 6 % | > 170 | > 170 |
| Verbindung Nr. 10 | 3 % | > 170 | 130 |
| | 6 % | > 170 | 160 |
| Verbindung Nr. 11 | 3 % | > 170 | 130 |
| | 6 % | > 170 | 160 |
| Verbindung Nr. 39 | 3 % | 140 | 80 |
| | 6 % | > 170 | 140 |
| Verbindung Nr. 40 | 3 % | 110 | 70 |
| | 6 % | 140 | 70 |
| Verbindung Nr. 41 | 3 % | > 170 | 110 |
| | 6 % | > 170 | 150 |
| Verbindung Nr. 42 | 3 % | 110 | 60 |
| | 6 % | > 170 | 90 |
| Verbindung Nr. 44 | 3 % | 110 | 70 |
| | 6 % | > 170 | 110 |
| Verbindung Nr. 45 | 3 % | > 170 | 110 |
| | 6 % | > 170 | 160 |
| Verbindung Nr. 46 | 3 % | > 170 | 120 |
| | 6 % | > 170 | 160 |
| Verbindung Nr. 47 | 3 % | > 170 | 50 |
| Verbindung Nr. 48 | 3 % | > 170 | 110 |
| Verbindung Nr. 51 | 3 % | 70 | 50 |
| | 6 % | > 170 | 110 |
| Verbindung Nr. 53 | 3 % | 120 | 60 |
| | 6 % | > 170 | 130 |
| Verbindung Nr. 63 | 3 % | 80 | 40 |
| | 6 % | > 170 | 80 |
| Verbindung Nr. 91 | 3 % | 110 | 70 |
| | 6 % | > 170 | 70 |
| Verbindung Nr. 92 | 3 % | > 170 | 100 |
| | 6 % | > 170 | 120 |
| Verbindung Nr. 118 | 3 % | 140 | 100 |
| Verbindung Nr. 119 | 3 % | 160 | 80 |
| Verbindung Nr. 120 | 3 % | 170 | 150 |
| Verbindung Nr. 121 | 3 % | 120 | 90 |

| | | | |
|---|---|---|---|
| Verbindung Nr. 125 | 3 % | 130 | 100 |
| Verbindung Nr. 148 | 3 % | 120 | 80 |
| Verbindung Nr. 149 | 3 % | 170 | 110 |

Beispiel 6: Photohärtung eines Weisslackes

Es wird ein Weisslack nach folgender Rezeptur hergestellt:
17,6 g Ebecryl® 593 (Polyesteracrylatharz der Fa. UCB, Belgien),
11,8 g N-Vinylpyrrolidon
19,6 g Titandioxid RTC-2 (Titandioxid der Fa. Tioxide, England),
19,6 g Sachtolith® HDS (Lithopone der Sachtleben Chemie, BRD),
11,8 g Trimethylolpropan-trisacrylat,
19,6 g Setalux® UV 2276 (acryliertes Epoxidharz auf der Basis von Bisphenol A, Kunstharzfabrik Synthese, Holland).

Die obigen Komponenten werden zusammen mit 125 g Glasperlen (Durchmesser 4 mm) in einer 250 ml Glasflasche während mindestens 24 Stunden auf eine Korngrösse von maximal 5 µm gemahlen.

Die so erhaltene Stammpaste wird in Portionen geteilt und jede Portion mit jeweils 2 % der in der Tabelle 6 angegebenen Photoinitiatoren durch Einrühren bei 60 °C gemischt und die Mischungen nochmals 16 Stunden mit Glasperlen gemahlen.

Die so hergestellten Weisslacke werden in einer Dicke von 70 µm mit einer Rakel auf Glasplatten aufgetragen. Die Proben werden einerseits in einem PPG-Bestrahlungsgerät mit einer Lampe von 80 W/cm und andererseits in einem Bestrahlungsgerät der Fa. Fusion Systems (USA) mit einer D-Lampe in jeweils einem Durchgang belichtet. Dabei wird die Geschwindigkeit des Durchganges der Proben durch das Bestrahlungsgerät laufend gesteigert bis keine ausreichende Härtung mehr eintritt. Die maximale Geschwindigkeit, bei der noch ein wischfester Lackfilm entsteht, ist in Tabelle 6 als "Härtungsgeschwindigkeit" angegeben.

Tabelle 6

| Photoinitiator (jeweils 2 %) | Härtungsgeschwindigkeit (m/min) | |
|---|---|---|
| | PPG-Gerät 80 W | Fusion D-Lampe |
| Verbindung Nr. 1 | 60 | 130 |
| Verbindung Nr. 3 | 50 | 170 |
| Verbindung Nr. 4 | 50 | 110 |
| Verbindung Nr. 6 | 140 | > 200 |
| Verbindung Nr. 7 | 80 | > 200 |
| Verbindung Nr. 8 | 70 | 150 |
| Verbindung Nr. 12 | 50 | 120 |
| Verbindung Nr. 14 | 70 | - |
| Verbindung Nr. 15 | 50 | - |

Beispiel 7: Sensibilisierte Photohärtung eines Weisslackes

Es wird wie in Beispiel 6 vorgegangen. Zusätzlich zu den 2 % Photoinitiator werden jedoch noch 0,5 % (bezogen auf den Lack) Isopropylthioxanthon als Sensibilisator zugesetzt. Dadurch tritt eine merkliche

Steigerung der Härtungsgeschwindigkeit ein. Die Ergebnisse sind in Tabelle 7 aufgeführt.

Tabelle 7

| Photoinitiator (jeweils 2 %) | Sensibilisator | Härtungsgeschwindigkeit (m/min) | |
|---|---|---|---|
| | | PPG-Gerät | Fusion D-Lampe |
| Verbindung Nr. 1 | - | 60 | 130 |
| | 0,5 % | 130 | 180 |
| Verbindung Nr. 3 | - | 50 | 170 |
| | 0,5 % | 100 | > 200 |
| Verbindung Nr. 4 | - | 50 | 110 |
| | 0,5 % | 80 | 160 |
| Verbindung Nr. 6 | - | 140 | > 200 |
| | 0,5 % | 170 | > 200 |
| Verbindung Nr. 7 | - | 80 | > 200 |
| | 0,5 % | 170 | > 200 |
| Verbindung Nr. 8 | - | 70 | 150 |
| | 0,5 % | 120 | > 200 |
| Verbindung Nr. 12 | - | 50 | 120 |
| | 0,5 % | 110 | > 200 |

Beispiel 8: Photohärtung einer schwarzen Offset-Druckfarbe

Eine photohärtbare schwarze Offset-Druckfarbe wird mit jeweils 3 % der in Tabelle 8 angegebenen Photoinitiatoren vermischt und in einer Auflage von 1,5 g/m$^2$ auf ein mit Aluminiumfolie kaschiertes Papier gedruckt, wobei wie in Beispiel 5 verfahren wird. Auch die Härtung geschieht wie in Beispiel 5 beschrieben im PPG-Gerät mit zwei Lampen à 80 Watt. Die Härtung wird durch den Transfer-Test kontrolliert - wie in Beispiel 5 beschrieben. Tabelle 8 gibt die maximale Transportgeschwindigkeit im Bestrahlungsgerät an, bei der der Transfer-Test noch eben bestanden wird.

Tabelle 8

| Photoinitiator | Härtungsgeschwindigkeit PPG-Gerät (m/min) |
|---|---|
| Verbindung Nr. 6 | 120 |
| Verbindung Nr. 8 | 130 |
| Verbindung Nr. 9 | 130 |
| Verbindung Nr. 10 | 110 |

Beispiel 9: Herstellung eines Photoresist

Eine Mischung von

Eine Mischung von

37,64 g Pentaerythrit-triacrylat
10,76 g Hexamethoxymethylmelamin (Cymel® 301, Cyanamid Corp.)
47,30 g eines carboxylgruppenhaltigen thermoplastischen Polyacrylates (Carboset® 525, Goodrich Corp.) und
4,30 g Polyvinylpyrrolidon

100,00 g

wird unter Zusatz von 0,5 g Irgalithgrün GLN (Ciba-Geigy AG) in 319 g Methylenchlorid und 30 g Methanol gelöst. Dieser Lösung werden die in Tabelle 9 angegebenen Mengen an Verbidung Nr. 10 als Photoinitiator zugesetzt.

Die viskose Lösung wird in einer Dicke von 200 µm auf Aluminiumbleche aufgetragen und die Proben werden 15 min bei 60° getrocknet. Die Trockenschichtdicke beträgt etwa 45 µm. Die Probe wird mit einer Polyesterfolie (76 µm) bedeckt. Auf die Folie wird ein optischer Stufenkeil mit 21 Stufen (Stouffer-Keil) gelegt und durch Vakuum fixiert.

Dann wird die Probe durch den Stufenkeil belichtet. Hierzu wird eine 5 kW Staublampe in einem Abstand von 30 cm verwendet. Die Belichtungsdauer beträgt 20 sec. Nach der Belichtung wird die Polyesterfolie entfernt und die Probe in einem Ultraschallbad entwickelt. Als Entwickler wird eine Lösung von 15 g $Na_2SiO_3 \cdot 9\ H_2O$, 0,16 g KOH, 3 g Polyethylenglykol 6000 und 0,5 g Lävulinsäure in 1000 g Wasser verwendet. Nach kurzer Trocknung bei Raumtemperatur wird die Probe beurteilt. Angegeben wird die höchste Stufe, welche komplett abgebildet ist und eine klebfreie Oberfläche aufweist.

Tabelle 9

| Initiator | Höchste abgebildete Stufe |
|---|---|
| 0,1 % Verbindung Nr. 10 | 10 |
| 0,5 % Verbindung Nr. 10 | 14 |
| 1,0 % Verbindung Nr. 10 | 16 |

Beispiel 10: Herstellung einer Druckplatte

88,6 Teile eines Butadien-Styrol-Copolymerisates (Cariflex® TR 1107, Shell Chemie) werden auf einem Kalander bei 140° aufgeschmolzen und bei 100° werden 11 Teile Hexandiol-diacrylat, 0,3 Teile eines phenolischen Antioxidans (Topanol® OC, ICI Comp.), 0,01 Teile Sudanschwarz B und 0,4 Teile der Verbindung Nr. 10 zugemischt und bei dieser Temperatur 10 min homogenisiert.

Aus dieser Mischung werden zwischen 2 Polyesterfolien Platten von 2 mm Dicke gepresst. Die Proben werden auf der Rückseite durch die Folie 2 min belichtet in einem BASF-Nyloprint-Belichter (40 W-Lampen). Dann wird die Folie der Vorderseite durch ein Test-Negativ ersetzt und diese Seite 6 Minuten durch das Negativ belichtet.

Die belichtete Probe wird in einem Bürstenbad mit einer Mischung von 4 Teilen Tetrachlorethylen und 1 Teil Butanol entwickelt, wobei die löslichen Teile ausgewaschen werden. Nach einer Trocknung von 60 min bei 80° wird die zweite Folie entfernt und die Druckplatte zur Fixierung nacheinander in eine 0,4%ige wässrige Bromlösung und in eine 1,15%ige $Na_2S_2O_3/Na_2CO_3$-Lösung getaucht und anschliessend mit Wasser gespült.

Schliesslich wird jede Seite 6 min nachbelichtet. Alle Teile des Test-Negativs sind deutlich abgebildet, die Lochtiefe beträgt 34 µm, die Reliefhöhe 450 µm.

**Patentansprüche**

**Patentansprüche für folgende Vertragsstaaten : AT, BE, CH, DE, FR, GB, IT, LI, NL, SE**

**1.** Eine Verbindung der Formel I, II, III oder IIIa

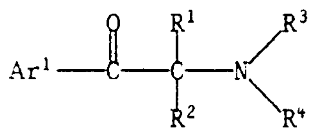

I

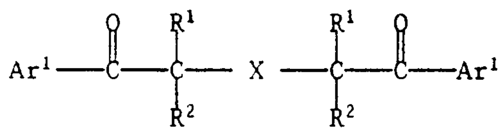

II

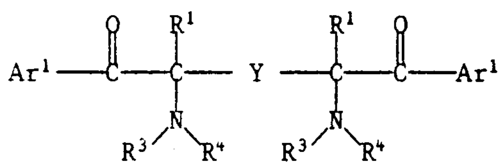

III

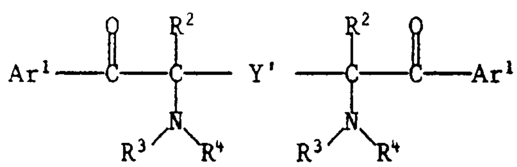

IIIa

worin $Ar^1$ einen aromatischen Rest der Formel IV, V, VI oder VII bedeutet,

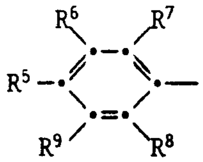 IV,

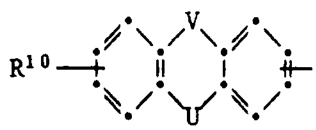 V

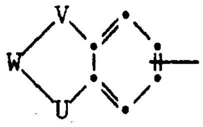 VI

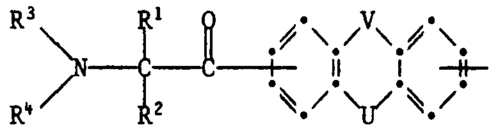 VII

worin

X einen zweiwertigen Rest der Formel

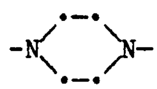 ,

$-N(R^{11})-$ oder $-N(R^{11})-R^{12}-N(R^{11})-$ bedeutet,

Y $C_1-C_6$-Alkylen, Xylylen, Cyclohexylen oder eine direkte Bindung bedeutet,

Y′ Xylylen, $C_4-C_8$-Alkendiyl, $C_6-C_{10}$-Alkadiendiyl, Dipentendiyl oder Dihydroxylylen bedeutet,

U $-O-$, $-S-$ oder $-N(R^{17})-$ bedeutet,

V $-O-$, $-S-$, $-N(R^{17})-$, $-CO-$, $-CH_2-$, $-CH_2CH_2-$, $C_2-C_6$-Alkyliden oder eine direkte Bindung bedeutet,

W unverzweigtes oder verzweigtes $C_1-C_7$-Alkylen oder $C_2-C_6$-Alkyliden bedeutet,

$R^1$ entweder

(a) ein Rest der Formel

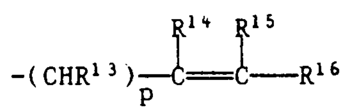

ist,
worin p null oder 1 ist,
oder

(b) ein Rest der Formel

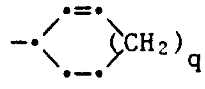

ist, wobei q 0, 1, 2 oder 3 bedeutet
oder

(c) ein Rest der Formel

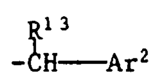

ist, worin $Ar^2$ einen unsubstituierten oder durch Halogen, OH, $C_1-$

$C_{12}$-Alkyl oder durch OH, Halogen, $-N(R^{11})_2$, $-C_1-C_{12}$-Alkoxy, $-COO(C_1-C_{18}$-Alkyl), $-CO(OCH_2CH_2)_nOCH_3$ oder $-OCO(C_1-C_4)$-Alkyl substituiertes $C_1-C_4$-Alkyl, $C_1-C_{12}$-Alkoxy oder durch $-COO(C_1-C_{18}$-Alkyl) oder $-CO(OCH_2CH_2)_nOCH_3$ substituiertes $C_1-C_4$-Alkoxy, $-(OCH_2CH_2)_nOH$, $-(OCH_2CH_2)_nOCH_3$, $C_1-C_8$-Alkylthio, Phenoxy, $-COO(C_1-C_{18}$-Alkyl), $-CO(OCH_2CH_2)_nOCH_3$, Phenyl oder Benzoyl substituierten Phenyl-, Naphthyl-, Furyl-, Thienyl- oder Pyridylrest bedeutet, worin n 1-20 ist,
oder
(d) zusammen mit $R^2$ einen Rest der Formel

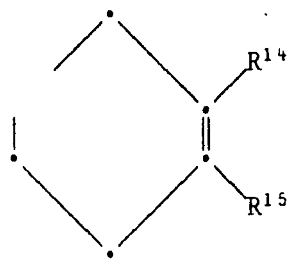

oder

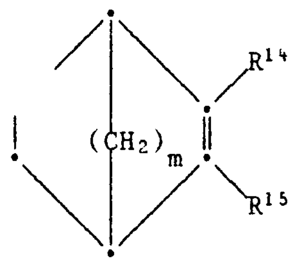

bildet, worin m 1 oder 2 ist,

$R^2$ eine der für $R^1$ gegebenen Bedeutungen hat oder $C_5-C_6$-Cycloalkyl, unsubstituiertes oder durch $C_1-C_4$-Alkoxy, Phenoxy, Halogen oder Phenyl substituiertes $C_1-C_{12}$-Alkyl oder unsubstituiertes oder durch Halogen, $C_1-C_{12}$-Alkyl oder $C_1-C_4$-Alkoxy substituiertes Phenyl bedeutet,

$R^3$ Wasserstoff, $C_1-C_{12}$-Alkyl, durch Hydroxy, $C_1-C_4$-Alkoxy, $-CN$ oder $-COO(C_1-C_4$-Alkyl) substituiertes $C_2-C_4$-Alkyl, $C_3-C_5$-Alkenyl, $C_5-C_{12}$-Cycloalkyl oder $C_7-C_9$-Phenylalkyl bedeutet,

$R^4$ $C_1-C_{12}$-Alkyl, durch Hydroxy, $C_1-C_4$-Alkoxy, $-CN$ oder $-COO(C_1-C_4$-Alkyl) substituiertes $C_2-C_4$-Alkyl, $C_3-C_5$-Alkenyl, $C_5-C_{12}$-Cycloalkyl, $C_7-C_9$-Phenylalkyl, Phenyl oder durch Halogen, $C_1-C_{12}$-Alkyl, $C_1-C_4$-Alkoxy oder $-COO(C_1-C_4$-Alkyl) substituiertes Phenyl bedeutet oder $R^4$ zusammen mit $R^2$ $C_1-C_7$-Alkylen, $C_7-C_{10}$-Phenylalkylen, o-Xylylen, 2-Butenylen oder $C_2-C_3$-Oxa- oder Azaalkylen bedeutet, oder

$R^3$ und $R^4$ zusammen $C_3-C_7$-Alkylen bedeuten, das durch $-O-$, $-S-$, $-CO-$ oder $-N(R^{17})-$ unterbrochen sein kann oder durch Hydroxy, $C_1-C_4$-Alkoxy oder $-COO(C_1-C_4$-Alkyl) substituiert sein kann,

$R^5$, $R^6$, $R^7$, $R^8$ und $R^9$ unabhängig voneinander Wasserstoff, Halogen, $C_1-C_{12}$-Alkyl, $C_5-C_6$-Cycloalkyl, Phenyl, Benzyl, Benzoyl oder eine Gruppe $-OR^{18}$, $-SR^{19}$, $-SO-R^{19}$, $-SO_2-R^{19}$, $-N(R^{20})(R^{21})$, $-NH-SO_2-R^{22}$ oder

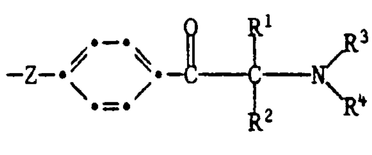

bedeuten, worin Z $-O-$, $-S-$, $-N(R^{11})-$, $-N(R^{11})-R^{12}-N(R^{11})-$ oder

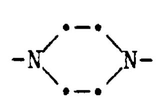

bedeutet, wobei im Falle, das $R^1$ Allyl und
$R^2$ Methyl ist, $R^5$ nicht $-OCH_3$ ist, und im Falle, dass $R^1$ Benzyl ist und

$R^2$ Methyl oder Benzyl ist, $R^5$ nicht $-OCH_3$, $-SCH_3$ oder $-SO-CH_3$ ist,

| | |
|---|---|
| $R^{10}$ | Wasserstoff, $C_1-C_{12}$-Alkyl, Halogen oder $C_2-C_8$-Alkanoyl bedeutet, |
| $R^{11}$ | Wasserstoff, $C_1-C_8$-Alkyl, $C_3-C_5$-Alkenyl, $C_7-C_9$-Phenylalkyl, $C_1-C_4$-Hydroxyalkyl oder Phenyl bedeutet, |
| $R^{12}$ | unverzweigtes oder verzweigtes $C_2-C_{16}$-Alkylen, das durch ein oder mehrere $-O-$, $-S-$ oder $-N(R^{11})-$ unterbrochen sein kann, |
| $R^{13}$ | Wasserstoff, $C_1-C_8$-Alkyl oder Phenyl bedeutet, |
| $R^{14}$, $R^{15}$ und $R^{16}$ | unabhängig voneinander Wasserstoff oder $C_1-C_4$-Alkyl bedeuten oder $R^{14}$ und $R^{15}$ zusammen $C_3-C_7$-Alkylen sind, |
| $R^{17}$ | Wasserstoff, $C_1-C_{12}$-Alkyl, das durch ein oder mehrere $-O-$ unterbrochen sein kann, $C_3-C_5$-Alkenyl, $C_7-C_9$-Phenylalkyl, $C_1-C_4$-Hydroxyalkyl, $-CH_2CH_2CN$, $-CH_2CH_2COO(C_1-C_4$-Alkyl), $C_2-C_8$-Alkanoyl oder Benzoyl bedeutet, |
| $R^{18}$ | Wasserstoff, $C_1-C_{12}$-Alkyl, durch $-CN$, $-OH$, $C_1-C_4$-Alkoxy, $C_3-C_6$-Alkenoxy, $-OCH_2CH_2CN$, $-OCH_2CH_2COO(C_1-C_4$-Alkyl), $-COOH$ oder $-COO(C_1-C_4$-Alkyl) substituiertes $C_1-C_6$-Alkyl, $-(CH_2CH_2O)_nH$ mit n = 2-20, $C_2-C_8$-Alkanoyl, $C_3-C_{12}$-Alkenyl, Cyclohexyl, Hydroxycyclohexyl, Phenyl, durch Halogen, $C_1-C_{12}$-Alkyl oder $C_1-C_4$-Alkoxy substituiertes Phenyl, $C_7-C_9$-Phenylalkyl oder $-Si-(C_1-C_8Alkyl)_r(Phenyl)_{3-r}$ mit r = 1, 2 oder 3 bedeutet, |
| $R^{19}$ | Wasserstoff, $C_1-C_{12}$-Alkyl, $C_3-C_{12}$-Alkenyl, Cyclohexyl, durch $-SH$, $-OH$, $-CN$, $-COO(C_1-C_4$-Alkyl), $C_1-C_4$-Alkoxy, $-OCH_2CH_2CN$ oder $-OCH_2CH_2COO(C_1-C_4$-Alkyl) substituiertes $C_1-C_6$-Alkyl, Phenyl, durch Halogen, $C_1-C_{12}$-Alkyl oder $C_1-C_4$-Alkoxy substituiertes Phenyl oder $C_7-C_9$-Phenylalkyl bedeutet, |
| $R^{20}$ und $R^{21}$ | unabhängig voneinander Wasserstoff, $C_1-C_{12}$-Alkyl, $C_2-C_4$-Hydroxyalkyl, $C_2-C_{10}$-Alkoxyalkyl, $C_3-C_5$-Alkenyl, $C_5-C_{12}$-Cycloalkyl, $C_7-C_9$-Phenylalkyl, Phenyl, durch Halogen, $C_1-C_{12}$-Alkyl oder $C_1-C_4$-Alkoxy substituiertes Phenyl, $C_2-C_3$-Alkanoyl oder Benzoyl bedeuten, oder $R^{20}$ und $R^{21}$ zusammen $C_2-C_8$-Alkylen bedeuten, das durch $-O-$, $-S-$ oder $-N(R^{17})-$ unterbrochen sein kann, oder durch Hydroxy, $C_1-C_4$-Alkoxy oder $-COO(C_1-C_4$-Alkyl) substituiert sein kann, und |
| $R^{22}$ | $C_1-C_{18}$-Alkyl, unsubstituiertes oder durch Halogen, $C_1-C_{12}$-Alkyl oder $C_1-C_8$-Alkoxy substituiertes Phenyl oder Naphthyl bedeutet, oder ein Säureadditionssalz einer solchen Verbindung. |

**2.** Eine Verbindung gemäss Anspruch 1 der Formel I, worin $Ar^1$ eine Gruppe der Formel IV, V oder VII ist und $R^1$, $R^2$, $R^3$, $R^4$, $R^5$, $R^6$, $R^7$, $R^8$, $R^9$, $R^{10}$, U, V und W die in Anspruch 1 gegebenen Bedeutungen haben.

**3.** Eine Verbindung gemäss Anspruch 2 der Formel I, worin $Ar^1$ eine Gruppe der Formel IV ist, $R^5$ und $R^6$ Wasserstoff, Halogen,$C_1-C_{12}$-Alkyl oder eine Gruppe $-OR^{18}$, $-SR^{19}$, $-SO-R^{19}$, $-SO_2-R^{19}$, $-N(R^{20})(R^{21})$, $-NH-SO_2-R^{22}$ oder

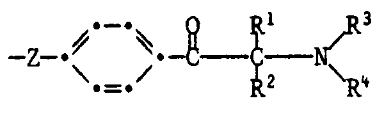

bedeuten, worin Z $-O-$, $-S-$, $-N(R^{11})-$ oder $-N(R^{11})-R^{12}-N(R^{11})-$ oder

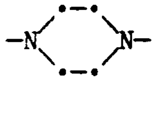

bedeutet,

$R^7$ und $R^8$ Wasserstoff oder Halogen und $R^9$ Wasserstoff, Halogen oder $C_1-C_{12}$-Alkyl sind und $R^1$, $R^2$, $R^3$, $R^4$, $R^{11}$, $R^{12}$, $R^{18}$, $R^{19}$, $R^{20}$ und $R^{22}$ die in Anspruch 1 gegebenen Bedeutungen haben, wobei im Falle, dass $R^1$ Allyl ist und $R^2$ Methyl ist, $R^5$ nicht $-OCH_3$ ist, und im Falle, dass $R^1$ Benzyl ist und $R^2$ Methyl oder Benzyl ist, $R^5$ nicht $-OCH_3$, $-SCH_3$ oder $-SOCH_3$ ist.

**4.** Eine Verbindung gemäss Anspruch 3 der Formel I, worin $Ar^1$ eine Gruppe der Formel IV ist, in der $R^5$ eine Gruppe $-OR^{18}$, $-SR^{19}$, $-N(R^{20})(R^{21})$ oder

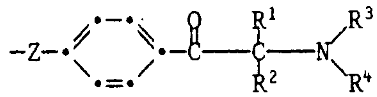

bedeutet, $R^6$ Wasserstoff, Halogen oder $C_1-C_4$-Alkyl bedeutet oder eine der für $R^5$ gegebenen Bedeutungen hat, $R^7$ und $R^8$ Wasserstoff oder Halogen und $R^9$ Wasserstoff oder $C_1-C_4$-Alkyl bedeuten, Z $-O-$, $-S-$ oder $-N(R^{11})-$ bedeutet,

$R^1$ entweder

(a) ein Rest der Formel

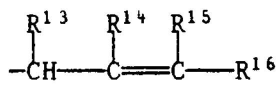

ist oder

(b) ein Rest der Formel $-CH(R^{13})-Ar^2$ ist, worin $Ar^2$ ein unsubstituierter oder durch Halogen, $C_1-C_{12}$-Alkyl, $C_1-C_4$-Alkoxy, $-(OCH_2CH_2)_nOCH_3$ oder Benzoyl substituierter Phenylrest ist, wobei n 1-10 ist,

$R^2$ eine der für $R^1$ gegebenen Bedeutungen hat oder $C_1-C_8$-Alkyl ist,

$R^3$ und $R^4$ unabhängig voneinander $C_1-C_{12}$-Alkyl, durch $C_1-C_4$-Alkoxy, $-CN$ oder $-COO(C_1-C_4$-Alkyl) substituiertes $C_2-C_4$-Alkyl, Allyl, Cyclohexyl oder Benzyl bedeuten oder $R^3$ und $R^4$ zusammen $C_4-C_6$-Alkylen bedeuten, welches durch $-O-$ oder $-N(R^{17})-$ unterbrochen sein kann,

$R^{11}$ Wasserstoff, $C_1-C_4$-Alkyl, Allyl, Benzyl oder $C_2-C_4$-Alkanoyl bedeutet, $R^{13}$, $R^{14}$, $R^{15}$ und $R^{16}$ unabhängig voneinander Wasserstoff oder Methyl bedeuten,

$R^{17}$ Wasserstoff, $C_1-C_4$-Alkyl, Benzyl, 2-Hydroxyethyl oder Acetyl bedeutet,

$R^{18}$ Wasserstoff, $C_1-C_4$-Alkyl, 2-Hydroxyethyl, 2-Methoxyethyl, 2-Allyloxyethyl, Allyl, Cyclohexyl, Phenyl, Benzyl oder $-Si(CH_3)_3$ bedeutet,

$R^{19}$ Wasserstoff, $C_1-C_{12}$-Alkyl, 2-Hydroxyethyl, 2-Methoxyethyl, Phenyl, p-Tolyl oder Benzyl bedeutet,

$R^{20}$ und $R^{21}$ unabhängig voneinander Wasserstoff, $C_1-C_{12}$-Alkyl, $C_2-C_6$-Alkoxy-alkyl, Acetyl, Allyl oder Benzyl bedeuten oder $R^{20}$ und $R^{21}$ zusammen $C_4-C_6$-Alkylen bedeuten, das durch $-O-$ oder $-N(R^{17})-$ unterbrochen sein kann,

wobei im Falle, dass $R^1$ Allyl ist, $R^5$ nicht $-OCH_3$ ist, und im Falle, dass $R^1$ Benzyl ist und $R^2$ Methyl oder Benzyl ist, $R^5$ nicht $-OCH_3$ oder $-SCH_3$ ist.

**5.** Eine Verbindung gemäss Anspruch 4, worin $Ar^1$ eine Gruppe der Formel IV ist, in der $R^5$ eine Gruppe $-OR^{18}$, $-SR^{19}$ $-N(R^{20})(R^{21})$ bedeutet, $R^6$ Wasserstoff, Chlor oder $C_1-C_4$-Alkyl bedeutet oder eine der für $R^5$ gegebenen Bedeutungen hat, $R^7$ und $R^8$ Wasserstoff oder Chlor und $R^9$ Wasserstoff oder $C_1-C_4$-Alkyl bedeuten, $R^1$ entweder (a) ein Rest der Formel $-CH_2-C(R^{14})=CH(R^{15})$ ist oder (b) ein Rest der Formel $-CH_2-Ar^2$ ist, worin $Ar^2$ ein unsubstituierter oder durch Halogen, $C_1-C_{12}$-Alkyl, $C_1-C_4$-Alkoxy, $-(OCH_2CH_2)_nOCH_3$ oder Benzoyl substituierter Phenylrest ist, wobei n 1-10 ist,

$R^2$ eine der für $R^1$ gegebenen Bedeutungen hat oder $C_1-C_8$-Alkyl ist,

$R^3$ und $R^4$ unabhängig voneinander $C_1-C_6$-Alkyl, 2-Methoxyethyl, Allyl oder Benzyl sind oder $R^3$ und $R^4$ zusammen Tetramethylen, Pentamethylen oder 3-Oxapentamethylen bedeuten,

$R^{14}$ und $R^{15}$ Wasserstoff oder Methyl bedeuten,

$R^{18}$ $C_1-C_4$-Alkyl, 2-Hydroxyethyl, 2-Methoxyethyl oder Phenyl bedeutet,

$R^{19}$ $C_1-C_{12}$-Alkyl, 2-Hydroxyethyl, 2-Methoxyethyl, Phenyl oder p-Tolyl bedeutet,

$R^{20}$ und $R^{21}$ Wasserstoff, $C_1-C_4$-Alkyl, 2-Methoxyethyl, Acetyl oder Allyl bedeuten oder

$R^{20}$ und $R^{21}$ zusammen $C_4-C_5$-Alkylen bedeuten, das durch $-O-$ oder $-N(CH_3)-$unterbrochen sein kann,
wobei im Falle, dass $R^1$ Allyl ist, $R^5$ nicht $-OCH_3$ ist, und im Falle, dass $R^1$ Benzyl ist und $R^2$ Methyl oder Benzyl ist, $R^5$ nicht $-OCH_3$ oder $-SCH_3$ ist.

**6.** Eine Verbindung gemäss Anspruch 5, worin $R^5$ eine Gruppe $-SR^{19}$ ist, $R^7$ und $R^8$ Wasserstoff sind, $R^1$ ein Rest der Formel

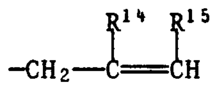

ist,

und alle anderen Substituenten die in Anspruch 5 gegebenen Bedeutungen haben.

**7.** Eine Verbindung gemäss Anspruch 6, worin $R^6$ und $R^9$ Wasserstoff sind und alle anderen Substituenten die in Anspruch 6 gegebenen Bedeutung haben.

**8.** Eine Verbindung gemäss Anspruch 6, worin $R^1$ Allyl ist und alle anderen Substituenten die in Anspruch 6 gegebene Bedeutung haben.

**9.** Eine Verbindung gemäss Anspruch 5, worin $R^5$ eine Gruppe $-N(R^{20})(R^{21})$ ist, $R^7$ und $R^8$ Wasserstoff sind und alle anderen Substituenten die in Anspruch 5 gegebenen Bedeutungen haben.

**10.** Eine Verbindung gemäss Anspruch 9, worin $R^6$ und $R^9$ Wasserstoff sind und alle anderen Substituenten die in Anspruch 9 gegebene Bedeutung haben.

**11.** Eine Verbindung gemäss Anspruch 9, worin $R^1$ Allyl oder Benzyl ist und alle anderen Substituenten die in Anspruch 9 gegebene Bedeutung haben.

**12.** Eine Verbindung gemäss Anspruch 3 der Formel I, worin $Ar^1$ eine Gruppe der Formel IV ist, in der $R^5$ Wasserstoff, Halogen oder $C_1-C_{12}$-Alkyl ist und $R^6$, $R^7$, $R^8$ und $R^9$ Wasserstoff sind, $R^1$ Allyl oder Benzyl bedeutet, $R^2$ $C_1-C_6$-Alkyl, Allyl oder Benzyl bedeutet, $R^3$ und $R^4$ unabhängig voneinander $C_1-C_{12}$-Alkyl, durch $C_1-C_4$-Alkyl, $-CN$ oder $-COO(C_1-C_4$-Alkyl) substituiertes $C_2-C_4$-Alkyl, Allyl, Cyclohexyl oder Benzyl bedeuten oder $R^3$ und $R^4$ zusammen $C_4-C_6$-Alkylen bedeuten, das durch durch $-O-$ oder $-N(R^{17})-$unterbrochen sein kann, und $R^{17}$ Wasserstoff, $C_1-C_4$-Alkyl oder 2-Hydroxyethyl bedeutet.

**13.** Verwendung einer Verbindung des Anspruches 1 als Photoinitiator für die Photopolymerisation ethylenisch ungesättigter Verbindungen.

**14.** Verwendung gemäss Anspruch 13 einer Verbindung des Anspruches 4 als Photoinitiator für die Photohärtung von pigmentierten Systemen wie Druckfarben oder Weisslacke.

**15.** Verwendung gemäss Anspruch 14 einer Verbindung des Anspruches 12 für die Photohärtung von unpigmentierten Systemen.

**16.** Verwendung gemäss Anspruch 13 einer Verbindung des Anspruches 1 als Photoinitiator für die Herstellung von Photoresists oder Druckplatten.

**17.** Verwendung gemäss Anspruch 13 einer Verbindung des Anspruches 1 als Photoinitiator für Aussenanstriche, die im Tageslicht oberflächlich nachhärten.

**18.** Photohärtbare Zusammensetzung, enthaltend
A) mindestens eine ethylenisch ungesättigte photopolymerisierbare Verbindung und
B) als Photoinitiator mindestens eine Verbindung des Anspruches 1.

**19.** Photohärtbare Zusammensetzung gemäss Anspruch 18, enthaltend

A) mindestens eine ethylenisch ungesättigte photopolymerisierbare Verbindung,
B) als Photoinitiator mindestens eine Verbindung des Anspruches 4 und
C) ein weisses oder farbiges Pigment.

**20.** Photohärtbare Zusammensetzung gemäss Anspruch 19, enthaltend
A) mindestens eine ethylenisch ungesättigte photopolymerisierbare Verbindung,
B) als Photoinitiator mindestens eine Verbindung des Anspruches 4,
C) ein weisses oder farbiges Pigment und
D) als Photosensibilisator eine aromatische Carbonylverbindung aus der Klasse der Benzophenone, Thioxanthone, Anthrachinone, 3 – Acylcumarine und 3 – (Aroylmethylen) – thiazoline.

**21.** Photohärtbares Gemisch gemäss Anspruch 18, enthaltend
A) mindestens eine ethylenisch ungesättigte photopolymerisierbare Verbindung und
B) als Photoinitiator ein Gemisch von
$B_1$) mindestens einer Verbindung des Anspruches 1 und
$B_2$) einem Aryl – titanocen – Derivat, das im Arylrest durch Fluor oder $CF_3$ substituiert ist.

**Patentansprüche für den folgenden Vertragsstaat: ES**

**1.** Photohärtbare Zusammensetzung, enthaltend
A) mindestens eine ethylenisch ungesättigte photopolymerisierbare Verbindung und
B) als Photoinitiator mindestens eine Verbindung der Formel I, II, III oder IIIa

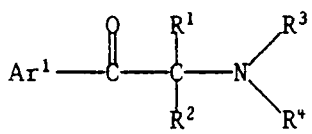
I

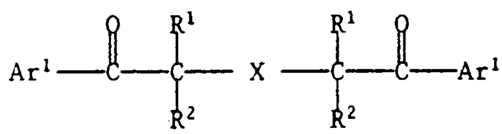
II

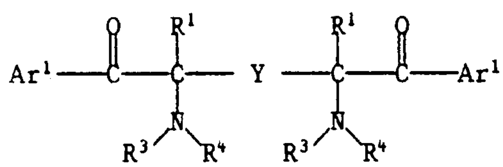
III

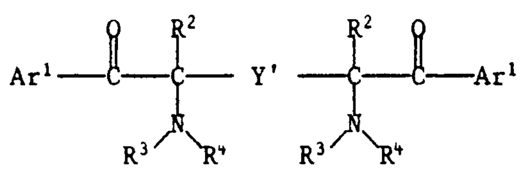
IIIa

worin $Ar^1$ einen aromatischen Rest der Formel IV, V, VI oder VII bedeutet,

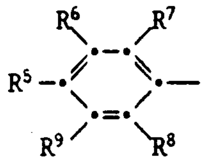 IV,

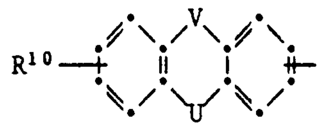 V

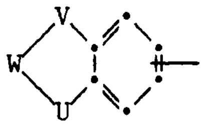 VI

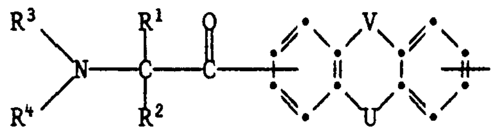 VII

worin

X einen zweiwertigen Rest der Formel

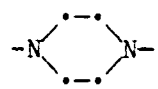 ,

$-N(R^{11})-$ oder $-N(R^{11})-R^{12}-N(R^{11})-$ bedeutet,

Y $C_1-C_6$-Alkylen, Xylylen, Cyclohexylen oder eine direkte Bindung bedeutet,

Y′ Xylylen, $C_4-C_8$-Alkendiyl, $C_6-C_{10}$-Alkadiendiyl, Dipentendiyl oder Dihydroxylylen bedeutet,

U $-O-$, $-S-$ oder $-N(R^{17})-$ bedeutet,

V $-O-$, $-S-$, $-N(R^{17})-$, $-CO-$, $-CH_2-$, $-CH_2CH_2-$, $C_2-C_6$-Alkyliden oder eine direkte Bindung bedeutet,

W unverzweigtes oder verzweigtes $C_1-C_7$-Alkylen oder $C_2-C_6$-Alkyliden bedeutet,

$R^1$ entweder

(a) ein Rest der Formel

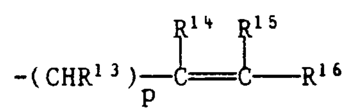

ist,
worin p null oder 1 ist,
oder

(b) ein Rest der Formel

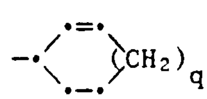

ist, wobei q 0, 1, 2 oder 3 bedeutet
oder

(c) ein Rest der Formel

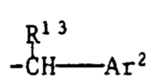

ist, worin $Ar^2$ einen unsubstituierten oder durch Halogen, OH, $C_1-C_{12}$-Alkyl oder durch OH, Halogen, $-N(R^{11})_2$, $-C_1-C_{12}$-Alkoxy,

$-COO(C_1-C_{18}-Alkyl)$, $-CO(OCH_2CH_2)_nOCH_3$ oder $-OCO(C_1-C_4)$-Alkyl substituiertes $C_1-C_4$-Alkyl, $C_1-C_{12}$-Alkoxy oder durch $-COO(C_1-C_{18}-Alkyl)$ oder $-CO(OCH_2CH_2)_nOCH_3$ substituiertes $C_1-C_4$-Alkoxy, $-(OCH_2CH_2)_nOH$, $-(OCH_2CH_2)_nOCH_3$, $C_1-C_8$-Alkylthio, Phenoxy, $-COO(C_1-C_{18}-Alkyl)$, $-CO(OCH_2CH_2)_nOCH_3$, Phenyl oder Benzoyl substituierten Phenyl-, Naphthyl-, Furyl-, Thienyl- oder Pyridylrest bedeutet, worin n 1-20 ist,

oder

(d) zusammen mit $R^2$ einen Rest der Formel

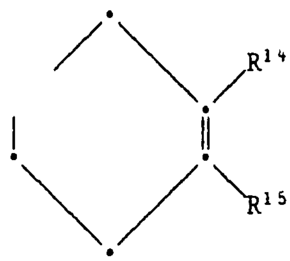

oder

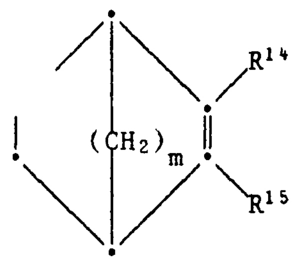

bildet, worin m 1 oder 2 ist,

$R^2$ eine der für $R^1$ gegebenen Bedeutungen hat oder $C_5-C_6$-Cycloalkyl, unsubstituiertes oder durch $C_1-C_4$-Alkoxy, Phenoxy, Halogen oder Phenyl substituiertes $C_1-C_{12}$-Alkyl oder unsubstituiertes oder durch Halogen, $C_1-C_{12}$-Alkyl oder $C_1-C_4$-Alkoxy substituiertes Phenyl bedeutet,

$R^3$ Wasserstoff, $C_1-C_{12}$-Alkyl, durch Hydroxy, $C_1-C_4$Alkoxy, $-CN$ oder $-COO(C_1-C_4-Alkyl)$ substituiertes $C_2-C_4$-Alkyl, $C_3-C_5$-Alkenyl, $C_5-C_{12}$-Cycloalkyl oder $C_7-C_9$-Phenylalkyl bedeutet,

$R^4$ $C_1-C_{12}$-Alkyl, durch Hydroxy, $C_1-C_4$-Alkoxy, $-CN$ oder $-COO-(C_1-C_4-Alkyl)$ substituiertes $C_2-C_4$-Alkyl, $C_3-C_5$-Alkenyl, $C_5-C_{12}$-Cycloalkyl, $C_7-C_9$-Phenylalkyl, Phenyl oder durch Halogen, $C_1-C_{12}$-Alkyl, $C_1-C_4$-Alkoxy oder $-COO(C_1-C_4-Alkyl)$ substituiertes Phenyl bedeutet oder $R^4$ zusammen mit $R^2$ $C_1-C_7$-Alkylen, $C_7-C_{10}$-Phenylalkylen, o-Xylylen, 2-Butenylen oder $C_2-C_3$-Oxa- oder Azaalkylen bedeutet, oder

$R^3$ und $R^4$ zusammen $C_3-C_7$-Alkylen bedeuten, das durch $-O-$, $-S-$, $-CO-$ oder $-N(R^{17})-$ unterbrochen sein kann, oder durch Hydroxy, $C_1-C_4$-Alkoxy oder $-COO(C_1-C_4-Alkyl)$ substituiert sein kann,

$R^5$, $R^6$, $R^7$, $R^8$ und $R^9$ unabhängig voneinander Wasserstoff, Halogen, $C_1-C_{12}$-Alkyl, $C_5-C_6$-Cycloalkyl, Phenyl, Benzyl, Benzoyl oder eine Gruppe $-OR^{18}$, $-SR^{19}$, $-SO-R^{19}$, $-SO_2-R^{19}$, $-N(R^{20})(R^{21})$, $-NH-SO_2-R^{22}$ oder

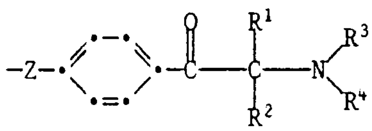

bedeuten, worin Z $-O-$, $-S-$, $-N(R^{11})-$, $-N(R^{11})-R^{12}-N(R^{11})-$ oder

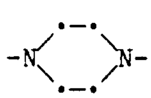

bedeutet, wobei im Falle, dass $R^1$ Allyl und $R^2$ Methyl ist, $R^5$ nicht $-OCH_3$ ist, und im Falle, dass $R^1$ Benzyl ist und

$R^2$ Methyl oder Benzyl ist, $R^5$ nicht $-OCH_3$, $-SCH_3$ oder $-SO-CH_3$ ist,

$R^{10}$ Wasserstoff, $C_1-C_{12}$-Alkyl, Halogen oder $C_2-C_8$-Alkanoyl bedeutet,

$R^{11}$ Wasserstoff, $C_1-C_8$-Alkyl, $C_3-C_5$-Alkenyl, $C_7-C_9$-Phenylalkyl, $C_1-C_4$-Hydroxyalkyl oder Phenyl bedeutet,

$R^{12}$ unverzweigtes oder verzweigtes $C_2-C_{16}$-Alkylen, das durch ein oder mehrere $-O-$, $-S-$ oder $-N(R^{11})-$ unterbrochen sein kann,

$R^{13}$ Wasserstoff, $C_1-C_8$-Alkyl oder Phenyl bedeutet,

$R^{14}$, $R^{15}$ und $R^{16}$ unabhängig voneinander Wasserstoff oder $C_1-C_4$-Alkyl bedeuten oder $R^{14}$ und $R^{15}$ zusammen $C_3-C_7$-Alkylen sind,

$R^{17}$ Wasserstoff, $C_1-C_{12}$-Alkyl, das durch ein oder mehrere $-O-$ unterbrochen sein kann, $C_3-C_5$-Alkenyl, $C_7-C_9$-Phenylalkyl, $C_1-C_4$-Hydroxyalkyl, $-CH_2CH_2CN$, $-CH_2CH_2COO(C_1-C_4\text{-Alkyl})$, $C_2-C_8$-Alkanoyl oder Benzoyl bedeutet,

$R^{18}$ Wasserstoff, $C_1-C_{12}$-Alkyl, durch $-CN$, $-OH$, $C_1-C_4$-Alkoxy, $C_3-C_6$-Alkenoxy, $-OCH_2CH_2CN$, $-OCH_2CH_2COO(C_1-C_4\text{-Alkyl})$, $-COOH$ oder $-COO(C_1-C_4\text{-Alkyl})$ substituiertes $C_1-C_6$-Alkyl, $-(CH_2CH_2O)_nH$ mit $n = 2-20$, $C_2-C_8$-Alkanoyl, $C_3-C_{12}$-Alkenyl, Cyclohexyl, Hydroxycyclohexyl, Phenyl, durch Halogen, $C_1-C_{12}$-Alkyl oder $C_1-C_4$-Alkoxy substituiertes Phenyl, $C_7-C_9$-Phenylalkyl oder $-Si(C_1-C_8\text{-Alkyl})_r(\text{Phenyl})_{3-r}$ mit $r = 1$, 2 oder 3 bedeutet,

$R^{19}$ Wasserstoff, $C_1-C_{12}$-Alkyl, $C_3-C_{12}$-Alkenyl, Cyclohexyl, durch $-SH$, $-OH$, $-CN$, $-COO(C_1-C_4\text{-Alkyl})$, $C_1-C_4$-Alkoxy, $-OCH_2CH_2CN$ oder $-OCH_2CH_2COO(C_1-C_4\text{-Alkyl})$ substituiertes $C_1-C_6$-Alkyl, Phenyl, durch Halogen, $C_1-C_{12}$-Alkyl oder $C_1-C_4$-Alkoxy substituiertes Phenyl oder $C_7-C_9$-Phenylalkyl bedeutet,

$R^{20}$ und $R^{21}$ unabhängig voneinander Wasserstoff, $C_1-C_{12}$-Alkyl, $C_2-C_4$-Hydroxyalkyl, $C_2-C_{10}$-Alkoxyalkyl, $C_3-C_5$-Alkenyl, $C_5-C_{12}$-Cycloalkyl, $C_7-C_9$-Phenylalkyl, Phenyl, durch Halogen, $C_1-C_{12}$-Alkyl oder $C_1-C_4$-Alkoxy substituiertes Phenyl, $C_2-C_3$-Alkanoyl oder Benzoyl bedeuten, oder
$R^{20}$ und $R^{21}$ zusammen $C_2-C_8$-Alkylen bedeuten, das durch $-O-$, $-S-$ oder $-N(R^{17})-$ unterbrochen sein kann, oder durch Hydroxy, $C_1-C_4$-Alkoxy oder $-COO(C_1-C_4\text{-Alkyl})$ substituiert sein kann, und

$R^{22}$ $C_1-C_{18}$-Alkyl, unsubstituiertes oder durch Halogen, $C_1-C_{12}$-Alkyl oder $C_1-C_8$-Alkoxy substituiertes Phenyl oder Naphthyl bedeutet, oder ein Säureadditionssalz einer solchen Verbindung.

**2.** Photohärtbare Zusammensetzung gemäss Anspruch 1, enthaltend
A) mindestens eine ethylenisch ungesättigte photopolymerisierbare Verbindung,
B) als Photoinitiator mindestens eine Verbindung des Anspruches 1 und
C) ein weisses oder farbiges Pigment.

**3.** Photohärtbare Zusammensetzung gemäss Anspruch 2, enthaltend
A) mindestens eine ethylenisch ungesättigte photopolymerisierbare Verbindung,
B) als Photoinitiator mindestens eine Verbindung des Anspruches 1,
C) ein weisses oder farbiges Pigment und
D) als Photosensibilisator eine aromatische Carbonylverbindung aus der Klasse der Benzophenone, Thioxanthone, Anthrachinone, 3-Acylcumarine und 3-(Aroylmethylen)-thiazoline.

**4.** Photohärtbares Gemisch gemäss Anspruch 1, enthaltend
A) mindestens eine ethylenisch ungesättigte photopolymerisierbare Verbindung und
B) als Photoinitiator ein Gemisch von
$B_1$) mindestens einer Verbindung des Anspruches 1 und
$B_2$) einem Aryl-titanocen-Derivat, das im Arylrest durch Fluor oder $CF_3$ substituiert ist.

## Claims

### Claims for the following Contracting States : AT, BE, CH, DE, FR, GB, IT, LI, NL, SE

**1.** A compound of the formula I, II, III or IIIa

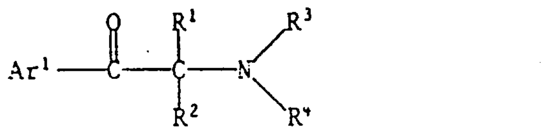

I

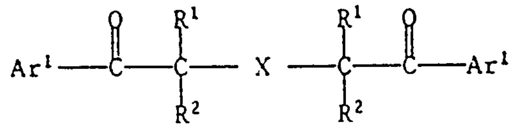

II

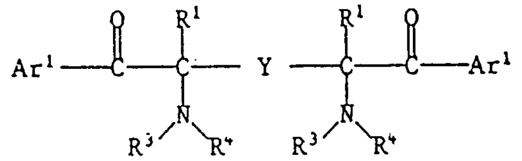

III

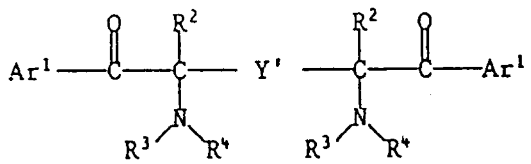

IIIa

in which $Ar^1$ is an aromatic radical of the formula IV, V, VI or VII,

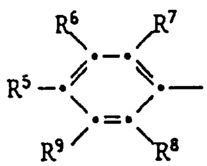

IV,

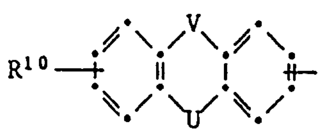

V

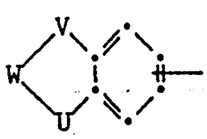

VI

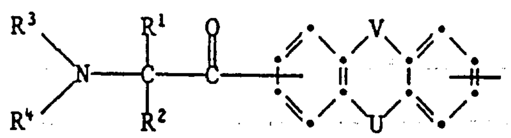

VII

in which X is a divalent radical of the formula

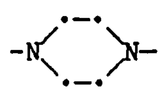

,

$-N(R^{11})-$ or $-N(R^{11})-R^{12}-N(R^{11})-$

Y is $C_1-C_6$alkylene, xylylene, cyclohexylene or a direct bond, Y' is xylylene, $C_4-C_8$alkenediyl, $C_6-C_{10}$alkadienediyl, dipentenediyl or dihyroxylylene, U is $-O-$, $-S-$ or $-N(R^{17})-$, V is $-O-$, $-S-$, $-N(R^{17})-$, $-CO-$, $-CH_2-$, $-CH_2CH_2-$, $C_2H_6$alkylidene or a direct bond, W is unbranched or branched $C_1-C_7$alkylene or $C_2-C_6$alkylidene, $R^1$ is either

(a) a radical of the formula

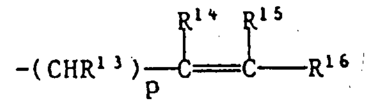

in which p is zero or 1, or (b) a radical of the formula

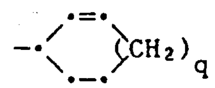

in which q is 0, 1, 2 or 3, or (c) a radical of the formula

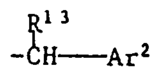

in which $Ar^2$ is a phenyl, naphthyl, furyl, thienyl or pyridyl radical which is unsubstituted or substituted by halogen, OH, $C_1-C_{12}$alkyl, $C_1-C_4$alkyl which is substituted by OH, halogen, $-N(R^{11})_2$, $-C_1-C_{12}$alkoxy, $-COO(C_1-C_{18}$alkyl), $-CO(OCH_2CH_2)_nOCH_3$ or $-OCO(C_1-C_4)$alkyl, $C_1-C_{12}$alkoxy, $C_1-C_4$alkoxy which is substituted by $-COO(C_1-C_{18}$alkyl) or $-CO(OCH_2CH_2)_nOCH_3$, $-(OCH_2CH_2)_nOH$, $-(OCH_2CH_2)_nOCH_3$, $C_1-C_8$alkylthio, phenoxy, $-COO(C_1-C_{18}$alkyl), $-CO(OCH_2CH_2)_nOCH_3$, phenyl or benzoyl, in which n is 1 – 20, or (d) together with $R^2$ forms a radical of the formula

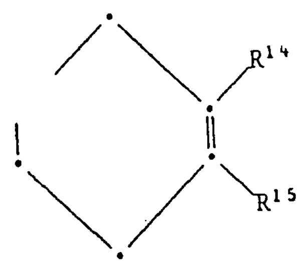

or

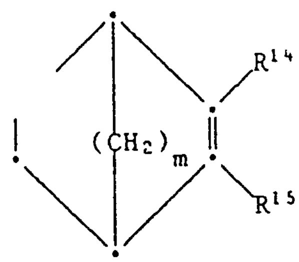

in which m is 1 or 2, $R^2$ has one of the meanings given for $R^1$ or is $C_5-C_6$cycloalkyl, $C_1-C_{12}$alkyl which is unsubstituted or substituted by $C_1-C_4$alkoxy, phenoxy, halogen or phenyl, or phenyl which is unsubstituted or substituted by halogen, $C_1-C_{12}$alkyl or $C_1-C_4$alkoxy, $R^3$ is hydrogen, $C_1-C_{12}$alkyl, $C_2-C_4$alkyl which is substituted by hydroxyl, $C_1-C_4$alkoxy, $-CN$ or $-COO(C_1-C_4$alkyl), $C_3-C_5$alkenyl, $C_5-C_{12}$cycloalkyl or $C_7-C_9$phenylalkyl, $R^4$ is $C_1-C_{12}$alkyl, $C_2-C_4$alkyl which is substituted by hydroxyl, $C_1-C_4$alkoxy, $-CN$ or $-COO(C_1-C_4$alkyl), $C_3-C_5$alkenyl, $C_5-C_{12}$cycloalkyl, $C_7-C_9$phenylalkyl, phenyl or phenyl which is substituted by halogen, $C_1-C_{12}$alkyl, $C_1-C_4$alkoxy or $-COO(C_1-C_4$alkyl) or $R^4$ together with $R^2$ is $C_1-C_7$alkylene, $C_7-C_{10}$phenylalkylene, o-xylylene, 2-butenylene or $C_2-C_3$oxa- or -azaalkylene, or $R^3$ and $R^4$ together are $C_3-C_7$alkylene, which can be interrupted by $-O-$, $-S-$, $-CO-$ or $-N(R^{17})-$ or can be substituted by hydroxyl, $C_1-C_4$alkoxy or $-COO(C_1-C_4$alkyl), $R^5$, $R^6$, $R^7$, $R^8$, and $R^9$ independently of one another are hydrogen, halogen, $C_1-C_{12}$alkyl, $C_5-C_6$cycloalkyl, phenyl, benzyl, benzoyl or a group $-OR^{18}$, $-SR^{19}$, $-SO-R^{19}$, $-SO_2-R^{19}$, $-N(R^{20})(R^{21})$, $-NH-SO_2-R^{22}$ or

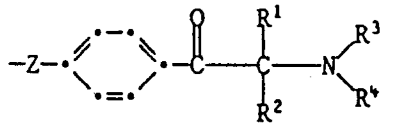

in which Z is $-O-$, $-S-$, $-N(R^{11})$, $-N(R^{11})-R^{12}-N(R^{11})-$ or

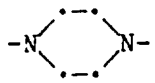

in which, in the case where $R^1$ is allyl and $R^2$ is methyl, $R^5$ is not $-OCH_3$, and in the case where $R^1$ is benzyl and $R^2$ is methyl or benzyl, $R^5$ is not $-OCH_3$, $-SCH_3$ or $-SO-CH_3$, $R^{10}$ is hydrogen, $C_1-C_{12}$alkyl, halogen or $C_2-C_8$alkanoyl, $R^{11}$ is hydrogen, $C_1-C_8$alkyl, $C_3-C_5$alkenyl, $C_7-C_9$phenylalkyl, $C_1-C_4$hydroxyalkyl or phenyl, $R^{12}$ is unbranched or branched $C_2-C_{16}$alkylene, which can be interrupted by one or more $-O$, $-S-$ or $-N(R^{11})-$, $R^{13}$ is hydrogen, $C_1-C_8$alkyl or phenyl, $R^{14}$, $R^{15}$ and $R^{16}$ independently of one another are hydrogen or $C_1-C_4$alkyl, or $R^{14}$ and $R^{15}$ together are $C_3-C_7$alkylene, $R^{17}$ is hydrogen, $C_1-C_{12}$alkyl, which can be interrupted by one or more $-O-$, $C_3-C_5$alkenyl, $C_7-C_9$phenylalkyl, $C_1-C_4$hydroxyalkyl, $-CH_2CH_2CN$, $-CH_2CH_2COO(C_1-C_4alkyl)$, $C_2-C_8$alkanoyl or benzoyl, $R^{18}$ is hydrogen, $C_1-C_{12}$alkyl, $C_1-C_6$alkyl which is substituted by $-CN$, $-OH$, $C_1-C_4$alkoxy, $C_3-C_6$alkenoxy, $-OCH_2CH_2CN$, $-OCH_2CH_2COO(C_1-C_4alkyl)$, $-COOH$ or $-COO-(C_1-C_4alkyl)$, $-(CH_2CH_2O)_nH$ where $n = 2-20$, $C_2-C_8$alkanoyl, $C_3-C_{12}$alkenyl, cyclohexyl, hydroxycyclohexyl, phenyl, phenyl which is substituted by halogen, $C_1-C_{12}$alkyl or $C_1-C_4$alkoxy, $C_7-C_9$phenylalkyl or $-Si(C_1-C_8alkyl)_r(phenyl)_{3-r}$ where $r = 1$, 2 or 3, $R^{19}$ is hydrogen, $C_1-C_{12}$alkyl, $C_3-C_{12}$alkenyl, cyclohexyl, $C_1-C_6$alkyl which is substituted by $-SH$, $-OH$, $-CN$, $-COO(C_1-C_4alkyl)$, $C_1-C_4$alkoxy, $-OCH_2CH_2CN$ or $-OCH_2CH_2COO(C_1-C_4alkyl)$, phenyl, phenyl which is substituted by halogen, $C_1-C_{12}$alkyl or $C_1-C_4$alkoxy or $C_7-C_9$phenylalkyl, $R^{20}$ and $R^{21}$ independently of one another are hydrogen, $C_1-C_{12}$alkyl, $C_2-C_4$hydroxyalkyl, $C_2-C_{10}$alkoxyalkyl, $C_3-C_5$alkenyl, $C_5-C_{12}$cycloalkyl, $C_7-C_9$phenylalkyl, phenyl, phenyl which is substituted by halogen, $C_1-C_{12}$alkyl or $C_1-C_4$alkoxy, $C_2-C_3$alkanoyl or benzoyl, or $R^{20}$ and $R^{21}$ together are $C_2-C_8$alkylene, which can be interrupted by $-O-$, $-S-$ or $-N(R^{17})-$, or can be substituted by hydroxyl, $C_1-C_4$alkoxy or $-COO(C_1-C_4alkyl)$ and $R^{22}$ is $C_1-C_{18}$alkyl, phenyl which is unsubstituted or substituted by halogen, $C_1-C_{12}$alkyl or $C_1-C_8$alkoxy or naphthyl, or an acid addition salt of such a compound.

**2.** A compound according to claim 1 of the formula I, in which $Ar^1$ is a group of the formula IV, V or VII and $R^1$, $R^2$, $R^3$, $R^4$, $R^5$, $R^6$, $R^7$, $R^8$, $R^9$, $R^{10}$, U, V and W are as defined in claim 1.

**3.** A compound according to claim 2 of the formula I, in which $Ar^1$ is a group of the formula IV, $R^5$ and $R^6$ are hydrogen, halogen, $C_1-C_{12}$alkyl or a group $-OR^{18}$, $-SR^{19}$, $-SO-R^{19}$, $-SO_2-R^{19}$, $-N(R^{20})-(R^{21})$, $-NH-SO_2-R^{22}$ or

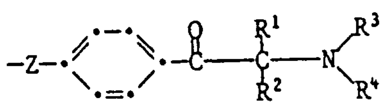

in which Z is $-O-$, $-S-$, $-N(R^{11})-$ or $-N(R^{11})-R^{12}-N(R^{11})-$ or

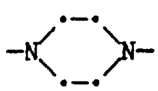

$R^7$ and $R^8$ are hydrogen or halogen, $R^9$ is hydrogen, halogen or $C_1-C_{12}$alkyl and $R^1$, $R^2$, $R^3$, $R^4$, $R^{11}$, $R^{12}$, $R^{18}$, $R^{19}$, $R^{20}$ and $R^{22}$ are as defined in claim 1, and in the case where $R^1$ is allyl and $R^2$ is methyl, $R^5$ is not $-OCH_3$, and in the case where $R^1$ is benzyl and $R^2$ is methyl or benzyl, $R^5$ is not $-OCH_3$, $-SCH_3$ or $-SOCH_3$.

**4.** A compound according to claim 3 of the formula I, in which $Ar^1$ is a group of the formula IV, in which $R^5$ is a group $-OR^{18}$, $-SR^{19}$, $-N(R^{20})(R^{21})$ or

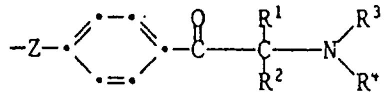

$R^6$ is hydrogen, halogen or $C_1-C_4$alkyl or has one of the meanings given for $R^5$, $R^7$ and $R^8$ are hydrogen or halogen, $R^9$ is hydrogen or $C_1-C_4$alkyl, Z is $-O-$, $-S-$ or $-N(R^{11})-$, $R^1$ is either (a) a radical of the formula

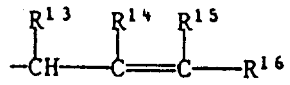

or (b) a radical of the formula $-CH(R^{13})-Ar^2$, in which $Ar^2$ is a phenyl radical which is unsubstituted or substituted by halogen, $C_1-C_{12}$alkyl, $C_1-C_4$alkoxy, $-(OCH_2CH_2)_nOCH_3$ or benzoyl, n being 1-10, $R^2$ has one of the meanings given for $R^1$ or is $C_1-C_8$alkyl, $R^3$ and $R^4$ independently of one another are $C_1-C_{12}$alkyl, $C_2-C_4$alkyl which is substituted by $C_1-C_4$alkoxy, $-CN$ or $-COO(C_1-C_4$alkyl), allyl, cyclohexyl or benzyl, or $R^3$ and $R^4$ together are $C_4-C_6$alkylene, which can be interrupted by $-O-$ or $-N(R^{17})-$, $R^{11}$ is hydrogen, $C_1-C_4$alkyl, allyl, benzyl or $C_2-C_4$alkanoyl, $R^{13}$, $R^{14}$, $R^{15}$ and $R^{16}$ independently of one another are hydrogen or methyl, $R^{17}$ is hydrogen, $C_1-C_4$alkyl, benzyl, 2-hydroxyethyl or acetyl, $R^{18}$ is hydrogen, $C_1-C_4$alkyl, 2-hydroxyethyl, 2-methoxyethyl, 2-allyloxyethyl, allyl, cyclohexyl, phenyl, benzyl or $-Si(CH_3)_3$, $R^{19}$ is hydrogen, $C_1-C_{12}$alkyl, 2-hydroxyethyl, 2-methoxyethyl, phenyl, p-tolyl or benzyl, and $R^{20}$ and $R^{21}$ independently of one another are hydrogen, $C_1-C_{12}$alkyl, $C_2-C_6$alkoxyalkyl, acetyl, allyl or benzyl, or $R^{20}$ and $R^{21}$ together are $C_4-C_6$alkylene, which can be interrupted by $-O-$ or $-N(R^{17})-$, and, in the case where $R^1$ is allyl, $R^5$ is not $-OCH_3$, and in the case where $R^1$ is benzyl and $R^2$ is methyl or benzyl, $R^5$ is not $-OCH_3$ or $-SCH_3$.

**5.** A compound according to claim 4, in which $Ar^1$ is a group of the formula IV in which $R^5$ is a group $-OR^{18}$, $-SR^{19}$ or $-N(R^{20})(R^{21})$, $R^6$ is hydrogen, chlorine or $C_1-C_4$alkyl or has one of the meanings given for $R^5$, $R^7$ and $R^8$ are hydrogen or chlorine, $R^9$ is hydrogen or $C_1-C_4$alkyl, $R^1$ is either (a) a radical of the formula $-CH_2-C(R^{14})=CH(R^{15})$ or (b) a radical or the formula $-CH_2-Ar^2$, in which $Ar^2$ is a phenyl radical which is unsubstituted or substituted by halogen, $C_1-C_{12}$alkyl, $C_1-C_4$alkoxy, $-(OCH_2CH_2)_nOCH_3$ or benzoyl, n being 1-10, $R^2$ has one of the meanings given for $R^1$ or is $C_1-C_8$alkyl, $R^3$ and $R^4$ independently of one another are $C_1-C_6$alkyl, 2-methoxyethyl, allyl or benzyl, or $R^3$ and $R^4$ together are tetramethylene, pentamethylene or 3-oxapentamethylene, $R^{14}$ and $R^{15}$ are hydrogen or methyl, $R^{18}$ is $C_1-C_4$alkyl, 2-hydroxyethyl, 2-methoxyethyl or henyl, $R^{19}$ is $C_1-C_{12}$alkyl, 2-hydroxyethyl, 2-methoxyethyl, phenyl or p-tolyl and $R^{20}$ and $R^{21}$ are hydrogen, $C_1-C_4$alkyl, 2-methoxyethyl, acetyl or allyl, or $R^{20}$ and $R^{21}$ together are $C_4-C_5$alkylene, which can be interrupted by $-O-$ or $-N(CH_3)-$, and in the case where $R^1$ is allyl, $R^5$ is not $-OCH_3$, and in the case where $R^1$ is benzyl and $R^2$ is methyl or benzyl, $R^5$ is not $-OCH_3$ or $-SCH_3$.

**6.** A compound according to claim 5, in which $R^5$ is a group $-SR^{19}$, $R^7$ and $R^8$ are hydrogen, $R^1$ is a radical of the formula

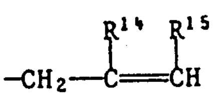

and all the other substituents are as defined in claim 5.

**7.** A compound according to claim 6, in which $R^6$ and $R^9$ are hydrogen and all the other substituents are as defined in claim 6.

**8.** A compound according to claim 6, in which $R^1$ is allyl and all the other substituents are as defined in claim 6.

9. A compound according to claim 5, in which $R^5$ is a group $-N(R^{20})(R^{21})$, $R^7$ and $R^8$ are hydrogen and all the other substituents are as defined in claim 5.

10. A compound according to claim 9, in which $R^6$ and $R^9$ are hydrogen and all the other substituents are as defined in claim 9.

11. A compound according to claim 9, in which $R^1$ is allyl or benzyl and all the other substituents are as defined in claim 9.

12. A compound according to claim 3 of the formula I, in which $Ar^1$ is a group of the formula IV in which $R^5$ is hydrogen, halogen or $C_1-C_{12}$alkyl, $R^6$, $R^7$, $R^8$ and $R^9$ are hydrogen, $R^1$ is allyl or benzyl, $R^2$ is $C_1-C_6$alkyl, allyl or benzyl, $R^3$ and $R^4$ independently of one another are $C_1-C_{12}$alkyl, $C_2-C_4$alkyl which is substituted by $C_1-C_4$alkoxy, $-CN$ or $-COO(C_1-C_4$alkyl), allyl, cyclohexyl or benzyl, or $R^3$ and $R^4$ together are $C_4-C_6$alkylene, which can be interrupted by $-O-$ or $-N(R^{17})-$, and $R^{17}$ is hydrogen, $C_1-C_4$alkyl or 2-hydroxyethyl.

13. Use of a compound of claim 1 as a photoinitiator for the photopolymerization of ethylenically unsaturated compounds.

14. Use according to claim 13 of a compound of claim 4 as a photoinitiator for photocuring pigmented systems such as printing inks or white lacquers.

15. Use according to claim 14 of a compound of claim 12 for photocuring unpigmented systems.

16. Use according to claim 13 of a compound of claim 1 as a photoinitiator for the production of photoresists or printing plates.

17. Use according to claim 13 of a compound of claim 1 as a photoinitiator for external paint films which subsequently harden on their surface in daylight.

18. A photocurable composition containing A) at least one ethylenically unsaturated photopolymerizable compound and B) at least one compound of claim 1 as a photoinitiator.

19. A photocurable composition according to claim 18, containing A) at least one ethylenically unsaturated photopolymerizable compound, B) at least one compound of claim 4 as a photoinitiator and C) a white or coloured pigment.

20. A photocurable composition according to claim 19, containing A) at least one ethylenically unsaturated photopolymerizable compound, B) at least one compound of claim 4 as a photoinitiator, C) a white or coloured pigment and D) an aromatic carbonyl compound from the benzophenone, thioxanthone, anthraquinone, 3-acylcoumarin or 3-(aroylmethylene)-thiazoline class as a photosensitizer.

21. A photocurable mixture according to claim 18, containing A) at least one ethylenically unsaturated photopolymerizable compound and B) a mixture of $B_1$) at least one compound of claim 1 and $B_2$) an aryltitanocene derivative which is substituted by fluorine or $CF_3$ in the aryl radical, as a photoinitiator.

**Claims for the following Contracting State : ES**

1. A photocurable composition containing A) at least one ethylenically unsaturated photopolymerizable compound and B) as a photoinitiator at least one compound of the formula I, II, III or IIIa

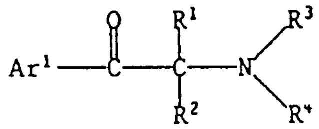

I

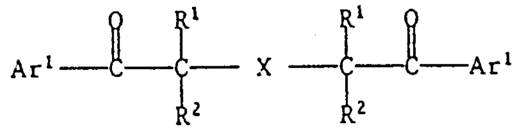

II

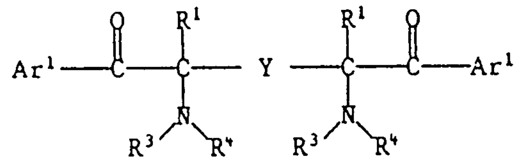

III

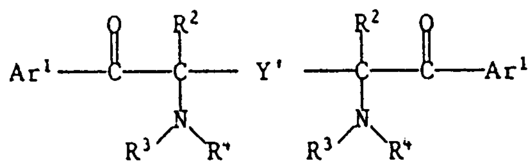

IIIa

in which $Ar^1$ is an aromatic radical of the formula IV, V, VI or VII,

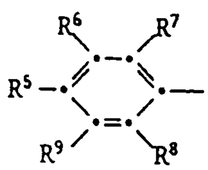

IV,

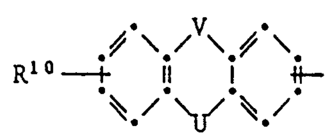

V

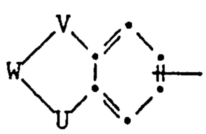

VI

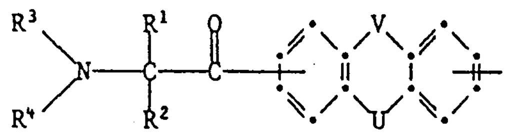

VII

in which X is a divalent radical of the formula

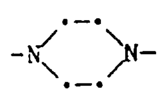

,

$-N(R^{11})-$ or $-N(R^{11})-R^{12}-N(R^{11})-$ Y is $C_1-C_6$alkylene, xylylene, cyclohexylene or a direct bond, Y' is xylylene, $C_4-C_8$alkenediyl, $C_6-C_{10}$alkadienediyl, dipentenediyl or dihyroxylylene, U is $-O-$, $-S-$ or $-N(R^{17})-$, V is $-O-$, $-S-$, $-N(R^{17})-$, $-CO-$, $-CH_2-$, $-CH_2CH_2-$, $C_2-C_6$alkylidene or a direct bond, W is unbranched or branched $C_1-C_7$alkylene or $C_2-C_6$alkylidene, $R^1$ is either
(a) a radical of the formula

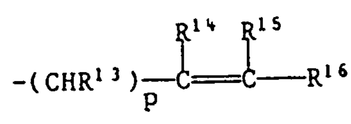

in which p is zero or 1, or (b) a radical of the formula

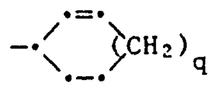

in which q is 0, 1, 2 or 3, or (c) a radical of the formula

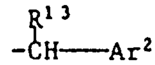

in which $Ar^2$ is a phenyl, naphthyl, furyl, thienyl or pyridyl radical which is unsubstituted or substituted by halogen, OH, $C_1-C_{12}$alkyl, $C_1-C_4$alkyl which is substituted by OH, halogen, $-N(R^{11})_2$, $-C_1-C_{12}$alkoxy, $-COO(C_1-C_{18}$alkyl), $-CO(OCH_2CH_2)_nOCH_3$ or $-OCO(C_1-C_4)$alkyl, $C_1-C_{12}$alkoxy, $C_1-C_4$alkoxy which is substituted by $-COO(C_1-C_{18}$alkyl) or $-CO(OCH_2CH_2)_nOCH_3$, $-(OCH_2CH_2)_nOH$, $-(OCH_2CH_2)_nOCH_3$, $C_1-C_8$alkylthio, phenoxy, $-COO(C_1-C_{18}$alkyl), $-CO(OCH_2CH_2)_nOCH_3$, phenyl or benzoyl, in which n is 1 − 20, or (d) together with $R^2$ forms a radical of the formula

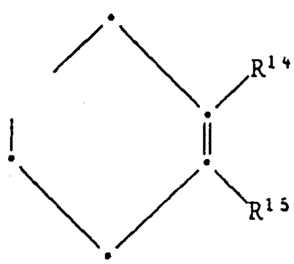

or

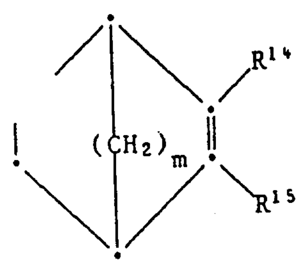

in which m is 1 or 2, $R^2$ has one of the meanings given for $R^1$ or is $C_5-C_6$cycloalkyl, $C_1-C_{12}$alkyl which is unsubstituted or substituted by $C_1-C_4$alkoxy, phenoxy, halogen or phenyl, or phenyl which is unsubstituted or substituted by halogen, $C_1-C_{12}$alkyl or $C_1-C_4$alkoxy, $R^3$ is hydrogen, $C_1-C_{12}$alkyl, $C_2-C_4$alkyl which is substituted by hydroxyl, $C_1-C_4$alkoxy, $-CN$ or $-COO(C_1-C_4$alkyl), $C_3-C_5$alkenyl, $C_5-C_{12}$cycloalkyl or $C_7-C_9$phenylalkyl, $R^4$is $C_1-C_{12}$alkyl, $C_2-C_4$alkyl which is substituted by hydroxyl, $C_1-C_4$alkoxy, $-CN$ or $-COO(C_1-C_4$alkyl), $C_3-C_5$alkenyl, $C_5-C_{12}$cycloalkyl, $C_7-C_9$phenylalkyl, phenyl or phenyl which is substituted by halogen, $C_1-C_{12}$alkyl, $C_1-C_4$alkoxy or $-COO(C_1-C_4$alkyl) or $R^4$ together with $R^2$ is $C_1-C_7$alkylene, $C_7-C_{10}$phenylalkylene, o−xylylene, 2−butenylene or $C_2-C_3$oxa− or −azaalkylene, or $R^3$ and $R^4$ together are $C_3-C_7$akylene, which can be interrupted by −O−, −S−, −CO− or $-N(R^{17})-$ or can be substituted by hydroxyl, $C_1-C_4$alkoxy or $-COO(C_1-C_4$alkyl), $R^5$, $R^6$, $R^7$, $R^8$, and $R^9$ independently of one another are hydrogen, halogen, $C_1-C_{12}$alkyl, $C_5-C_6$cycloalkyl, phenyl, benzyl, benzoyl or a group $-OR^{18}$, $-SR^{19}$, $-SO-R^{19}$, $-SO_2-R^{19}$, $-N(R^{20})(R^{21})$, $-NH-SO_2-R^{22}$ or

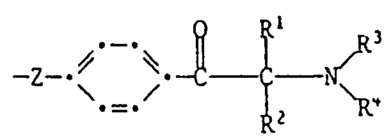

in which Z is −O−, −S−, $-N(R^{11})-$, $-N(R^{11})-R^{12}-N(R^{11})-$ or

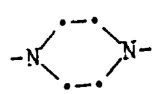

in which, in the case where $R^1$ is allyl and $R^2$ is methyl, $R^5$ is not $-OCH_3$, and in the case where $R^1$ is benzyl and $R^2$ is methyl or benzyl, $R^5$ is not $-OCH_3$, $-SCH_3$ or $-SO-CH_3$, $R^{10}$ is hydrogen, $C_1-C_{12}$alkyl, halogen or $C_2-C_8$alkanoyl, $R^{11}$ is hydrogen, $C_1-C_8$alkyl, $C_3-C_5$alkenyl, $C_7-C_9$phenylalkyl,

$C_1-C_4$hydroxyalkyl or phenyl, $R^{12}$ is unbranched or branched $C_2-C_{16}$alkylene, which can be interrupted by one or more $-O-$, $-S-$ or $-N(R^{11})-$, $R^{13}$ is hydrogen, $C_1-C_8$alkyl or phenyl, $R^{14}$, $R^{15}$ and $R^{16}$ independently of one another are hydrogen or $C_1-C_4$alkyl, or $R^{14}$ and $R^{15}$ together are $C_3-{}_7$alkylene, $R^{17}$ is hydrogen, $C_1-C_{12}$alkyl, which can be interrupted by one or more $-O-$, $C_3-C_5$alkenyl, $C_7-C_9$phenylalkyl, $C_1-C_4$hydroxyalkyl, $-CH_2CH_2CN$, $-CH_2CH_2COO(C_1-C_4$alkyl), $C_2-C_8$alkanoyl or benzoyl, $R^{18}$ is hydrogen, $C_1-C_{12}$alkyl, $C_1-C_6$alkyl which is substituted by $-CN$, $-OH$, $C_1-C_4$alkoxy, $C_3-C_6$alkenoxy, $-OCH_2CH_2CN$, $-OCH_2CH_2COO(C_1-C_4$alkyl), $-COOH$ or $-COO-(C_1-C_4$alkyl), $-(CH_2CH_2O)_nH$ where n = 2-20, $C_2-C_8$alkanoyl, $C_3-C_{12}$alkenyl, cyclohexyl, hydroxycyclohexyl, phenyl, phenyl which is substituted by halogen, $C_1-C_{12}$alkyl or $C_1-C_4$alkoxy, $C_7-C_9$phenylalkyl or $-Si(C_1-C_8$alkyl$)_r$(phenyl$)_{3-r}$ where r = 1, 2 or 3, $R^{19}$ is hydrogen, $C_1-C_{12}$alkyl, $C_3-C_{12}$alkenyl, cyclohexyl, $C_1-C_6$alkyl which is substituted by $-SH$, $-OH$, $-CN$, $-COO(C_1-C_4$alkyl), $C_1-C_4$alkoxy, $-OCH_2CH_2CN$ or $-OCH_2CH_2COO(C_1-C_4$alkyl), phenyl, phenyl which is substituted by halogen, $C_1-C_{12}$alkyl or $C_1-C_4$alkoxy or $C_7-C_9$phenylalkyl, $R^{20}$ and $R^{21}$ independently of one another are hydrogen, $C_1-C_{12}$alkyl, $C_2-C_4$hydroxyalkyl, $C_2-C_{10}$alkoxyalkyl, $C_3-C_5$alkenyl, $C_5-C_{12}$cycloalkyl, $C_7-C_9$phenylalkyl, phenyl, phenyl which is substituted by halogen, $C_1-C_{12}$alkyl or $C_1-C_4$alkoxy, $C_2-C_3$alkanoyl or benzoyl, or $R^{20}$ and $R^{21}$ together are $C_2-C_8$alkylene, which can be interrupted by $-O-$, $-S-$ or $-N(R^{17})-$, or can be substituted by hydroxyl, $C_1-C_4$alkoxy or $-COO(C_1-C_4$alkyl) and $R^{22}$ is $C_1-C_{18}$alkyl, phenyl which is unsubstituted or substituted by halogen, $C_1-C_{12}$alkyl or $C_1-C_8$alkoxy or naphthyl, or an acid addition salt of such a compound.

**2.** A photocurable composition according to claim 1, containing A) at least one ethylenically unsaturated photopolymerizable compound, B) at least one compound of claim 1 as a photoinitiator and C) a white or coloured pigment.

**3.** A photocurable composition according to claim 2, containing A) at least one ethylenically unsaturated photopolymerizable compound, B) at least one compound of claim 1 as a photoinitiator, C) a white or coloured pigment and D) an aromatic carbonyl compound from the benzophenone, thioxanthone, anthraquinone, 3-acylcoumarin or 3-(aroylmethylene)-thiazoline class as a photosensitizer.

**4.** A photocurable mixture according to claim 1, containing A) at least one ethylenically unsaturated photopolymerizable compound and B) a mixture of $B_1$) at least one compound of claim 1 and $B_2$) an aryltitanocene derivative which is substituted by fluorine or $CF_3$ in the aryl radical, as a photoinitiator.

**Revendications**

**Revendications pour les Etats contractants suivants : AT, BE, CH, DE, FR, GB, IT, LI, NL, SE**

**1.** Composé de formule 1, II, III, ou III a,

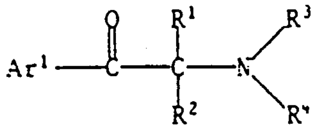
I

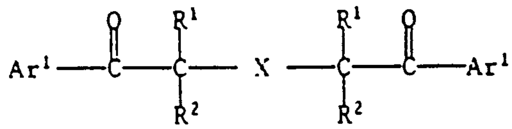
II

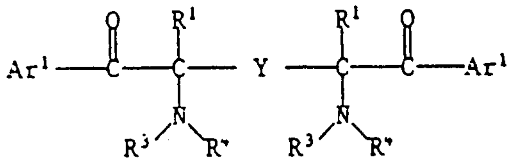
III

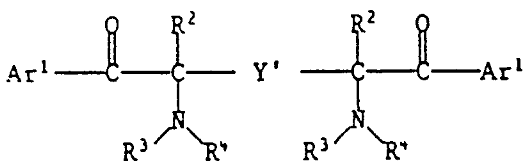
IIIa

dans lesquelles $Ar^1$ représente un reste aromatique répondant aux formules IV, V, VI ou VII :

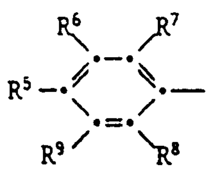
IV,

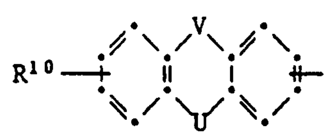
V

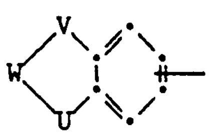
VI

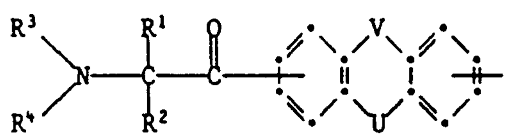
VII

dans lesquelles,
X représente un reste divalent de formule

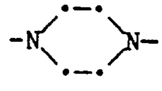
,

$-N(R^{11})-$ ou $-N(R^{11})-R^{12}-N(R^{11})-$

Y représente un groupe alkylène en $C_1$ à $C_6$, xylylène, cyclohexylène ou une liaison directe,

Y' représente un groupe xylylène, alcène diyle en $C_4$ à $C_8$, alcadiène diyle en $C_6$ à $C_{10}$, dipentène diyle ou dihydroxylylène,

U représente $-O-$, $-S-$ ou $-N(R^{17})-$,

V représente $-O-$, $-S-$, $-N(R^{17})-$, $-CO,-CH_2-$, $CH_2CH2-$, un groupe alkylidène en $C_2$ à

$C_6$ ou une liaison directe,

W représente un groupe alkylène en $C_1$ à $C_7$ ou alkylydène en $C_2$ à $C_6$ non ramifiés ou ramifiés,

$R^1$ représente :

(a) un reste de formule

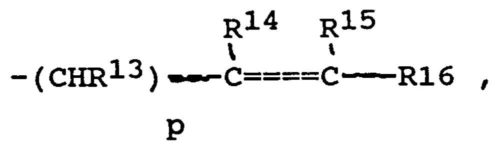

dans laquelle est nul ou vaut 1,

ou ,

(b) un reste de formule

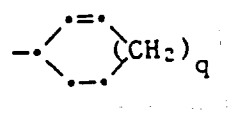

dans laquelle q vaut 0, 1, 2 ou 3,

ou,

(c) un reste de formule

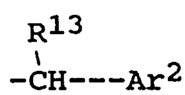

dans laquelle $Ar^2$ représente un reste phényle, naphtyle, furyle, thiényle ou pyridyle non substitué ou substitué par de l'halogène, par un groupe OH, alkyle en $C_1$ à $C_{12}$ ou par un groupe alkyle en $C_1$ à $C_4$ (substitué par OH, par un halogène, par un groupe $-N(R^{11})_2$, $-$alcoxy en $C_1$ à $C_{12}$, $-COO$(alkyle en $C_1$ à $C_{18}$), $-CO(OCH_2CH_2)_nOCH_3$ ou $-OCO-$alkyle (en $C_1$ à $C_4$), un groupe alcoxy en $C_1$ à $C_{12}$, un groupe alcoxy en $C_1$ à $C_4$ (substitué par un reste $-COO$ (alkyle en $C_1$ à $C_{18}$) ou $-CO(OCH_2CH_2)_nOCH_2$), un groupe $-(OCH_2CH_2)_nOH$, $-$ $-(OCH_2CH_2)_nOCH_3$, alkylthio en $C_1$ à $C_8$, phénoxy, $-COO$(alkyle en $C_1$ à $C_{18}$), $-CO(OCH_2CH_2)_nOCH_3$, phényle ou benzyle, l'indice n valant 1 à 20,

Ou

(d) $R^1$ forme avec $R^2$ un reste de formule

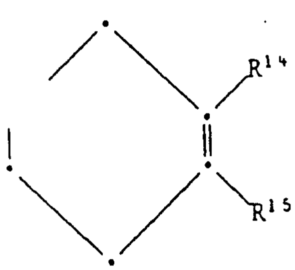

ou

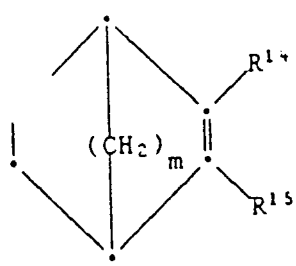

l'indice m valant 1 ou 2,

$R^2$ possède l'un des sens indiqués pour $R^1$ ou représente un groupe cycloalkyle en $C_5$ ou $C_6$, un groupe alkyle en $C_1$ à $C_{12}$ (non substitué ou substitué par un groupe alcoxy en $C_1$ à $C_4$, phénoxy, halogéno ou phényle) ou un groupe phényle (non substitué ou substitué par de l'halogène, par un groupe alkyle en $C_1$ à $C_{12}$ ou alcoxy en $C_1$ à $C_4$),

$R^3$ représente un atome d'hydrogène, un groupe alkyle en $C_1$ à $C_{12}$, un groupe alkyle en $C_2$ à $C_4$ (substitué par un groupe hydroxy, alcoxy en $C_1$ à $C_4$, $-CN$ ou $-COO$(alkyle en $C_1$ à $C_4$)), alcényle en $C_3$ à $C_5$, cycloalkyle en $C_5$ à $C_{12}$ ou phénylalkyle en $C_7$ à $C_9$,

$R^4$ représente un groupe alkyle en $C_1$ à $C_{12}$, un groupe alkyle en $C_2$ à $C_4$ (substitué par un groupe hydroxy, alcoxy en $C_1$ à $C_4$, $-CN$ ou $-COO$ (alkyle en $C_1$ à $C_4$)), alcényle en $C_3$ à $C_5$, cycloalkyle en $C_5$ à $C_{12}$, phénylalkyle en $C_7$ à $C_9$, phényle ou phényle (substitué par de l'halogène, par un groupe alkyle en $C_1$ à $C_{12}$, alcoxy en $C_1$ à $C_4$ ou $-$ COO(alkyle en $_1$1 à $C_4$)), ou bien $R^1$ forme avec $R^2$ un groupe alkylène en $C_1$ à $C_7$, phénylalkylène en $C_7$ à $C_{10}$, o-xylylène, 2-butenylène ou un groupe oxa-ou aza-alkylène en $C_2$ ou $C_3$,

ou bien

$R^3$ et $R^4$ forment ensemble un groupe alkylène en $C_3$ à $C_7$, qui peut être interrompu par $-O-$, $-S-$, $-CO-$ ou $-N(R^{17})-$, ou qui peut porter comme substituant au moins un groupe hydroxy, alcoxy en $C_1$ à $C_4$ ou $-COO$(alkyle en $C_1$ à $C_4$),

$R^5$, $R^6$, $R^7$, $R^8$ et $R^9$ représentent chacun, indépendamment l'un de l'autre, un atome d'hydrogène ou d'halogène, un groupe alkyle en $C_1$ à $C_{12}$, cycloalkyle en $C_5$ ou $C_6$, phényle, benzyle, benzoyle ou un groupe $-OR^{18}$, $-SR^{19}$' $-SOR^{19}$, $-SO_2-R^{19}$, $-N(R^{20})(R^{21})$, $-NH-SO_2-R^{22}$ ou

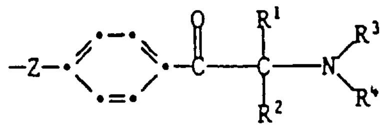

formule dans laquelle Z représente $-O-$, $-S-$, $-N(R^{11})-$, $-$ $N(R^{11})-R^{12}-N(R^{11})-$ ou

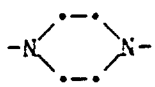

et si $R^1$ represente un groupe allyle et $R^2$ représente un groupe méthyle, $R^3$ ne représente pas $-OCH_3$ et, si $R^1$ représente un groupe benzyle et $R^2$ un groupe méthyle ou benzyle, $R^3$ ne représente pas $-CH_3$, $-SCH_3$ ou $-SO-CH_3$,

$R^{10}$ représente un atome d'hydrogène, un groupe alkyle en $C_1$ à $C_{12}$ un atome d'halogène ou un groupe alcanoyle en $C_2$ à $C_8$,

$R^{11}$ représente un atome d'hydrogène, un groupe alkyle en $C_1$ à $C_8$, alcényle en $C_3$ à $C_5$, phénylalkyle en $C_7$ à $C_9$, hydroxyalkyle en $C_1$ à $C_4$, ou phényle ,

$R^{12}$ représente un groupe alkylène en $C_2$ à $C_{16}$, non ramifié ou ramifié , qui peut être interrompu par un ou plmusieurs $-O-$, $-S-$ ou $-N(R11)-$,

$R^{13}$ représente un atome d'hydrogène, un groupe alkyle en $C_1$ à $C_8$ ou phényle,

$R^{14}$, $R^{15}$ et $R^{16}$ représentent chacun, indépendamment l'un de l'autre, un atome d'hydrogène ou un groupe alkyle en $C_1$ à $C_4$, ou bien $R^{14}$ et $R^{15}$ forment ensemble un groupe alkylène en $C_3$ à $C_7$,

$R^{17}$ représente un atome d'hydrogène, un groupe alkyle en $C_1$ à $C_{12}$, qui peut être interrompu par un ou plusieurs $-O-$, un groupe alcényle en $C_3$ à $C_5$, phénylalkyle en $C_7$ à $C_9$, hydroxyalkyle en $C_1$ à $C_4$, $-CH_2CH_2CN$, $CH_2CH_2COO$(alkyle en $C_1$ à $C_4$), alcanoyle en $C_2$ à $C_8$ ou benzoyle,

$R^{18}$ représente un atome d'hydrogène, un groupe alkyle en $C_1$ à $C_{12}$, un groupe alkyle en $C_1$ à $C_6$ (substitué par $-CN$, $-OH$, un groupe alcoxy en $C_1$ à $C_4$, alcénoxy en $C_3$ à $C_6$, $-OCH_2CH_2CN$, $-OCH_2CH_2COO$ (alkyle en $C_1$ à $C_4$), $-COOH$ ou $-COO$(alkyle en $C_1$ à $C_4$)), $-(CH_2CH_2O)_nH$ avec n valant 2 à 20, un groupe alcanoyle en $C_2$ à $C_8$, alcényle en $C_3$ à $C_{12}$, cyclohexyle, hydroxycyclohexyle, phényle, phényle (substitué par de l'halogène, par un groupe alkyle en $C_1$ à $C_{12}$ ou alcory en $C_1$ à $C_4$), un groupe phénylalkyle en $C_7$ à $C_9$, ou un groupe $-Si$(alkyle en $C_1$ à $C_8)_r$ (phényle)$_{3-r}$, avec $_r$ valant 1, 2 ou 3,

$R^{19}$ représente un atome d'hydrogène, un groupe alkyle en $C_1$ à $C_{12}$, alcényle en $C_3$ à $C_{12}$, cyclohexyle, un groupe alkyle en $C_1$ à $C_6$ (substitué par $-SH$, $-OH$, $-CN$, $-$ COO(alkyle en $C_1$ à $C_4$), alcoxy en $C_1$ à $C_4$, $-OCH_2CH_2CN$ ou $-$ $OCH_2CH_2COO$(alkyle en $C_1$ à $C_4$)), un groupe phényle, un groupe phényle substitué par de l'halogène, par un groupe alkyle en $C_1$ à $C_{12}$ ou alcoxy en $C_1$ à $C_4$) ou un groupe phénylakyle en $C_7$ à $C_9$,

$R^{20}$ et $R^{21}$ représentent chacun, indépendamment l'un de l'autre, un atome d'hydrogène, un groupe alkyle en $C_1$ à $C_{12}$, hydroxy alkyle en $C_2$ à $C_4$, alcoxy alkyle en $C_2$ à $C_{10}$, alcényle en $C_3$ à $C_5$, cycloalkyle en $C_5$ à $C_{12}$, phénylalkyle en $C_7$ à $C_9$, phényle, phényle (substitué par de l'halogène, par un groupe alkyle en $C_1$ à $C_{12}$ ou alcoxy en $C_1$ à $C_4$), alcanoyle en $C_2$ ou $C_3$ ou benzoyle, ou bien,

$R^{20}$ et $R^{21}$ forment ensemble un groupe alkylène en $C_2$ à $C_8$, qui peut être interrompu par $-O-$,

$-S-$, ou $-N(R^{17})-$, ou qui peut être substitué par un groupe hydroxy, alcoxy en $C_1$ à $C_4$, ou $-COO$(alkyle en $C_1$ à $C_4$),

et

$R^{22}$ représente un groupe alkyle en $C_1$ à $C_{18}$, un groupe phényle ou naphtyle (non substitué ou substitué par de l'halogène, par un groupe alkyle en $C_1$ à $C_{12}$, ou alcoxy en $C_1$ à $C_8$) ou un sel d'addition d'un tel composé.

**2.** Composé selon la revendication 1, de formule I dans laquelle $Ar^1$ représente un groupe de formule IV, V ou VII, et $R^1$, $R^2$ $R^3$, $R^4$, $R^5$, $R^6$, $R^7$, $R^8$, $R^9$, $R^{10}$, U, V et W ont les sens indiqués à la revendication 1.

**3.** Composé selon la revendication 2, de formule I dans laquelle $Ar^1$ représente un groupe de formule IV, $R^5$ et $R^6$ représente chacun un atome d'hydrogène ou d'halogène ou un groupe alkyle en $C_1$ à $C_{12}$ ou un groupe $-OR^{18}$, $-SR^{19}$, $-SO-R^{19}$, $SO_2-R^{19}$, $-N(R^{20})(R^21)$, $-NH-SO_2-R^{22}$ ou

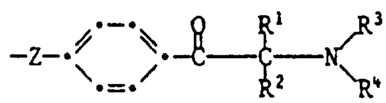

formule dans laquelle Z représente $-O-$, $-S-$, $-N(R^{11})-$ ou $-N(R^{11})-$ $R^{12}-N(R^{11})-$ ou

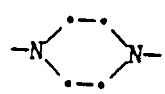

$R^7$ et $R^8$ représentent chacun un atome d'hydrogène ou d'halogène et $R^9$ représente un atome d'hydrogène, d'halogène ou un groupe alkyle en $C_1$ à $C_{12}$, et $R^1$, $R^2$, $R^3$, $R^4$, $R^{11}$, $R^{12}$, $R^{18}$, $R^{19}$, $R^{20}$ et $R^{22}$ ont les sens indiqués à la revendication 1, et, si $R^1$ représente un groupe allyle et $R^2$ représente un groupe méthyle, $R^5$ ne représente pas un groupe $-OCH_3$ et, si $R^1$ représente un groupe benzyle et $R^2$ représente un groupe méthyle ou benzyle, $R^5$ ne représente pas $-OCH_3$, $-SCH_3$ ou $-SOCH_3$.

**4.** Composé selon la revendication 3, de formule I, dans laquelle $Ar^1$ représente un groupe de formule IV, dans laquelle $R^5$ représente un groupe $-OR^{18}$, $-SR^{19}$, $-N(R^{20})(R_{21})$ ou

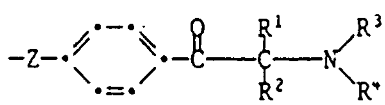

$R^6$, représente un atome d'hydrogène ou d'halogène ou un groupe alkyle en $C_1$ à $C_4$, ou a l'un des sens indiqués pour $R^5$; $R^7$ et $R^8$ représentent chacun un atome d'hydrogène ou d'halogène et $R^9$ représente un atome d'hydrogène ou un groupe alkyle en $C_1$ à $C_4$;

Z représente $-O-$, $-S-$ ou $-N(R^{11})-$;

$R^1$ représente :

(a) un reste de formule :

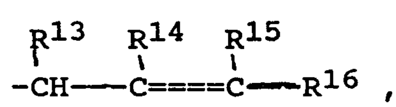

ou

(b) un reste de formule $-CH(R^{13})-Ar^2$ dans laquelle $Ar^2$ représente un reste phényle non substitué ou substitué par de l'halogène, par un groupe alkyle en $C_1$ à $C_{12}$, par un groupe alcoxy en $C_1$ à $C_4$, par $-(OCH_2CH_2)_n-OCH_3$ ou par un groupe benzyle, et n vaut 1 à 10;

$R^2$ a l'un des sens indiqués pour $R^1$ ou représente un groupe alkyle en $C_1$ à $C_8$,

$R^3$ et $R^4$ représentent chacun, indépendamment l'un de l'autre, un groupe alkyle en $C_1$ à $C_{12}$ un

groupe alkyle en $C_2$ à $C_4$ (substitué par un groupe alcoxy en $C_1$ à $C_4$ $-CN$ ou $-COO$ (alkyle en $C_1$ à $C_4$)), un groupe allyle, cyclohexyle ou benzyle, ou bien $R^3$ et $R^4$ forment ensemble un groupe alkylène en $C_4$ à $C_6$, qui peut être interrompu par $-O-$ ou par $-N(R^{17})-$,

$R^{11}$ représente un atome d'hydrogène, un groupe alkyle en $C_2$ à $C_4$, allyle, benzyle ou alcanoyle en $C_2$ à $C_4$,

$R_{13}$, R14, $R^{15}$ et $R^{16}$ représentent chacun, indépendamment l'un de l'autre un atome d'hydrogène ou un groupe méthyle,

$R^{17}$ représente un atome d'hydrogène, un groupe alkyle en $C_1$ à $C_4$, benzyle, 2-hydroxyéthyle ou acétyle;

$R^{18}$ représente un atome d'hydrogène, un groupe alkyle en $C_1$ à $C_4$, 2-hydroxyéthyle, 2-méthoxyéthyle, 2-allyle oxyéthyle, allyle, cyclohexyle, phényle, benzyle ou $-Si(CH_3)_3$;

$R^{19}$ représente un atome d'hydrogène, un groupe alkyle en $C_1$ à $C_{12}$, 2-hydroxyéthyle, 2-méthoxyéthyle, phényle, p-tolyle ou benzyle;

$R^{20}$ et $R^{21}$ représentent chacun, indépendamment l'un de l'autre, un atome d'hydrogène, un groupe alkyle en $C_1$ à $C_{12}$, alcoxy -alkyle en $C_{12}$ à $C_6$, acétyle, allyle ou benzyle, ou bien $R^{20}$ et $R^{21}$ forment ensemble un groupe alkylène en $C_4$ à $C_6$, lequel peut être interrompu par $-O-$, ou par $-N(R^{17})-$,

et, si $R^1$ représente un groupe allyle, $R^5$ ne représente pas $-OCH_3$, et, si $R^1$ représente un groupe benzyle et $R^2$ représente un groupe méthyle ou benzyle, $R^5$ ne représente pas un groupe $-OCH_3$ ou $-SCH_3$.

**5.** Composé selon la revendication 4, dans lequel $Ar^1$ représente un groupe de formule IV, $R^5$ représente un groupe $-OR^{18}$, $-SR^{19}-N(^{20})(R^{21})$, $R^6$ représente un atome d'hydrogène ou de chlore ou un groupe alkyle en $C_1$ à $C_4$, ou a l'un des sens indiqués pour $R^5$; $R^7$ et $R^8$ représentent chacun un atome d'hydrogène ou de chlore et $R^9$ représente un atome d'hydrogène ou un groupe alkyle en $C_1$ à $C_4$;

$R^1$ représente (a) un reste de formule $-CH_2-C(R^{14})=CHR^{15})$ ou bien (b) un reste de formule $-CH_2-AR^2$, le srnbole $Ar^2$ représentant un reste phényle non substitué, ou substitué par un atome d'halogène, par un groupe alkyle en $C_1$ à $C_{12}$, par un groupe alcoxy en $C_1$ à $C_4$, par $-(OCH_2CH_2)-_nOCH_3$ ou par un groupe benzoyle, et n vaut 1 à 10;

$R^2$ a l'un des sens indiqués pour $R^1$ ou représente un groupe alkyle en $C_1$ à $C_8$,

$R^3$ et $R^4$ représentent chacun, indépendamment l'un de l'autre, un groupe alkyle en $C_1$ à $C_6$, 2-méthoxyéthyle, allyle ou benzyle, ou bien $R^3$ et $R^4$ forment ensemble un groupe tétraméthylène, pentaméthylène ou 3-oxapentaméthylène,

$R^{14}$ et $R^{15}$ représentent chacun un atome d'hydrogène ou un groupe méthyle,

$R^{18}$ représente un groupe alkyle en $C_1$ à $C_4$, 2-hydroxyéthyle, 2-méthoxyéthyle ou phényle,

$R^{19}$ représente un groupe alkyle en $C_1$ à $C_{12}$, 2-hydroxyéthtyle, 2-méthoxyéthyle, phényle ou p-tolyle,

$R^{20}$ et $R^{21}$ représentent chacun un atome d'hydrogène, un groupe alkyle en $C_1$ à $C_4$, 2-méthoxéthyle, acétyle ou allyle ou bien,

$R^{20}$ et $R^{21}$ forment ensemble un groupe alkylène en $C_4$ ou $C_5$, qui peut être interrompu par $-O-$ ou $-N(CH_3)-$,

et, si $R^1$ représente un groupe allyle, $R^5$ ne représente pas $-OCH_3$, et, si $R^1$ représente un groupe benzyle et si $R^2$ représente un groupe méthyle ou benzyle, $R^5$ ne représente pas un groupe $-OCH_3$ ou $-SCH_3$.

**6.** Composé selon la revendication 5, dans lequel $R^5$ représente un groupe $-SR^{19}$, $R^7$ et $R^8$ représentent chacun un atome d'hydrogène; $R^1$ représente un reste de formule

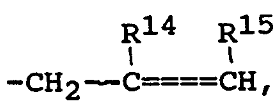

et tous les autres substituants ont les sens indiqués à la revendication 5.

**7.** Composé selon la revendication 6, dans lequel et $R^9$ représentent chacun un atome d'hydrogène et tous les autres substituants ont le sens indiqué à la revendication 6,

**8.** Composé selon la revendication 6, dans lequel $R^1$ représente un groupe allyle et tous les autres substituants ont le sens indiqué à la revendication 6.

**9.** Composé selon la revendication 5, dans lequel $R^5$ représente un groupe $-N(R^{20})(R^{21})$; $R^7$ et $R^8$ représentent chacun un atome d'hydrogène et tous les autres substituants ont les sens indiqués à la revendication 5.

**10.** Composé selon la revendication 9, dans lequel $R^6$ et $R^9$ représentent chacun un atome d'hydrogène et tous les autres substituants ont le sens indiqué à la revendication 9,

**11.** Composé selon la revendication 9, dans lequel $R^1$ représente un groupe allyle ou benzyle, et tous les autres substituants ont le sens indiqué à la revendication 9.

**12.** Composé selon la revendication 3, de formule I, dans laquelle $Ar^1$ représente un groupe de formule IV;
$R^5$ représente un atome d'hydrogène ou d'halogène ou un groupe alkyle en $C_1$ à $C_{12}$ et, $R^6$, $R^7$, $R^8$ et $R^9$ représentent chacun un atome d'hydrogène; $R^1$ représente un groupe allyle ou benzyle; $R^2$ représente un groupe alkyle en $C_1$ à $C_6$, allyle ou benzyle; $R^3$ et $R^4$ représentent chacun, indépendamment l'un de l'autre, un groupe alkyle en $C_1$ à $C_{12}$, un groupe alkyle en $C_2$ à $C_4$ (substitué par un groupe alcoxy en $C_1$ à $C_4$, par $-CN$ ou par $-COO$ (alkyle en $C_1$ à $C_4$)), allyle, cyclohexyle ou benzyle; ou bien $R^3$ et $R^4$ forment ensemble un groupe alkylène en $C_4$ à $C_6$, qui peut être interrompu par $-O-$ ou par $N(R^{17})$, et $R^{17}$ représente un atome d'hydrogène, un groupe alkyle en $C_1$ à $C_4$, ou 2-hydroxyéthyle.

**13.** Utilisation d'un composé selon la revendication 1, comme photo-amorceur pour la photopolymérisation de composés à insaturation éthylénique.

**14.** Utilisation selon la revendication 13 d'un composé selon la revendication 4 comme photo-amorceur pour le photodurcissement de systèmes pigmentés, comme des encres d'impression ou des laques ou peintures blanches.

**15.** Utilisation selon la revendication 14 d'un composé selon la revendication 12 pour le photodurcissement de systèmes non pigmentés.

**16.** Utilisation selon la revendication 13 d'un composé de la revendication 1 comme photo-amorceur pour la préparation d'une photoréserve ou de plaques d'impression.

**17.** Utilisatrion selon la revendication 13 d'un composé de la revendication 1 comme photo-amorceur pour des peintures pour l'extérieur, qui durcissent ensuite en surface sous l'effet de la lumière du jour.

**18.** Composition photodurcissable, contenant :
A) au moins un composé photopolymérisable, à insaturation éthylénique et,
B) comme photo-amorceur, au moins un composé selon la revendication 1.

**19.** Composition photodurcissable selon la revendication 18, contenant :
A) au moins un composé photopolymérisable, à insaturation éthylénique ,
B) comme photo-amorceur, au moins un composé selon la revendication 4, et
C) un pigment blanc ou coloré.

**20.** Composition photodurcissable selon la revendication 19, contenant :
A) au moins un composé photopolymérisable, à insaturation éthylénique
B) comme photo-amorceur, au moins un composé selon la revendication 4,
C) un pigment blanc ou coloré et,
D) comme photosensibilisateur, un composé carbonylé aromatique choisi dans la classe des benzophénones, des thioxanthones, des anthraquinones, des 3-acylcoumarines et des 3-(aroylméthylène)-thiazolines.

**21.** Mélanges photodurcissables selon la revendication 18, contenant :
A) au moins un composé photopolymérisable à insaturation éthylénique , et

B) comme photo-amorceur, un mélange de :
B1) au moins un composé selon la revendication 1, et
B2) un dérivé d'aryl-titanocène, qui est substitué dans le reste aryle par du fluor ou par $CF_3$ .

**Revendications pour l'Etat contractant suivant : ES**

**1.** Composition photodurcissable contenant :
A) au moins un composé photopolymérisable à insaturation éthylénique, et ,
B) comme photo-amorceur, au moins un composé répondant aux formules I, II, III ou IIIa,

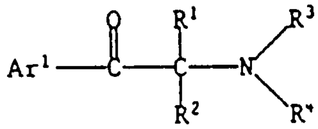
I

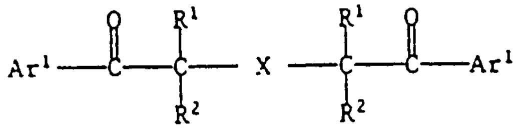
II

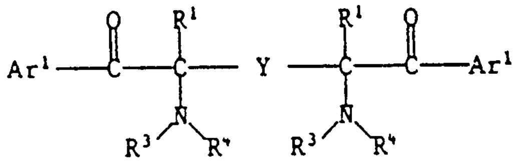
III

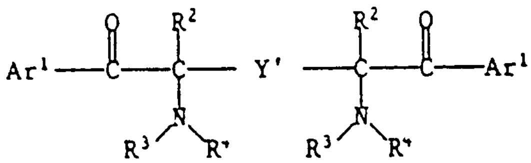
IIIa

dans lesquelles $Ar^1$ représente un reste aromatique répondant aux formules IV, V, VI ou VII :

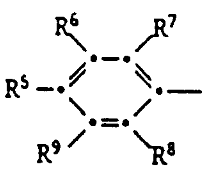
IV,

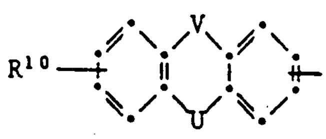
V

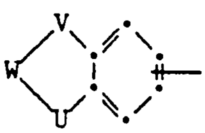
VI

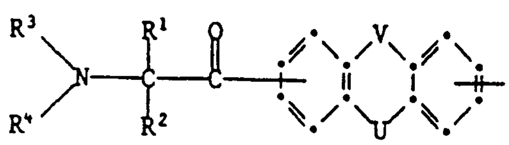
VII

dans lesquelles,
X représente un reste divalent de formule

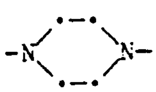
,

$-N(R^{11})-$ ou $-N(R^{11})-R^{12}-N(R^{11})-$

Y représente un groupe alkylène en $C_1$ à $C_6$, xylylène, cyclohexylène ou une liaison directe,

Y' représente un groupe xylylène, alcène diyle en $C_4$ à $C_8$, alcadiène diyle en $C_6$ à $C_{10}$, dipentènediyle ou dihydroxylylène,

U représente $-O-$, $-S-$ ou $-N(R^{17})-$,

V représente $-O-$, $-S-$, $-N(R^{17})-$, $-CO$, $-CH_2-$, $-CH_2CH_2-$, un groupe alkylydène en $C_2$ à $C_6$ ou une liaison directe,

W représente un groupe alkylène en $C_1$ à $C_7$ ou alkylidène en $C_2$ à $C_6$, non ramifiés ou ramifiés,

$R^1$ représente :

(a) un reste de formule

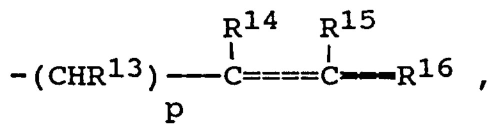

dans laquelle est nul ou vaut 1,

ou

(b) un reste de formule

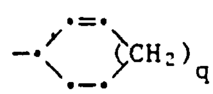

dans laquelle q vaut 0, 1, 2 ou 3,

ou,

(c) un reste de formule

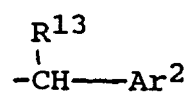

dans laquelle $Ar^2$ représente un reste phényle, naphtyle, furyle, thiényle ou pyridyle non substitué ou substitué par de l'halogène, par un groupe OH, alkyle en $C_1$ à $C_{12}$ du par un groupe alkyle en $C_1$ à $C_4$ (substitué par OH, par un halogène, par un groupe $-N(R^{11})_2$, $-$alcoxy en $C_1$ à $C_{12}$, $-COO$(alkyle en $C_1$ à $C_{18}$), $-CO(OCH_2CH_2)_nOCH_3$ ou $-OCO-$alkyle (en $C_1$ à $C_4$), un groupe alcoxy en $C_1$ à $C_{12}$, un groupe alcoxy en $C_1$ à $C_4$ (substitué par un reste $-COO$ (alkyle en $C_1$ à $C_{18}$) ou $-CO(OCH_2CH_2)_nCH_2$), un groupe $-(OCH_2CH_2)_nOH$, $-(OCH_2CH_2)_nOCH_3$, alkylthio en $C_1$ à $C_8$, phénoxy, $-COO$(alkyle en $C_1$ à $C_{18}$), $-CO(OCH_2CH_2)_nOCH_3$, phényle ou benzyle, l'indice n valant 1 à 20,

Ou

(d) $R^1$ forme avec $R^2$ un reste de formule

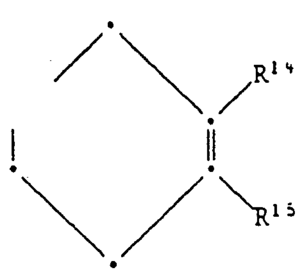

ou

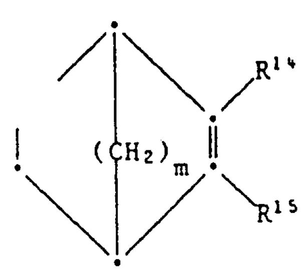

l'indice m valant 1 ou 2,

$R^2$ possède l'un des sens indiqués pour $R^1$ ou représente un groupe cycloalkyle en $C_5$ ou $C_6$, un groupe alkyle en $C_1$ à $C_{12}$ (non substitué ou substitué par un groupe alcoxy en $C_1$ à $C_4$, phénoxy, halogéno ou phényle) ou un groupe phényle (non substitué ou substitué par de l'halogène, par un

groupe alkyle en $C_1$ à $C_{12}$ ou alcoxy en $C_1$ à $C_4$),

$R^3$ représente un atome d'hydrogène, un groupe alkyle en $C_1$ à $C_{12}$, un groupe alkyle en $C_2$ à $C_4$ (substitué par un groupe hydroxy, alcoxy en $C_1$ à $C_4$, $-CN$ ou $-COO$(alkyle en $C_1$ à $C_4$)), alcényle en $C_3$ à $C_5$, cycloalkyle en $C_5$ à $C_{12}$, ou phénylalkyle en $C_7$ à $C_9$,

$R^4$ représente un groupe alkyle en $C_1$ à $C_{12}$, un groupe alkyle en $C_2$ à $C_4$ (substitué par un groupe hydroxy, alcoxy en $C_1$ à $C_4$ $-CN$ ou $-COO$ (alkyle en $C_1$ à $C_4$)), alcényle en $C_3$ à $C_5$, cycloalkyle en $C_5$ à $C_{12}$, phénylalkyle en $C_7$ à $C_9$, phényle ou phényle (substitué par de l'halogène, par un groupe alkyle en $C_1$ à $C_{12}$, alcoxy en $C_1$ à $C_4$ ou $-COO$(alkyle en $C_1$ à $C_4$)), ou bien $R^1$ forme avec $R^2$ un groupe alkylène en $C_1$ à $C_7$, phénylalkylène en $C_7$ à $C_{10}$, o-xylylène, 2-butenylène ou un groupe oxa-ou aza-alkylène en $C_2$ ou $C_3$,

ou bien

$R^3$ et $R^4$ forment ensemble un groupe alkylène en $C_3$ à $C_7$, qui peut être interrompu par $-O-$, $-S-$, $-CO-$ ou $-N(R^{17})-$, ou qui peut porter comme substituant au moins un groupe hydroxy, alcoxy en $C_1$ à $C_4$ ou $-COO$(alkyle en $C_1$ à $C_4$),

$R^5$, $R^6$, $R^7$, $R^8$ et $R^9$ représentent chacun, indépendamment l'un de l'autre, un atome d'hydrogène ou d'halogène, un groupe alkyle en $C_1$ à $C_{12}$, cycloalkyle en $C_5$ ou $C_6$, phényle, benzyle, benzoyle ou un groupe $-OR^{18}$, $-SR^{19}$, $-SOR^{19}$ $-SO_2-R^{19}$, $-N(R^{20})(R^{21})$, $-NH-SO_2-R^{22}$ ou

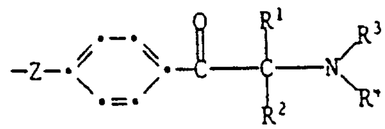

formule dans laquelle Z représente $-O-$, $-S-$, $-N(R^{11})-$, $-N(R^{11})-R^{12}-N(R^{11})-$ou

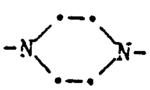

et si $R^1$ représente un groupe allyle et $R^2$ représente un groupe méthyle, $R^3$ ne représente pas $-OCH_3$ et, si $R^1$ reprasente un groupe benzyle et R2 un groupe méthyle ou benzyle, $R^3$ ne représente pas $-OCH_3$, $-SCH_3$ ou $-SO-CH_3$,

$R^{10}$ représente un atome d'hydrogène, un groupe alkyle en $C_1$ à $C_{12}$, un atome d'halogène ou un groupe alcanoyle en $C_2$ à $C_8$,

$R^{11}$ représente un atome d'hydrogène, un groupe alkyle en $C_1$ à $C_8$, alcényle en $C_3$ à $C_5$, phénylalkyle en $C_7$ à $C_9$, hydroxyalkyle en $C_1$ à $C_4$, ou phényle ,

$R^{12}$ représente un groupe alkylène en $C_2$ à $C_{16}$, non ramifié ou ramifié , qui peut être interrompu par un ou plmusieurs $-O-$, $-S-$ ou $-N(R^{11})-$,

$R^{13}$ représente un atome d'hydrogène, un groupe alkyle en $C_1$ à $C_8$ ou phényle,

$R^{14}$, $R^{15}$ et $R^{16}$ représentent chacun, indépendamment l'un de l'autre, un atome d'hydrogène ou un groupe alkyle en $C_1$ à $C_4$, ou bien $R^{14}$ et $R^{15}$ forment ensemble un groupe alkylène en $C_3$ à $C_7$,

$R^{17}$ représente un atome d'hydrogène, un groupe alkyle en $C_1{}^1$ à $C_{12}$, qui peut être interrompu par un ou plusieurs $-O-$, un groupe alcényle en $C_3$ à $C_5$, phénylalkyle en $C_7$ à $C_9$, hydroxyalkyle en $C_1$ à $C_4$, $-CH_2CH_2CN$, $-CH_2CH_2COO$(alkyle en $C_1$ à $C_4$), alcanoyle en $C_2$ à $C_8$ ou benzoyle,

$R^{18}$ représente un atome d'hydrogène, un groupe alkyle en $C_1$ à $C_{12}$, un groupe alkyle en $C_1$ à $C_6$ (substitué par $-CN$, $-OH$, un groupe alcoxy en $C_1$ à $C_4$, alcénoxy en $C_3$ à $C_6$, $-OCH_2CH_2CN$, $-OCH_2CH_2COO$ (alkyle en $C_1$ à $C_4$), $-COH$ ou $-COO$(alkyle en $C_1$ à $C_4$)), $-(CH_2CH_2O)_nH$ avec n valant 2 à 20, un groupe alcanoyle en $C_2$ à $C_8$, alcényle en $C_3$ à $C_{12}$, cyclohexyle, hydroxycyclohexyle, phényle, phényle (substitué par de l'halogène, par un groupe alkyle en $C_1$ à $C_{12}$ ou alcoxy en $C_1$ à $C_4$), un groupe phénylalkyle en $C_7$ à $C_9$, ou un groupe $-Si$(alkyle en $C_1$ à $C_8$)$_r$ (phényle)$_{3-r}$, avec $_r$ valant 1, 2 ou 3,

$R^{19}$ représente un atome d'hydrogène, un groupe alkyle en $C_1$ à $C_{12}$, alcényle en $C_3$ à $C_{12}$, cyclohexyle, un groupe alkyle en $C_1$ à $C_6$ (substitué par $-SH$, $-OH$, $-CN$, $-COO$(alkyle en $C_1$ à $C_4$), alcoxy en $C_1$ à $C_4$, $-OCH_2CH_2CN$ ou $-OCH_2CH_2COO$(alkyle en $C_1$ à $C_4$)), un groupe phényle, un groupe phényle substitué par de l'halogène, par un groupe alkyle en $C_1$ à $C_{12}$ ou alcoxy en $C_1$ à $C_4$) ou un groupe phénylalkyle en $C_77$ à $C_99$,

$R^{20}$ et $R^{21}$ représentent chacun, indépendamment l'un de l'autre, un atome d'hydrogène, un groupe alkyle en $C_1$ à $C_{12}$, hydroxy alkyle en $C_2$ à $C_4$, alcoxy alkyle en $C_2$ à $C_{10}$, alcényle en $C_3$ à $C_5$, cycloalkyle en $C_5$ à $C_{12}$, phénylalkyle en $C_7$ à $C_9$, phényle, phényle (substitué par de l'halogène, par un groupe alkyle en $C_1$ à $C_{12}$ ou alcory en $C_1$ à $C_4$), alcanoyle en $C_2$ ou $C_3$ ou benzoyle, ou bien,

$R^{20}$ et $R^{21}$ forment ensemble un groupe alkylène en $C_2$ à $C_8$, qui peut être interrompu par $-O-$, $-S-$, ou $-N(R^{17})-$, ou qui peut être substitué par un groupe hydroxy, alcoxy en $C_1$ à $C_4$, ou $-COO$(alkyle en $C_1$ à $C_4$),

et

$R^{22}$ représente un groupe alkyle en $C_1$ à $C_{18}$, un groupe phényle ou naphtyle (non substitué ou substitué par de l'halogène, par un groupe alkyle en $C_1$ à $C_{12}$, ou alcoxy en $C_1$ à $C_8$) ou un sel d'addition d'un tel composé.

**2.** Composition photodurcissable selon la revendication 1, contenant :
A) au moins un composé photopolymérisable, à insaturation éthylénique,
B) comme photo−amorceur, au moins un composé selon la revendication 1, et
C) un pigment blanc ou coloré .

**3.** Composition durcissable selon la revendication 2, contenant :
A) au moins un composé photopolymérisable, à insaturation éthylénique ,
B) comme photo−amorceur, au moins un composé selon la revendication 1,
C) un pigment blanc ou coloré, et
D) comme photosensibilisateur, un composé carbonylé aromatique choisi dans la classe des benzéphénones, des thioxanthones, des anthraquinones, des 3−acylcoumarines et des 3−(aroylméthylène)−thiazolines.

**4.** Mélange photodurcissable selon la revendication 1, contenant :
A) au moins un composé photopolymérisable, à insaturation éthylénique, et
B) comme photo−amorceur, un mélange de
B1) au moins un composé selon la revendication 1, et
B2) un dérivé d'aryl−titanocène, qui est substitué dans le reste aryle par du fluor ou par $CF_3$ .